(19)

**Europäisches Patentamt**
**European Patent Office**
**Office européen des brevets**

(11) **EP 4 478 263 A1**

(12) **EUROPEAN PATENT APPLICATION**

(43) Date of publication:
18.12.2024 Bulletin 2024/51

(51) International Patent Classification (IPC):
**G06N 10/60** (2022.01)

(21) Application number: 24155882.4

(22) Date of filing: 05.02.2024

(52) Cooperative Patent Classification (CPC):
**G06N 10/60; G16C 10/00;** G16C 20/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.06.2023 GB 202308768**

(71) Applicant: **Phasecraft Limited**
**London Greater London W1T 4PW (GB)**

(72) Inventors:
• **SANHUEZA, Raul Antonio Santos**
**London, W1W 6XB (GB)**
• **SHERIDAN, Evan**
**London, W1W 6XB (GB)**
• **CUBITT, Toby Stanley**
**London, W1W 6XB (GB)**

(74) Representative: **Cavanna, Edward Paul et al**
**Mathys & Squire**
**The Shard**
**32 London Bridge Street**
**London SE1 9SG (GB)**

(54) **VQE FOR IMPROVED DFT SIMULATIONS**

(57)     A method of performing improved Kohn-Sham Density Functional Theory, DFT, calculations, for simulation of a material, chemical, or biological system includes: (i) constructing a model Hamiltonian of the system to be simulated; (ii) inputting an electron density of the system to be simulated; (iii) constructing a Kohn-Sham potential and (iv) executing a quantum Kohn-Sham DFT algorithm iteration using the electron density from (ii) and the Kohn-Sham potential from (iii) to compute an updated electron density and an updated total energy of the system to be simulated. The Kohn-Sham potential is constructed by: (1) providing a second Hamiltonian based on the model Hamiltonian; (2) calculating a ground state energy of the second Hamiltonian, as a function of electron density, using a quantum algorithm executed by a noisy intermediate-scale quantum, NISQ, processor; (3) computing a quantum-obtained exchange correlation energy functional, as a function of electron density, from the NISQ processor-obtained ground state energy of step (2); (4) obtaining a quantum-obtained exchange correlation potential functional by differentiation of the quantum-obtained exchange correlation energy functional from step (3); and (5) using the quantum-obtained exchange correlation potential functional from (4) to construct the Kohn-Sham potential.

Figure 3

**Description**

FIELD OF THE INVENTION

The present application relates to methods and apparatuses for performing hybrid quantum-classical density functional theory calculations. In particular, the application relates to methods for improved DFT simulation of material, chemical, and biological systems on noisy intermediate scale quantum processors.

BACKGROUND OF THE INVENTION

Materials systems constitute large, interacting and inhomogeneous quantum many-body problems. As such, understanding and describing their properties is a notoriously difficult problem. Exact simulation of materials on classical computers is restricted to small system sizes. Quantum simulation on quantum computers promises precise computation of their dynamics but remains out of reach of current hardware. Instead, a number of well-reasoned approximations across different time and length scales have been used to make consistently reliable predictions. For example, as exact simulation is restricted on classical devices to small systems then instead of exact simulation one performs approximation simulations that are well reasoned. Density Functional Theory (DFT) is one example of a well-reasoned and well-known approximation at the atomic scale, with a known source of error that is the approximation of the unknown universal functional.

**Density Functional Theory**

Classical DFT is the principal and most widely used method to simulate a broad range of ground state properties for materials systems at a quantum mechanical level. At the atomic scale, Density Functional Theory (DFT) is the predominant computational method used for computing groundstate electronic and structural properties for a broad range of atomic, molecular and materials systems. Understanding and describing the properties materials systems is a notoriously difficult problem as these constitute large, interacting, and inhomogeneous many-body problems that warrant quantum simulation for a precise understanding of their dynamics. The central quantum mechanical quantity that encapsulates these processes is the many body wavefunction, which scales exponentially with the number of particles. As a result, the purview of exact simulation of materials on classical computers is restricted to small system sizes, and a number of well-reasoned approximations across different time and length scales are required to make consistently reliable predictions. One of the main motivations of DFT is to avoid the classical intractabilities of simulating many body systems, and its many successes have bolstered its popularity in many areas of physics and chemistry. In the most common formulation of DFT, its algorithmic complexity scales as $O(N^3)$, with $N$ being the number of particles, whereas the full quantum many body simulation scales as $O(4^N)$.

Density Functional Theory (DFT) is the gold standard classical algorithm for predicting these properties, but relies on approximations of the unknown universal functional, limiting its general applicability for many fundamental and technologically relevant systems. At the atomic scale, ab initio Density Functional Theory (DFT) has the premier status among computational methods that compute ground state electronic and structural properties for a broad spectrum of atoms, molecules and materials. The essence of the approximation is to replace the many body wavefunction as the central quantity that is solved for - with that of the one body density - which instead is only a function of the 3 spatial dimensions. Thus, the exponential classical computational cost of computing the ground state of an interacting many-body wave system is replaced with a polynomial time classical variational algorithm for a fictitious non-interacting single particle system. Under certain conditions the solution of this non-interacting system is guaranteed to produce the ground state properties of the interacting system - allowing for tractable and efficient simulations beyond the scope of exact classical wavefunction methods. This is achieved by absorbing all of the many-body complexity into something known as the exchange correlation (XC) functional; however this is generally QMA-hard to compute.

DFT computations have their basis in the Hohenberg-Kohn and Kohn-Sham theorems, which show that for any electronic structure Hamiltonian, there always exists a non-interacting Hamiltonian from which a groundstate density can be constructed that is identical to that of the full many-body system. Perhaps the best-known implementation of DFT is its ab initio formulation for the many electron Hamiltonian within the Born-Oppenheimer approximation, where the ions generate a static, periodic varying external potential. Of course, the local -- also known as Kohn-Sham (KS) -- potential in this non-interacting Hamiltonian is itself determined by the many-body groundstate density. So, finding the correct KS potential for a given system is at least as hard as the original many-body problem. In Kohn-Sham DFT, the system is then mapped from the many-particle Hilbert space onto the effective non-interacting Kohn-Sham space, via the Kohn Sham potential. Particularly well-known parametrisations of the XC potential, which is the only unknown contribution within KS potential, involve exact computations of the homogeneous electron gas.

In DFT, a way of getting around this is to approximate the energy, as a functional of the density. This energy functional $E[n]$, where $n$ is the groundstate density, decomposes into four parts:

$$E[n] = T[n] + U_H[n] + E_{\text{ext}}[n] + E_{\text{xc}}[n],$$

$$(1)$$

the kinetic energy term $T[n]$, the Coulomb (or "Hartree") term $U_H[n]$, the external potential $E_{\text{ext}}[n]$, and the "exchange-correlation" (XC) term $E_{\text{xc}}[n]$. This latter term is essentially defined as everything not accounted for by the other terms:

$$E_{\text{xc}}[n] = E[n] - T[n] - U_H[n] - E_{\text{ext}}[n].$$

$$(2)$$

The kinetic, Hartree, and external terms can be computed classically within KS DFT. Meanwhile, $E_{\text{xc}}[n]$ only depends on the electron-electron interactions, so in principle is a universal functional that applies to every many-body electronic structure problem. However, since knowledge of this universal functional would allow all many-body problems to be solved, determining the exact form of this universal functional is believed to be intractable. Instead, in DFT, various heuristic approximations of the XC functional are used. Simple and compact approximations are often the most useful, interpretable and computationally efficient. In its most common formulation, the algorithmic complexity of DFT scales as $O(N^3)$, with $N$ being the number of particles, whereas the full quantum many-body simulation scales exponentially in the particle number, $N$, on classical computers. Thus, DFT is intrinsically linked to the XC functional design and choice, where simple and compact approximations have shown to be the most useful, interpretable, and computationally efficient. The performance of DFT is intrinsically limited by the accuracy of the XC functional approximation. Classical DFT replaces the exact XC functional with various approximations, establishing the field of approximate functional design, which has enjoyed abundant success for non-interacting systems. The struggle of DFT to fully express the many body correlations present in the ground state of interacting electronic systems fundamentally limits the present applicability of DFT. In principle, the exact functional is only accessible through a full many body solution, which cannot be achieved on classical computers except in a very small number of scenarios. Therefore, the potential for success of DFT calculations for simulations to inform practical applications implicitly relies on compact approximations of the unknown exchange correlational functional, and so the design of such functionals has become a primary pillar of classical materials simulation research.

Over the past 50 years, hundreds of approximate XC energy functionals have been proposed, for both continuous basis sets as well as model Hamiltonians, and used in virtually every facet of atomistic materials modelling. This has resulted in the foundational papers of DFT becoming some of the most cited works in the scientific literature and the birth of the field of ab-initio materials design. The most basic and best-known of these is the local density approximation (LDA), which is determined by exact computation of the homogeneous, non-interacting electron gas. Other more sophisticated parameterisations exist and are used in modern DFT computations with different levels of classical algorithmic complexity. Although DFT is a very successful method for studying weakly correlated electronic systems, a number of challenges still remain, from choosing the most appropriate functional for the system at hand to the general failure of XC functionals when applied to strongly correlated systems.

When applying classical functionals to strongly correlated electronic systems, DFT can often qualitatively fail in its predictions. There are two prevalent DFT camps that separate the fields of weakly and strongly correlated electronic systems. Typically, real space DFT is used to study weakly correlated systems, while lattice Hamiltonians are used for the latter, to fully account for all of the electronic correlations present in strongly correlated system.

The lattice formulation of DFT for model Hamiltonians is an active area of research, where minimal models, such as the Hubbard model or tight binding Hamiltonians, are fully characterised as a Kohn-Sham system. In this formalism, coined Lattice DFT (LDFT), density functionals are constructed for lattice models, which can then be used in iterative Kohn-Sham DFT algorithms.

XC functional design for correlated lattice models has also been explored classically in the context of various lattice inhomogeneities. It was first used in semiconductor models but has since been more routinely applied to one dimension Hubbard chains where exact parametrisations of the XC functional are available, based on the Bethe Ansatz. The role of the discontinuity in XC potentials on model and real systems is particularly important. In particular, implementing the Bethe Ansatz LDA functional for the one dimensional Hubbard model has allowed comparison to investigate how the XC discontinuity relates to molecular (hydrogen chains, dimolecules) and periodic materials systems. Exact functional results have been benchmarked against a number of state of the art classical functionals.

Exact Diagonalisation can be used to compute an approximation to the exchange correlation functional. In 2d this approach has been used to highlight ground state properties of large inhomogeneous sheets of graphene in a tight binding model with Hubbard type interactions, where the exact functional was parametrised using Exact Diagonalisation data. This was demonstrated by using Exact Diagonalisation on the homogeneous 2D Hubbard model for small clusters of graphene flakes to construct an approximation to the exchange correlation potential. This representation of the functional was then used for much larger sizes of graphene systems including inhomogeneities and the results compared to predictions of the ground state by Lieb's theorem for Hubbard models on bipartite graphs.

As mentioned above, a number of challenges still remain: from choosing the most appropriate functional for each system, to the general failure of XC functionals when applied to strongly correlated systems. There are many functionals which have impressive records across broad material classes when predicting certain properties, while completely failing for others. For example, many functionals tend to struggle with describing bond disassociation: when electrons localise on ions upon stretching as the inter-atomic distance is increased. This is closely related to the misprediction of lattice constants in solids even when the electronic structure is qualitatively correct. Moreover, for strongly correlated materials systems the current generation of XC potentials often make qualitatively bad predictions for most electronic and structural ground state properties, let alone attaining quantitatively accurate results. In recent years exact many body corrections to DFT based on embedding schemes have demonstrated considerable promise. Marrying real space DFT with lattice Hamiltonians, in approximations such as the GW approximation or dynamical mean field theory, DMFT, is the current state of the art approach to modelling strongly correlated materials systems.

In recent years, quantum many body approaches, such as DMFT, and quasiparticle self-consistent GW approaches have alleviated some of these issues in some strongly correlated systems, however they tackle the issue via exact embedding approaches. As the XC functional is not modified or updated, instead exact quantum simulations for restricted regions of the Hilbert space must be performed multiple times. Such exact quantum simulations require extensive classical computational power for each new material.

The first studies of DFT and quantum computing analysed its computational complexity. However, with the advent of readily available near-term devices, there have been a number of early studies of the development of hybrid quantum-DFT algorithms being applied to small molecules and mid-gap defect states in solid state materials. Generally, in these works DFT is bootstrapped by VQE and quantum subspace expansions by active space embedding methods, where the full Kohn-Sham space of electrons is partitioned into separate classical and quantum sections.

In addition, in recent years machine learning methods have been trained against exact results, showing utility for parametrising the XC functional for molecules and for the Hubbard model. Sequences of tests in 1d that explore the effect of impurities in these systems have been performed. It is now clear that improving methods for XC functional design is a key factor in unlocking the full range of capabilities afforded by Kohn-Sham DFT.

There is also a formulation in the spirit of DFT in the Zumbach Maschke basis that outlines a theoretical proposal to compute an approximation of the XC potential based on density matrix measurements that iteratively correct the single-particle density towards the true solution. Improvements to DFT computations have also been proposed using a technical approach where a local approximation of the XC functional is calculated based on the Zumbach Maschke basis, by performing density matrix measurements on a quantum computer. In this case, density matrix measurements means computing the one- and two-body reduced density matrices on a quantum computer. The one-body reduced density matrix is,

$$\rho_{ij} = \langle \Psi_{GS} | c_i^\dagger c_j | \Psi_{GS} \rangle$$

where $|\Psi_{GS}\rangle$ is the ground state. While the two-body reduced density matrix is given by:

$$\Gamma_{ijkl} = \langle \Psi_{GS} | c_i^\dagger c_k^\dagger c_l c_j | \Psi_{GS} \rangle.$$

The proposed method states that once the ground state is known and these measurements are performed then the XC potential can be estimated. Thus, the formulation of DFT in the Zumbach Maschke basis outlines a technique to compute a local approximation of the XC functional based on density matrix measurements.

For the fault tolerant framework, an approach has been proposed that could obtain the exact XC functional by using machine learning, but this requires the use of quantum algorithms that are not suited for near term devices. The Levy-Lieb formalism of DFT has been applied to the one dimensional Hubbard model using quantum algorithms, such as VQE, to compute the ground state properties of the Hubbard dimer, while supplementing the technique with a quantum kernel that can learn the density functionals of observables by machine learning. This approach focuses on how to apply machine learning to low dimensional functions, for example the Hubbard dimer, and may not be scalable to allow application to larger physical, material, chemical, or biological systems.

In another instance, a mapping between the KS Hamiltonian and an auxiliary Hamiltonian is implemented on a quantum computer and it has been posited that solving the auxiliary system instead can result in computational speed up. This approach was then applied to 1D Hubbard chain of size $L = 8$ and a hydrogen molecular chain but retained the use of classical approximations of the XC energy functional. **Quantum Computation**

Recently, quantum computing hardware has become sufficiently advanced to allow non-trivial demonstrations of quantum computation of groundstate properties. Many different quantum algorithms have been proposed for finding groundstates. However, the most widely studied on near-term quantum hardware is the variational quantum eigensolver (VQE). At its heart, like many classical variational algorithms, VQE is based on the Rayleigh-Ritz variational characterisation of the eigenvalues of the Hamiltonian H:

$$\lambda_0(H) = \min_{|\psi\rangle} \langle\psi|H|\psi\rangle .$$

$$(3)$$

However, in contrast with classical variational algorithms, in VQE the state $|\psi\rangle$ is generated - and its energy $E(|\psi\rangle)$ = $\langle\psi|H|\psi\rangle$ with respect to the Hamiltonian $H$ measured - on a quantum computer. This requires that a suitable, efficiently preparable class of quantum states $|\psi(\vec{p})\rangle$ be chosen, with parameters $\vec{p}$ over which to optimise. Because quantum computers are able to efficiently construct states that cannot be described or computed efficiently on classical computers, VQE has the potential to outperform classical variational methods.

The accuracy of VQE results depends on whether this variational class contains a good approximation to the groundstate, and many different variational classes have been proposed and studied. The performance of VQE also depends on the success of the classical optimisation routine used to optimise the measured energy $E(|\psi(\vec{p})\rangle)$ over the parameters $\vec{p}$. Theoretically, good choices of the variational class and classical outer optimiser show promise in solving certain quantum many-body problems.

It is generally accepted that the computational efficiency of exact quantum simulations may be vastly improved through the use of reliable and efficient quantum computers that lack the drawbacks of currently available, and near-term, NISQ computers. However, the current regime of noisy intermediate-scale quantum (NISQ) hardware is notoriously subject to significant rates of noise and error. Noisy intermediate-scale quantum processors are currently limited by significant issues with gate fidelity, and decoherence, which limit them to low numbers of qubits. The success or otherwise of VQE therefore also depends, crucially, on hardware noise. In turn, this limits possible applications to those which can be performed by quantum algorithms that are accurate for shallow-depth quantum circuits. Although non-trivial VQE computations have now been demonstrated on real quantum devices, none have yet gone beyond system sizes that can be simulated on classical computers. Additionally, near term quantum algorithms such as VQE, may not succeed in producing good approximations to the ground state of interacting systems, in particular as the system becomes more parametrised. Although the results reproduce the correct physical properties in these cases, the VQE *state* itself, as generated on the quantum computer, is not necessarily close to the true groundstate. As such, the direct application of VQE to interacting systems using NISQ processors has caveats and uncertainties when computing ground state properties for simulation of real-world.

## SUMMARY OF THE INVENTION

Quantum computers open up new avenues for modelling the physical properties of materials and molecules. In this work we develop a hybrid quantum/classical algorithm called *quantum enhanced DFT* (QEDFT) that systematically constructs quantum approximations of the universal functional using data obtained from a quantum computer. QEDFT combines the complementary strengths of DFT and quantum computation in a new hybrid quantum/classical approach to modelling materials and molecules.

Aspects and examples of the invention are set out in the claims and aim to address at least a part of the above described technical problem, and related problems.

In particular, the invention set out herein is directed to an improved DFT algorithm by utilising current and near-term NISQ processors and intentional simplification of the systems to be simulated and careful selection of which parts of the DFT algorithm are performed by quantum computation rather than classical computation in order to maximise the benefits of such a hybrid quantum-classical DFT algorithm. In this way, the methods can be thought of, in part, as providing a bridge between a complex system to be simulated on the one hand and a limited capacity and robustness in the otherwise seemingly ideal environment for simulations of quantum systems.

As will be apparent from the foregoing, this application discloses several advantageous approaches to performing DFT on a hybrid quantum-classical processing system. These include:
A hybrid quantum classical algorithm that uses low-depth and poor-quality quantum simulations to improve the quality of solutions over classical approximations for classical Density Functional Theory simulations.

A systematic method to design local quantum exchange correlation functionals for lattice models.

Quantum algorithms for the exact extraction of external potential to density mappings and the respective inversion.

Density constrained parametrised quantum circuits for inhomogenous interacting fermionic systems.

A quantum kernel method for the extrapolation to the thermodynamic limit of the local density exchange correlation energy functional.

A hybrid quantum classical algorithm for the multivariate DFT XC functional through global perturbations via the external potential.

Disclosed herein is a method of performing improved Kohn-Sham Density Functional Theory, DFT, calculations, for simulation of a material, chemical, or biological system, comprising the steps of: (i) constructing a model Hamiltonian of the system to be simulated; (ii) inputting an electron density of the system to be simulated; (iii) constructing a Kohn-Sham potential by:

(1) providing a second Hamiltonian based on the model Hamiltonian;
(2) calculating a ground state energy of the second Hamiltonian, as a function of electron density, using a quantum algorithm executed by a noisy intermediate-scale quantum, NISQ, processor;
(3) computing a quantum-obtained exchange correlation energy functional, as a function of electron density, from the NISQ processor-obtained ground state energy of step (2);
(4) obtaining a quantum-obtained exchange correlation potential functional by differentiation of the quantum-obtained exchange correlation energy functional from step (3);
(5) using the quantum-obtained exchange correlation potential functional from (4) to construct the Kohn-Sham potential; and

(iv) executing a quantum Kohn-Sham DFT algorithm iteration using the electron density from (ii) and the Kohn-Sham potential from (iii) to compute an updated electron density and an updated total energy of the system to be simulated.

In general terms, this can be thought of as using the quantum computer to approximate the XC functional which is then fed into a classical DFT iteration to find the groundstate, i.e., the quantum computer is tasked with steering the DFT iteration towards an accurate solution, rather than using a quantum computer to find the groundstate itself. In the detailed description, we provide specific examples where a Variational Quantum Eigensolver (VQE) is used for the quantum part of the QEDFT computation. But we note that our approach is general, and the person skilled in the art would appreciate that any other quantum groundstate approximation algorithm could be used in step (iii)(2) of QEDFT.

We benchmark the QEDFT algorithm on the Fermi-Hubbard model, both numerically and on data from experiments on real quantum hardware. We find that QEDFT surpasses the quality of groundstate results obtained from Hartree-Fock DFT, as well as from direct application of conventional quantum algorithms such as VQE. Furthermore, we demonstrate that QEDFT works even when only noisy, low-depth quantum computation is available, by benchmarking the algorithm on data obtained from Google's quantum computer.

We further show how QEDFT also captures quintessential properties of strongly correlated Mott physics for large Fermi-Hubbard systems using functionals generated on much smaller system sizes. Our results indicate that QEDFT can be applied to realistic materials and molecular systems and has the potential to outperform the direct application of either DFT or VQE alone, without the requirement of large scale or fully fault-tolerant quantum computers.

Advantageously, this method combines the merits of both DFT and quantum algorithms, by using results from a quantum algorithm to provide a qualitatively improved exchange correlation potential functional (XC functional) that can nudge the DFT algorithm towards more precise solutions. The improvement in exchange correlation functional arises from step (2) where the ground state computation is performed by quantum algorithms on an NISQ. Advantageously, this method relies only on measurements of the ground state energy to calculate an improved XC functional for use in step (iv) and does not explicitly require updates to the form of the XC functional iteratively based on quantum-computed density matrix measurements. Furthermore, as the computation of the ground state energy is the only step that must be carried out by an NISQ processor, the use of quantum resources can be minimized while still producing an improvement in the efficiency and accuracy of DFT simulations for a wide variety systems that are traditionally difficult to simulate using DFT. Additionally, low quality quantum algorithms can be successfully used in this way meaning that this method can be executed employing NISQ processors with limited numbers of qubits rather than needing to wait for further innovation in quantum computation hardware. Once the ground state energy has been computed, the quantum-obtained XC potential can be used to construct a Kohn-Sham potential and provided to a DFT calculation on a classical computer for any target molecular system and this can be used to estimate ground state properties such as energies, forces, and densities. These ground state properties may then ultimately be used to inform higher level quantum mechanics (QM) and molecular mechanics (MM) simulations. Furthermore, this method is flexible and capable of scaling with the size of available quantum processor, whereas the classical approaches to approximating the exchange correlation can only be applied for small system sizes. Specifically, it is possible to design an approximation to the XC potential using a system which is not the same size - importantly the system may be orders of magnitude smaller than the target system. For example, the exchange correlation functional from a $1 \times 50$ simulation may be used within the DFT simulation of a $1 \times 1000$ system and still have the ability to nudge the DFT variational algorithm towards more precise solutions. This represents a significantly more efficient method for DFT simulation of large systems. Although inherent finite-size errors may arise from the use of a $1 \times 50$ functional in this context, it may still be used and prove competitive against state-of-the-art classical methods of approximating the exchange correlation functional. In this sense, we incorporate into the approach both that within a NISQ environment only low depth and small qubit cases are possible.

Our empirical study, presented in the detailed description, shows that QEDFT has a number of advantages over both Hartree-Fock DFT alone, and VQE (or other purely-quantum) approaches performed alone.

Advantageously, QEDFT usually achieves better accuracy than either Hartree-Fock DFT alone, or quantum VQE alone.

Advantageously, QEDFT does not necessarily rely on quantitatively accurate quantum computations (e.g. high fidelity with the true groundstate), so it may provide a path to quantum advantage even when the quantum hardware is noisy.

Advantageously, QEDFT can be applied to large many-body systems on small quantum hardware, so it may still be able to provide a quantum advantage for real materials even while the available hardware is too small to fit a full quantum simulation of the material.

Advantageously, this method can be applied to periodic varying potentials - which covers all solids in their metallic, insulating, semiconducting and superconducting phases - including defect driven or doped systems. Furthermore, strongly correlated electron systems, which are difficult to deal with by classical DFT methods, can also be simulated with this improved method of DFT.

Advantageously, DFT in the Kohn Sham formulation implemented as a self-consistent variational algorithm is amenable to dealing with a certain level of error in the approximation of the XC potential. This means that even if the XC potential is quantitatively erroneous for certain density values, if it is qualitatively accurate near the ground state then it will evaluate the ground state energy and density accurately there. If at a single iteration the evaluation of the XC potential is incorrect, the next iteration may this corrects for itself. The relatively high tolerance of Kohn-Sham DFT to quantitative errors in XC potential means that this method is robust and more tolerant of potentially noisy estimates of the ground state calculated using NISQ processors.

Optionally, computing an updated electron density in step (iv) comprises computing $n_{GS,i} = 2\sum_{j}^{occ/2}\left|\phi_j(i)\right|^2$ and mixing it with the previous density estimate, $n^{(j+1)} = \alpha n^{(j)} + (1 - \alpha)n^{(j+1)}$, where $j$ is a DFT iteration index and $\alpha$ is a linear mixing parameter.

Optionally, providing the model Hamiltonian in step (i) includes either: providing an inhomogeneous Hamiltonian; or providing a homogeneous Hamiltonian, wherein the homogeneous Hamiltonian is the inhomogeneous Hamiltonian with an external potential removed.

Advantageously, this method of DFT can be applied to simulate systems that are homogeneous and inhomogeneous. This allows for complex systems with non-zero external potentials to be modelled.

Optionally, when the model Hamiltonian provided in step (i) is inhomogeneous then the second Hamiltonian provided in step (1) is homogeneous, further wherein the model Hamiltonian has an external potential and the second Hamiltonian is based on the model Hamiltonian with the external potential removed.

When the model Hamiltonian of the system to be simulated is inhomogeneous (for example in strongly correlated electron systems), the qualitative features of the XC functional can be approximated well by calculation of an XC functional from the ground state of a homogeneous version of the model Hamiltonian that has been calculated using a quantum algorithm as in step (2). By providing a homogeneous Hamiltonian, related to the inhomogeneous Hamiltonian by removal of an external potential term, in step (1) it is possible to reduce the complexity of the quantum ground state calculation while still being able to calculate an improved XC functional that is capable of steering DFT simulations of the inhomogeneous system. Because the method is robust against quantitative errors in XC functional and the improvements arise from improved qualitative XC functionals improvements to DFT can be achieved even on current NISQ processors which frequently do not have the gate fidelity to achieve quantitatively accurate simulation of even homogeneous systems directly.

Optionally, calculating the ground state of the second Hamiltonian on an NISQ processor further comprises using a fermion-to-qubit encoding to encode qubits of the NISQ processor.

The fermion-to-qubit encoding can be chosen from a number of possible encodings - e.g. Jordan-Wigner, compact, and Bravi-Kitaev - to streamline encoding of the NISQ processor based on the form of the second Hamiltonian and the NISQ processor hardware. The encoding defines how fermion operators are mapped into qubit operators which manifest as gates in the quantum circuit.

Optionally, the fermion-to-qubit encoding is a Jordan Wigner encoding that maps fermionic creation and annihilation operators of the second Hamiltonian to Pauli strings comprising Pauli X, Y and Z operators.

Jordan-Wigner encoding is a well-known fermion-to-qubit mapping which allows for easy conversion of known model Hamiltonians, e.g., the Hubbard model, into Pauli operators that can be easily converted into a quantum circuit. This encoding is compatible with current and near term NISQ processors because it is well understood how to convert Pauli operators into quantum gates, such as CNOT and RZ gates, which are commonly native to NISQ hardware. This is convenient because it can reduce the amount computational processing required to represent Hamiltonians as appropriate quantum circuits. Conventional Jordan Wigner mapping associates each fermionic mode with a qubit.

Optionally, calculating the ground state of the second Hamiltonian on an NISQ processor further comprises enacting qubit operations generated by unitary qubit operators on the qubits of the quantum information processor.

In order to compute ground state energies for these models XC potentials are created that can be used in the DFT algorithm. Quantum algorithm are used to determine the order and on what qubits the gates which appear in the circuit are executed. A quantum calculation of the ground state comprises the complete execution of a quantum circuit (a way of representing the qubit operations and the qubits on which they act during the algorithm) after which an observable is measured, in this case the ground state energy. The encoding defines how fermion operators are mapped into qubit operators which manifest as gates in the quantum circuit. A realistic practical assumption may be that CNOT and RZ gates are native on the hardware device. The second Hamiltonian, in terms of Pauli strings, may therefore be decomposed into a sequence of CNOT and RZ gates.

Optionally, the model Hamiltonian is the Hubbard model with an external potential term, and the second Hamiltonian is the Hubbard model.

Using DFT to study model Hamiltonians such as the Hubbard model, is useful for a number of reasons, chief among them being that model Hamiltonians already have exact solutions (for small system sizes or certain dimensions), so it is straightforward to assess the quality of a new approximation. The nomenclature of using inhomogeneous for the Hubbard model and an external potential can be related to the similar use of describing materials by an inhomogeneous electron gas.

Optionally, execution of the quantum algorithm by a noisy intermediate-scale quantum, NISQ, processor further comprises executing a Variational Quantum Eigensolver, VQE, algorithm using the NISQ processor.

VQE is a hybrid quantum-classical algorithm for approximating ground state energies on quantum computers. While it is possible to use other quantum algorithms, including for example the Dissipative Quantum Eigensolver (a method for provably approaching a known, e.g. ground, quantum state of an encoded Hamiltonian), VQE has particularly good performance for standard homogeneous Hamiltonians like the Hubbard model. The QC gates are determined by a combination of the algorithm selected for approximating the ground state energy, the choice of fermion-to-qubit encoding, and the particular material or molecule the method is being performed on. As it is well known how to prepare Hamiltonians for simulation using VQE this is a convenient choice.

For VQE we use the standard options as mentioned above. We emphasise however that, unlike when solving problems directly using VQE, we do not necessarily need very accurate approximations for the novel methods disclosed herein, so we can choose lower-depth VQE leveraging the synergy between the classical and quantum systems as explained above, and still obtain good results, where VQE alone at that circuit depth performs poorly or is even provably incapable of finding the true ground state and ground state energy.

To measure the ground state energy using a quantum algorithm we choose VQE, but in general are not restricted to doing so. To deal with continuous systems the VQE circuit can be changed to reflect the internal symmetries of the considered materials system. So, for example, modelling graphene would require a Hubbard model with next-nearest neighbour hopping on a hexagonal lattice. In practice these are all accounted for in the current generations of VQE algorithms. Advantageously, VQE evaluation of an XC functional qualitatively captures some of the main features of the XC potential, even where it is not quantitatively accurate. An important example of these qualitative features is whether or not the XC functional has a discontinuity. More generally, any approximation to the XC potential that incorporates the effects of exchange and correlation of fermions may succeed in nudging the DFT algorithm to more accurate solutions in strongly interacting parameter regimes, with respect to the functionals that do not model exchange or correlation. It is also possible to incorporate constraints into the XC potential that are already known. For example, if the considered system has a fundamental gap or is fully occupied, it is possible to include extra data to the XC potential functions that take advantage of symmetries and known properties of the system. In other words, it is possible to introduce error mitigation techniques when constructing the XC functional obtained from VQE simulations. These techniques effectively reduce the sensitivity of the quantum algorithm to noise, as well reasoned and physically motivated post processing on the quantum data can be performed.

Advantageously, it is possible to use quantum simulated ground state data from low depth VQE circuits and for small lattice sizes and then encode that information into the exchange correlation functional that be used for much larger system sizes, i.e., it is independent of system size. In doing so, it is also possible to systematically improve the approximation by extrapolating away finite sizes effects, incorporate constraints that must be respected and improve with deeper circuits.

Advantageously, we find that combining DFT and VQE in this method outperforms both Hartree-Fock DFT alone, and quantum VQE alone. Moreover, this remains the case even when the VQE computation itself is very noisy, and its output is far from the true XC functional. Despite the VQE output being quantitatively inaccurate, it captures key qualitative features of the exact XC functional that classical approximations struggle to reproduce. And this suffices to steer the DFT iteration to converge to more accurate solutions than it would otherwise reach.

For large systems, the XC functional is essentially a continuous object, whereas a quantum computer can only compute (or approximate) its value at a discrete set of points, with intermediate points approximated by interpolation. In principle, assuming the VQE algorithm were able to compute the exact value of the functional at any given point, the finer the discretisation the more accurate the functional approximation. However, the fact that a discretised functional is being computed has a significant benefit. The number of points in the discretisation corresponds directly to the size of the quantum system used in the quantum computation to compute the value of the functional. Advantageously, therefore, even if the quantum hardware is too small to fit the full system being simulated, one can still use VQE to approximate the XC functional on the quantum computer at the finest discretisation that will fit on the device, and then use this in a DFT calculation of the groundstate density of the much larger system of interest.

Optionally, the VQE algorithm is implemented as a VQE circuit which is lower depth than VQE circuits required to find the ground state of the model Hamiltonian directly.

Advantageously, low quality and low depth data (both from simulations and real quantum computer runs) can be used to improve DFT simulations, even though the quantum approximations themselves are low quality and from low-depth computations on noisy, small- scale quantum hardware. This method advantageously allows DFT simulation of systems with model Hamiltonians that are too complex for direct solution with VQE on current and near-term NISQ processors due to decoherence limitations. It is surprising that the ground-state approximation produced with a noisy NISQ processor can still produce a DFT output that falls within 1% of in particular parameter regimes (e.g. as a specific example $\frac{U}{t} = 4$, $n_x = 1$, $n_y = 8$, with a quarter filling in an impurity potential) of the Hubbard model. This is in contrast to the classical mean field results, which fall outside of this 1% interval. Additionally, the approach does reasonably well at capturing qualitative features of the XC potential as compared to exact classical methods - in particular, the discontinuity of the XC potential is well captured by VQE methods. The XC potential in the Hartree Fock approximation by contrast, perhaps the most famous single particle method after DFT, is zero everywhere by definition, so it is impossible to infer how Mott physics appears in the functional with Hartree Fock. Advantageously, for strongly correlated systems ( $\frac{U}{t} > 1$ ) the estimation of the density is also well described.

Optionally, the VQE algorithm uses a Hamiltonian Variational Ansatz, HVA.

There are different possible VQE ansatzes, the inventors have chosen the popular Hamiltonian Variational Ansatz (HVA) for VQE simulations. For HVA, the initial state can be advantageously prepared from the $U = 0$ ground state of the Hubbard model, which comprises a layer of depth $n_x n_y$ - 1 of Givens rotations acting on adjacent modes. The decomposition of the initial state into signals depends on the native gates available on the hardware device. $n_x$; $n_y$ are the system dimensions, e.g here we consider 2d. The HVA may also be updated with single qubit rotation gates when an inhomogeneous model is under consideration, as opposed to just taking the free fermion terms, for example if the second Hamiltonian in step (2) is inhomogeneous.

Optionally, obtaining a quantum-obtained exchange correlation potential functional in step (4) further comprises using a quantum kernel method for continuous representation of the exchange correlation energy functional as a function of electron density.

This allows for continuous representations of the exchange correlation potential, which can be more easily applied to large systems, whilst reducing finite size effects. Optionally, the quantum kernel method comprises interpolative and/or extrapolative procedures for obtaining the exchange correlation energy functional as a function of electron density.

Interpolative and/or extrapolative procedures are well known and already optimised procedures that allow for construction of continuous representations. In this case, continuous representations of the exchange correlation potential, which can then be more easily applied to large or complex systems, whilst reducing finite size effects. Using interpolative and/or extrapolative procedures also allows for a qualitatively improved XC functional while being able to modify the number of required VQE/quantum algorithm calls on the NISQ processor. The maximum number of calls is related to the size of the system to be simulated and the number of system sizes being considered.

Optionally, the quantum kernel method further comprises using a Local Density Approximation, LDA, to construct a continuous representation of the exchange correlation energy functional.

In the classical method of DFT for solids, the Local Density Approximation (LDA) uses the Quantum Monte Carlo simulations of the homogeneous electron gas to approximate the XC functional, and then uses DFT to (in theory) solve the interacting inhomogeneous (due to the presence of an external potential) electron gas model. The application of LDA to the XC energy functional in step (4) expresses the XC energy functional in a compact representation so that the XC potential functional can be easily computed by differentiation, i.e.

$$V_{XC}^{QLDA}(n_i, U) = \frac{\partial E_{XC}^{QLDA}(n, U)}{\partial n} \Big|_{n=n_i} .$$

Thus, for a system of size L, $n_i$ ranges from $n_1$ to $n_L$ and the maximum number of VQE calls to approximate the XC energy functional is given by *2L*. By using interpolative and extrapolative techniques it is possible to modify the number of these calls, whose upper bound will be $2\,\Pi_i L_i$, where $L_i$ is the set containing the number of system sizes considered, e.g. $L = \{4, 5, 6\}$ would require at most 240 quantum computations to be performed.

Optionally, the material system to be simulated is a strongly correlated electron system.

This method is particularly well suited to improvements in simulating strongly correlated electron systems because it is possible to use a simple model incorporating Mott physics as the second Hamiltonian in order to obtain a much-improved qualitative XC potential functional that includes essential features, such as discontinuities, needed for simulating such systems. Additionally, simulation of complex, real-world systems generally requires the ability to incorporate the properties of strongly correlated electrons which has been a previous failure of DFT due to inability to produce quality XC potential functional for such systems.

Optionally, the Kohn-Sham DFT algorithm is a Kohn-Sham Lattice Density Functional Theory, LDFT, algorithm.

An advantage of considering lattice models over the electronic Hamiltonian is that, for electronic Hamiltonians, a physically reasoned basis must be chosen for the system at hand, and this introduces additional, complicated, technical considerations. Additionally, model Hamiltonians already have exact solutions (for small system sizes or certain dimensions), so it is straightforward to assess the quality of a new method of exchange correlation functional approximation. However, the algorithmic procedures for lattice models and electronic Hamiltonians are equivalent and either may be used with the method described herein.

Optionally, the NISQ processor requires fewer qubits to calculate the ground state of the second Hamiltonian than the number of qubits required to calculate the ground state of the model Hamiltonian of the system to be simulated directly.

By reformulating the model Hamiltonian as a second Hamiltonian for which the ground state is calculated, it is possible to reduce the quantum resources required for this method. This is advantageous because it means that a significantly improved method of DFT can be implemented using current and near term NISQ processors rather than needing to wait for significant advances in hardware for quantum computation.

Optionally, a quantum Kohn-Sham DFT algorithm iteration in step (iv) comprises the steps of:

(a) taking the electron density from (ii) and the Kohn-Sham potential from (iii) as an input to construct Kohn-Sham equations;

(b) solving the Kohn-Sham equations;

(c) providing the updated electron density, and the updated total energy of the system to be simulated, based on solutions of the Kohn-Sham equations in step (b); and

(d) checking if a convergence condition is met, further wherein the condition for convergence is evaluated based on the electron density input in step (ii) and the updated electron density.

Regarding the ground state problem and DFT, the ground-state density minimises the total energy functional which enforces the functional derivative to be zero and results in a variational equation for wavefunctions that can construct the ground state density. This equation takes the form of a single-particle Schrodinger equation which can be treated as a standard eigenvalue problem. All of many-body physics manifest in the quantum-computed exchange correlation potential contribution of an otherwise fully determined effective single particle potential. These equations are solved for self consistently, as the XC potential depends on the density, and the density depends on the unknown wavefunctions. At self-consistency, these wavefunctions no longer change and are used to construct the ground state density and energy.

Optionally, evaluating the convergence condition in step (d) comprises calculating:

($\alpha$) a density difference metric from the electron density input in step (ii) and the updated electron density; and/or

($\beta$) an energy difference metric from a total energy of the system to be simulated, based on the electron density from step (ii), and the updated total energy of the system to be simulated,

and comparing the density difference metric from ($\alpha$) and/or the density difference metric from ($\beta$) with a threshold condition for each difference metric.

A possible density difference metric is the L2 norm of density vectors, given by $\Delta\rho = \frac{1}{L}\sum_j^L |\rho_{j,current} - \rho_{j,previous}|$. A reasonable energy difference metric may be $\Delta E = |E_{updated} - E_{step\ (ii)}|$, and the threshold condition may then take the form of asking if $\Delta E < X$, then convergence is considered to be met and the iterative stops. $X$ is typically less than $10^{-8}$.

Optionally, further quantum Kohn-Sham DFT algorithm iterations, are performed by providing the updated electron density from (c) as the input in step (ii) if the convergence condition in (d) is not met such that steps (ii) to (iv) continue until the convergence condition for the electron density, and/or the convergence condition for the total energy of the system to be simulated, is met in step (d).

The manner in which we have implemented the procedure allows for a clean separation of the quantum and the classical processing, so that the method is not dependent in any way on fast communication between the classical and quantum parts. However, we also highlight that there are alternative implementations of our method that would require on-the-fly calls to the quantum computer. In the workflow where the quantum computer, or NISQ processor is called at each cycle of the classical DFT algorithm, this would necessarily need to be automated. In this case at each iterative step of the DFT algorithm the XC potential must be queried for a given density and this evaluation is performed on-the-fly.

Optionally, a further Kohn-Sham DFT iteration is performed in a classical manner, the classical Kohn-Sham DFT iteration comprising the steps (ii) to (iv), wherein step (ii) receives the updated electron density from (c) as an input and step (iii) replaces steps (1) to (5) by providing a classically obtained exchange correlation potential functional as an input to construct the Kohn-Sham potential.

An advantage of this approach is that it combines classical and quantum iterations to reduce the number of calls to NISQ but can still yield an unexpectedly improved accuracy.

Optionally, further classical Kohn-Sham DFT iterations are performed until the convergence condition for the electron density, and/or the convergence condition for the total energy of the system to be simulated, is met in step (d).

This improved method of DFT can provide a significant advantage over methods that employ traditional XC functional approximations, even when only one quantum DFT is performed, and the remaining iterations performed until convergence is deems to be met. This allows for the number quantum computing resources to be minimised. Thus, this method of improved DFT can be realised even with in the current limitations in quantum computation hardware.

Optionally, further quantum or classical Kohn-Sham DFT algorithm iterations are performed, including at least one further quantum Kohn-Sham DFT iteration, until the convergence condition for the electron density, and/or the convergence condition for the total energy of the system to be simulated, is met in step (d).

Further quantum DFT iterations may be executed, to allow for simulation of more complicated material, chemical, and biological systems where it may be necessary to nudge the XC potential functional more than once to improve the accuracy of the simulation. In this way, the method can be optimised for accuracy and computational efficiency, allowing for simulations that are as fast as possible without sacrificing accuracy.

Optionally, further quantum Kohn-Sham DFT iterations are performed at periodic intervals with classical Kohn-Sham DFT algorithm iterations in between, until the convergence condition for the electron density, and/or the convergence condition for the total energy of the system to be simulated, is met in step (d).

We note that in all of these formulations it is also possible, and may be advantageous, to "toggle" the calls to the quantum computer, e.g. maybe the QC is only called every 10 DFT iterations, rather than every single iteration, to reduce the total number of calls to the quantum computer.

Optionally, the updated electron density, and the updated total energy of the system to be simulated, from step (c) are used to construct ground state properties of the model Hamiltonian describing the material system which is to be simulated.

Knowledge of the ground state properties of the model Hamiltonian allows for calculation of many different properties of a material, chemical, or biological systems. Some examples of such properties include energies, forces, and densities which influence the behaviours of these systems. In addition to materials modelling, qDFT could also be applied to quantum chemical systems. By using qDFT as a subroutine to predict ground state properties it could also be used for Molecular Dynamics (MD) and structure searching applications, including simulation of biological systems such as covalent inhibitors in Myotonic Dystrophy applications.

Optionally, the method may comprise, prior to steps (i) to (iv), the step of performing at least one classical Kohn-Sham DFT algorithm iteration using a classical exchange correlation potential functional.

This method may also be advantageously combined with existing methods for DFT.

Also disclosed herein is a hybrid quantum-classical apparatus for simulation of a material, chemical, or biological system, by way of enacting the above described method.

The method is not a priori tied the hardware details. However, as with all quantum computation methods for materials or chemistry systems, for any concrete material or molecule it is necessary (or at least highly advantageous) to match the interaction structure of the material or model to the interaction structure of the qubits in the quantum computer.

Optionally, the NISQ processor may be a Superconducting qubit based platform. These platforms have the most impressive results for large system sizes, but this is because other platforms are restricted to small qubit numbers at the moment. As VQE is a quantum-classical hybrid method, it communicates between the quantum computer to the classical computer to update the weights of the parametrised quantum circuit, as determined by the choice of encoding and ansatz.

The parameters one needs to know about a quantum device are its connectivity, native gates, and number of available qubits for computation. The resulting VQE circuit encapsulates the encoding and initial state that is run on the NISQ processor. The parameters needed to set up NISQ hardware for a specific VQE process is the representation of the fermionic Hamiltonian decomposed into native gates on that processor. As VQE is a hybrid-classical algorithm, this VQE circuit is parametrised by variational parameters which are updated using a classical optimised algorithm run on a classical computer between each query to the quantum computer. Here the classical optimiser which is used is crucial in ensuring the success of a NISQ computation, but standard off-the-shelf classical optimisers can be used for this purpose. Furthermore, it is possible to also tailor the choice of decomposition into QC gates to the specific quantum hardware capabilities, for example qubit layout, connectivity.

Optionally, the hybrid quantum-classical apparatus may comprise:

a noisy intermediate-scale quantum, NISQ, processor; and

at least one classical processor,

wherein the NISQ processor is used to calculate the ground state energy of the second Hamiltonian in step (2).

Optionally, the at least one classical processor is used to perform the quantum Kohn-Sham DFT iterations in step (iv).

Optionally, the at least one classical processor is used to perform the classical Kohn-Sham DFT iteration(s).

Optionally, the at least one classical processor is used to perform all of the steps of the disclosed method except for step (2).

Near term quantum methods can greatly benefit from an efficient quantum-classical algorithmic protocol, as these devices are restricted in the number of qubits available and circuit depth achievable before decoherence of the qubits occurs, rendering the simulation overcome by noise. Here we follow an approach where the quantum device produces classical data which informs a classical algorithm that is run on a classical computer, so the computational load is optimally split between classical and quantum resources. In particular, we use the quantum computer to produce an approximation to the exact exchange correlation energy for molecular systems, which can be used to create classical representations of the exchange correlation potential required within a DFT algorithm.

Other steps may also be performed on the NISQ processor, as appropriate to the capability of the hardware. More than one NISQ processor may be utilised, and/or more than one classical processor may be used.

Optionally, the at least one classical processor performs steps comprising at least one of:

a fermion-to-qubit mapping of the second Hamiltonian into a parameterised quantum circuit;

updating VQE parameters based on the variational principle;

obtaining a continuous representation of the exchange correlation energy functional or the exchange correlation potential functional;

storing an exchange correlation potential functional locally or transmitting it to a remote location; or

storing an exchange correlation energy functional locally or transmitting it to a remote location.

Storing the exchange correlation potential functional locally or transmitting it to a remote location allows for this method to be optionally implemented with a clean separation between the quantum executed processes and the classically executed processes. For example, software may be used to compute the ground state energy value at a given density, for a Hamiltonian which may be passed to an automatic internal library that is fully automated to return the ground state energy, or the exchange correlation potential functional, or the exchange correlation energy and density for a given external potential. This may also be thought of at a high level as, there being multiple possible approaches, for example: we can compute the XC functional via VQE during the DFT loop, at the density in the current DFT iteration - the *online* approach; or we can use VQE to approximate the entire universal XC functional in advance, and then use this VQE-computed XC functional in the DFT iteration - the *offline* approach. There are pros and cons to both the online and offline approaches. The online approach only evaluates the XC functional at densities required during the DFT iterations whereas the offline approach may compute it at many densities that are not needed; thus, relatively speaking, an online approach may be considered to provide a more efficient method in terms of *quantum* computational resources.

On the other hand, the online approach requires calling out to the quantum computer during the DFT computation, thereby limiting the speed of the DFT computation whereas the offline approach decouples the quantum and classical computation. Thus, relatively speaking, the offline approach may be considered a more efficient method in terms of DFT computation speed. Moreover, since we are approximating the universal functional, in principle the VQE functional approximation only needs to be computed once, and thereafter the same functional can be used in multiple QEDFT computations.

Optionally, at least one classical processor is part of a control apparatus for the NISQ processor.

Also disclosed herein is a non-transient computer readable medium comprising instructions which cause a computer to enact the above-described method steps.

Initialisation of the quantum circuits may be automated via software, or it can be performed semi-manually. In the workflow where the XC functional is computed in advance and stored for later lookup, it could be done semi-manually, though automating it would be convenient.

The quantum computer is used to construct an approximation to the exact exchange correlation potential. This does not have to be computed in real-time during the DFT algorithm. It could also be precomputed and then stored in a lookup table or database, that can then be used by a classical DFT algorithm. DFT is a variational algorithm and thus is iterative, so at each step of the DFT cycle a query to this database is performed or the quantum computer is called directly.

The main idea that we propose here is to combine the merits of both classical DFT with near term quantum algorithms such as VQE. The essence of the approach is to bootstrap DFT with low-depth and poor-precision VQE simulations to construct quantum enhanced XC functionals. By doing so we nudge the classical DFT method towards better solutions than those obtained with classical functional approximations. The merits of this approach compared to state-of-the-art classical functionals are shown by applying it to the Hubbard model in a number of parameter regimes and dimensionalities.

## BRIEF DESCRIPTION OF THE FIGURES

Figure 1A is a flow chart illustrating steps in a hybrid method for generating an XC functional as applied to the specific example of the Hubbard dimer, including equations and code for each step;

Figure 1B shows images corresponding to each of the steps in Figure 1A;

Figure 2A shows a flow chart for a DFT loop as part of the methods disclosed herein, as applied to the specific example of the Hubbard dimer, including equations and code for each step;

Figure 2B shows images corresponding to the steps in Figure 2A;

Figure 3 shows an overview of the hybrid quantum-classical DFT process;

Figure 4 shows representative data for the energy per site computed using a homogeneous Hamiltonian in the DFT loop;

Figure 5 is the exchange correlation potential calculated from Figure 4;

Figure 6 shows representative data for the ground state energy, highlighting the convergence within DFT;

Figure 7 shows representative data of the ground state density for an example of a 1D Hubbard model as a function of U using various models;

Figure 8 illustrates a trade-off between complexity and computational complexity often encountered in DFT;

Figure 9 shows a quantum circuit for performing VQE;

Figure 10 shows a plot of a universal functional within the Kohn-Sham theorem for U=5;

Figure 11 shows a plot of potential-density invertibility within the Kohn-Sham theorem for U=5;

Figure 12 shows the homogeneous energy modelled by various methods;

Figure 13 shows the correlation energy of the same system as Figure 12 modelled by various methods;

Figure 14 shows the kinetic energy of the same system as Figure 12 modelled by various methods;

Figure 15 shows the Hartree-Fock energy of the same system as Figure 12 modelled by various methods;

Figure 16 shows the XC potential of the same system as Figure 12 modelled by various methods;

Figure 17 shows a comparison between different models for calculating an electron density of a small 1D chain;

Figure 18 shows the difference between the ground state density for the exact result and various methods shown in Figure 17 without constraints;

Figure 19 shows a comparison of three methods after 5 Kohn-Sham iterations;

Figures 20 and 21 show various parameters as calculated after 5 Kohn-Sham iterations for BALDA and VQE respectively;

Figure 22 shows the difference between the ground state density for the exact result and various methods;

Figure 23 is a schematic of a quantum-classical hybrid algorithm to accelerate and improve over standard classical methods for the simulation of covalent inhibitors in Myotonic Dystrophy applications;

Figure 24 is a schematic showing the interrelationships between various subroutines in the overall process; and

Figure 25 is a schematic illustrating the algorithmic workflow of quantum computation within a quantum/materials modelling calculation.

Figure 26 shows the XC energy (left) and potential (right) for the $1 \times 12$ Fermi-Hubbard model at $U/t = 4$ using different methods. The solid lines are produced using a cubic spline. The VQE results are obtained using a classical emulator. Note that the continuous LDA approximations perform badly in this system.

Figure 27 shows the effect of U and system size on the XC potential for 1D Fermi Hubbard models.

Figure 28 shows the $\log_{10}$ Frobenius norm error between the VQE XC potential functionals and the exact functional for 1D Fermi-Hubbard models at different values of interaction strength $U/t$ and system size $n_x$.

Figure 29 shows the effect of U and system size on the XC potential for 2D Fermi Hubbard models.

Figure 30 shows the $\log_{10}$ Frobenius norm error between the VQE XC potential functionals and the exact functional for 2d Fermi-Hubbard models at different values of interaction strength $U/t$ and system size $[n_x, n_y]$.

Figures 31 show the $1 \times 12$ Fermi Hubbard model for $U = 4$ and at quarter-filling with an external quadratic potential. Figure 31A illustrates the convergence of the total energy per lattice site within the DFT loop. Figure 31B displays the density as predicted using different DFT functionals and the corresponding exact methods (ED/VQE).

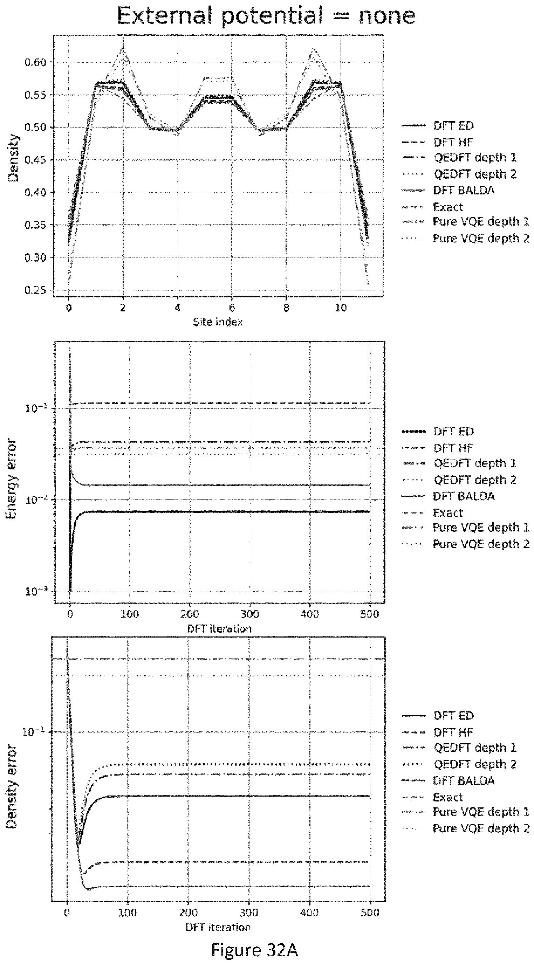

Figures 32A-C show the $1 \times 12$ Fermi Hubbard model for $U = 4$ and at quarter-filling for different external potentials (in each of 32A-C, where 32A has no external potential, 32B has a quadratic potential, and 32C has an impurity potential). The first row in each of 32A-C compares the DFT densities compared to the exact predictions. The second row in each of 32A-C monitors the convergence of the difference in total energy against the exact solution over the duration of the DFT computation. The third row in each of 32A-C monitors the convergence of the difference in total density against the exact solution over the duration of the DFT computation.

Figure 33 shows the $1 \times 12$ Fermi Hubbard model at quarter-filling with a quadratic external potential and its respective errors in energy (left panel) and density (right panel) for different interaction strengths. All simulations use DFT apart from the dashed lines.

Figure 34 shows the performance of the finite size scaling of XC functionals in the convergence of the total energy error (top panel) and density error (bottom panel) with respect to the exact solution for the quadratic external potential. The left column shows the results produced with $1 \times 6$ XC potential. The right column shows the results produced with $1 \times 12$ XC potential.

Figures 35 show the $1 \times 200$ Fermi Hubbard model for $U = 10$ and at quarter filling within a confining quadratic potential using a QEDFT functional generated on a $1 \times 12$ homogeneous Fermi-Hubbard system. Figure 35A illustrates the convergence of the total energy per lattice site within the DFT loop. Figure 35B, the density as predicted using different DFT functionals.

Figure 36 shows the $1 \times 200$ Fermi Hubbard model at quarter-filling with a quadratic external potential and its respective errors in energy (left panel) and density (right panel) for different interaction strengths. All simulations use DFT and the BALDA DFT functional is used as the exact benchmark.

Figure 37 shows the DFT energy convergence $3 \times 3$ Fermi Hubbard model for $U/t = 10$ and at quarter-filling for no external potential and an impurity potential at the (1,2) site. Dashed lines represent non DFT calculations, which are either the exact solution or pure VQE emulations.

Figure 38 shows the per site density percentage error equation **Error! Reference source not found.** for the $3 \times 3$ Fermi Hubbard model with impurity at site (1,2) for $U/t = 8$. Pure VQE methods are highlighted in the top panel, while the other methods use DFT.

Figure 39 shows the $3 \times 3$ Fermi Hubbard model at quarter-filling with an impurity potential at the (1, 2) site and its respective errors in energy (left panel) and density (right panel) for different interaction strengths. All simulations use DFT apart from the dashed lines.

Figure 40 shows the performance of the finite size scaling of XC functionals in the convergence of the total energy error with respect to the exact solution for an impurity at site (1,2). The left panel shows the results produced with $2 \times 2$ XC potential. The right shows the results produced with $2 \times 4$ XC potential.

Figure 41 shows the density per site of the $20 \times 20$ Fermi Hubbard model at half-filling at $U/t = 4$ based on DFT calculations using functionals generated on a $3 \times 3$ lattice. The external potential is an impurity potential that concentrates fermions to a square at the centre of the lattice.

Figure 42 shows the effect of system size on the XC potential for 1D and 2D Fermi Hubbard models at $U/t = 4$ run on quantum hardware and classical simulations.

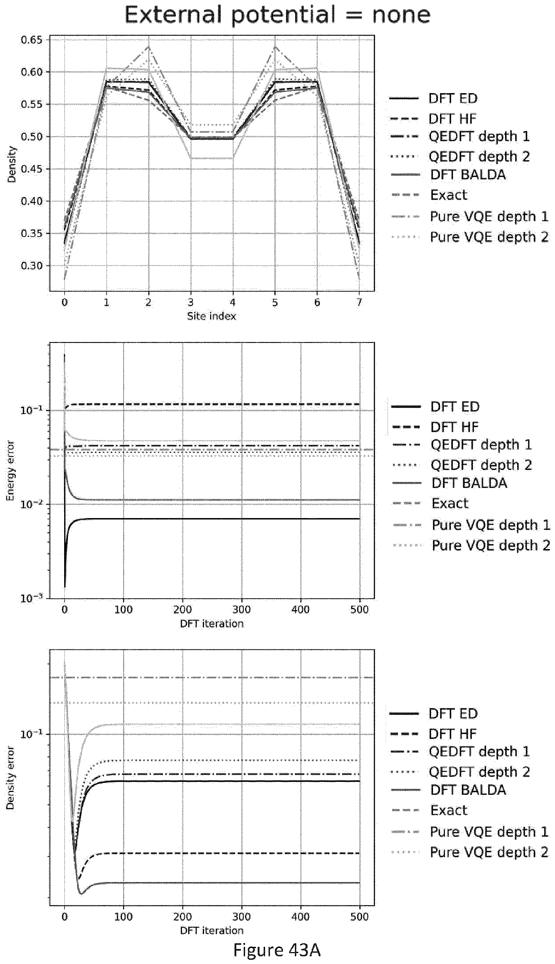

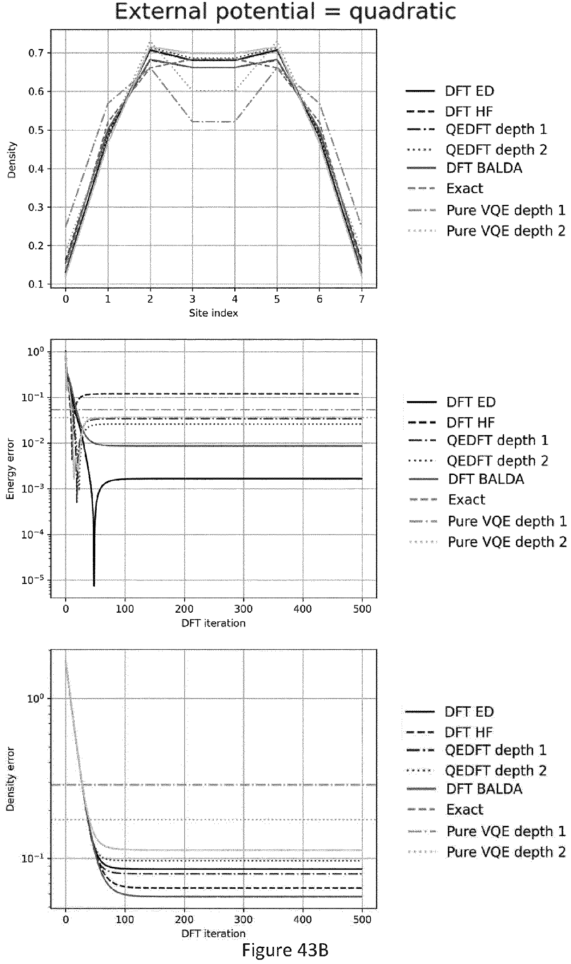

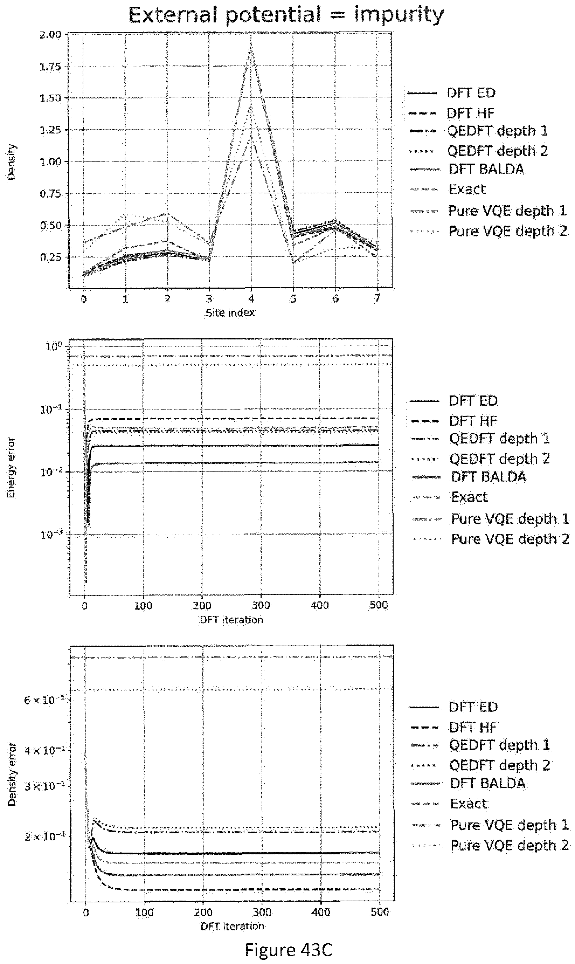

Figures 43 show the $1 \times 8$ Fermi Hubbard model for $U = 4$ and at quarter-filling for different external potentials (43A shows no external potential, 43B shows a quadratic external potential, and 43C shows an impurity potential) including results on hardware. The first (top) row in each of 43A-C compares the DFT densities compared to the exact predictions. The second (middle) row in each of 43A-C monitors the convergence of the difference in total energy against the exact solution over the duration of the DFT computation. The third (bottom) row in each of 43A-C monitors the convergence of the difference in total density against the exact solution over the duration of the DFT computation.

Figure 44 shows the $1 \times 200$ Fermi Hubbard model for $U = 4$ and at quarter filling within a confining quadratic potential using a QEDFT functional generated on a $1 \times 8$ homogeneous Fermi-Hubbard system using hardware and emulation the density as predicted using different DFT functionals.

Figure 45 shows the DFT energy convergence $2 \times 4$ Fermi Hubbard model for $U/t = 4$ and at quarter-filling for no external potential and an impurity potential at the (1,1) site using the hardware data. Dashed lines represent non DFT calculations, which are either the exact solution or pure VQE emulations.

Figure 46 shows the per site density percentage error (equation (65)) for the quarter-filled $2 \times 4$ Fermi Hubbard model with impurity at site (1,2) for $U/t = 4$ including hardware data. Pure VQE methods are highlighted in the top panel, while the other methods use DFT.

DETAILED DESCRIPTION

In this work, we introduce and benchmark a new hybrid quantum/classical approach to the many-body electronic structure problem: *quantum-enhanced DFT* (QEDFT), which combines the complementary strengths of DFT and quantum computation.

We benchmark the QEDFT algorithm on the Fermi-Hubbard model, both numerically and on data from experiments on real quantum hardware. We find that QEDFT surpasses the quality of groundstate results obtained from Hartree-Fock DFT, as well as from direct application of conventional quantum algorithms such as VQE. Furthermore, we demonstrate that QEDFT works even when only noisy, low-depth quantum computation is available, by benchmarking the algorithm on data obtained from Google's quantum computer.

We further show how QEDFT also captures quintessential properties of strongly correlated Mott physics for large Fermi-Hubbard systems using functionals generated on much smaller system sizes. Our results indicate that QEDFT can be applied to realistic materials and molecular systems and has the potential to outperform the direct application of either DFT or VQE alone, without the requirement of large scale or fully fault-tolerant quantum computers.

Rather than using the quantum computer to find the groundstate itself, we instead use the quantum computer to approximate the XC functional, which is then fed into a classical DFT iteration. Thus, the quantum computer is not tasked with finding the groundstate itself, but rather with steering the DFT iteration towards an accurate solution. In this work, we focus on using VQE for the quantum part of the QEDFT computation. But we note that our approach is general, and any other quantum groundstate approximation algorithm could be used in QEDFT.

We find that combining DFT and VQE in this way outperforms both Hartree-Fock DFT alone, and quantum VQE alone. Moreover, this remains the case even when the VQE computation itself is very noisy, and its output is far from the true XC functional. Despite the VQE output being quantitatively inaccurate, it captures key qualitative features of the exact XC functional that classical approximations struggle to reproduce. And this suffices to steer the DFT iteration to more accurate solutions than it would otherwise converge to.

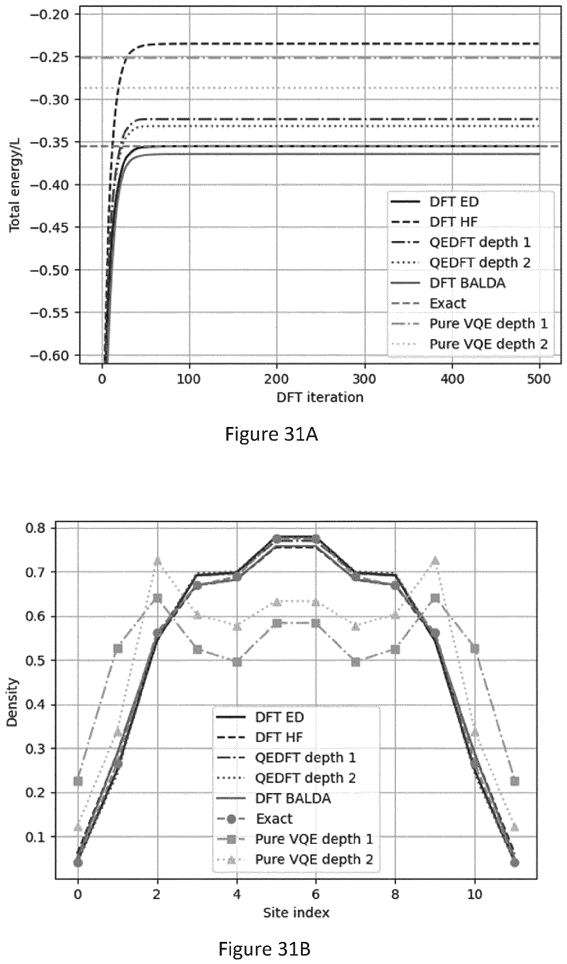

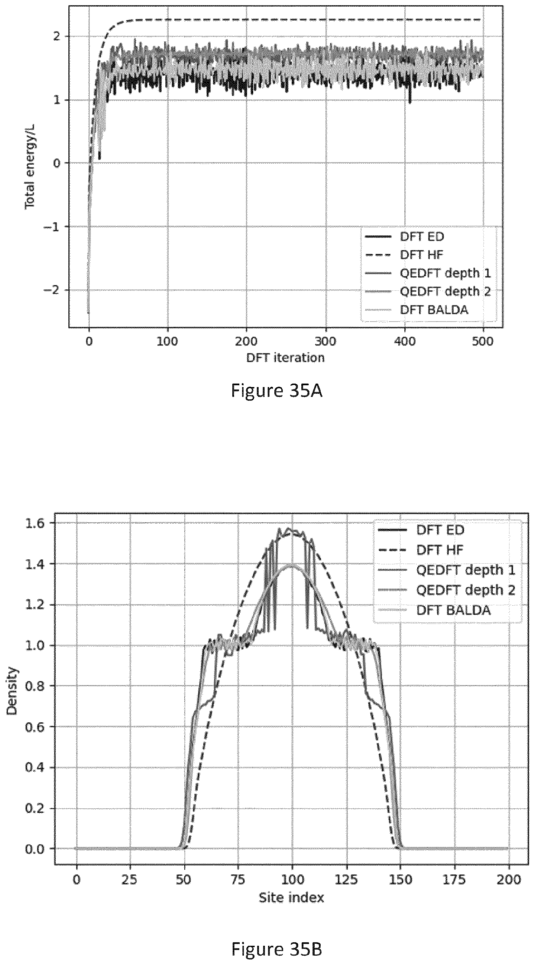

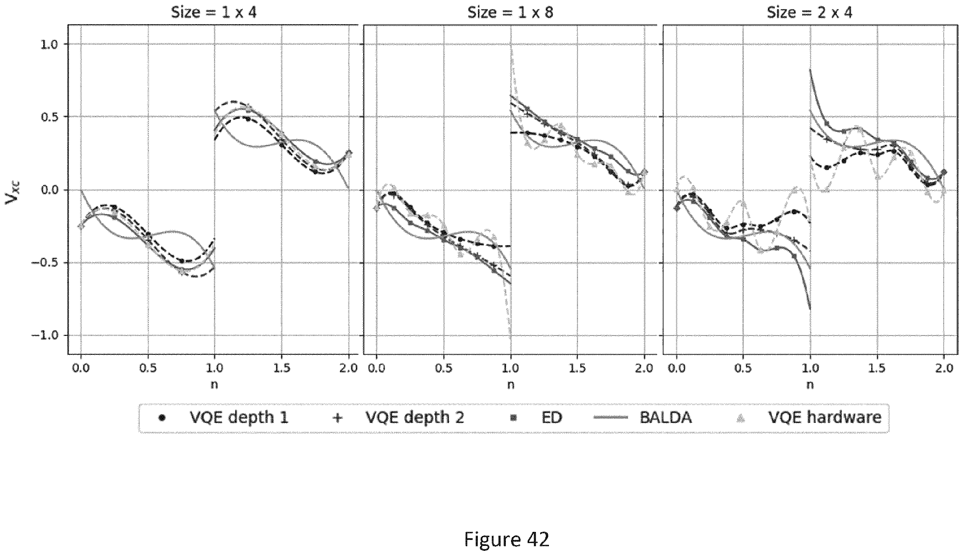

In this detailed description, all of the numerics and hardware experiments across 1d and 2d Fermi-Hubbard systems serve to support these claims. Here, we highlight a subset of our results that particularly illustrate the above advantages and improvements. Figure 31 shows that QEDFT achieves better accuracy than either DFT alone or VQE alone on the 1D Fermi-Hubbard chain. Figures 42, 43, and 45 support the claim that QEDFT succeeds even when the output of the quantum part of the computation is noisy and quantitatively inaccurate. Figures 35, 40, and 41 show results obtained for large 1D and 2D Fermi-Hubbard lattices using small quantum computations.

To benchmark the QEDFT algorithm, the evaluation of the XC functional must be transformed into a groundstate computation suitable for VQE. At a high level, there are two possible approaches: we can compute the XC functional via VQE during the DFT loop, at the density in the current DFT iteration; we call this the *online* approach. Or we can use VQE to approximate the entire universal XC functional in advance, and then use this VQE-computed XC functional in the DFT iteration; we call this the *offline* approach. There are pros and cons to each approach: the online approach only evaluates the XC functional at densities actually required during the DFT iteration; whereas the offline approach may compute it at many densities that are not actually needed. However, the online approach requires calling out to the quantum computer during the DFT computation thereby limiting the speed of the DFT computation, whereas the offline approach decouples the quantum and classical computation. Moreover, since we are approximating the universal functional, in principle the VQE functional approximation only needs to be computed once, and thereafter the same functional can be used in multiple QEDFT computations.

## Outline

We now describe the QEDFT algorithm in more technical detail and apply it to a number of many-body lattice models. Lattice models are good systems to benchmark the approach on, as they allow direct comparison to other quantum algorithms which have largely been tested in the lattice setting. Furthermore, because quantum computers consist of qubits, fundamentally any quantum simulation must be reduced in some way to a discretised model. Thus, a lattice formulation of QEDFT is preferred in any case, whether explicitly or implicitly.

In **'Lattice DFT',** we review the formulation of DFT on lattices (LDFT), which differs slightly from the more familiar continuum formulation of DFT.

In **'QEDFT',** we describe how the XC functional is transformed into a groundstate problem amenable to quantum computation. We also explain how a quantum treatment on small system sizes can be used to interpolate approximations of the XC functional, which can then be used in DFT for much larger system sizes.

As a simple illustrative example, we first show in the section **'A quantum density functional theory for the Hubbard model'** how our QEDFT approach looks for the Hubbard dimer and then extend this to more complex Fermi-Hubbard models.

The representative results are then presented in a section divided into two subsections, **'Numerical Results'** and **'Hardware Example - Google'.**

In **'Numerical Results',** we compare the performance of the QEDFT algorithm numerically with both (simulated) VQE, and with DFT under a number of choices of classical functional approximations. We also explore the qualitative features of the QEDFT functional and their contribution to the algorithm's performance.

In **'Hardware Example - Google',** we run the QEDFT algorithm on the 1D and 2D Fermi-Hubbard model using VQE data obtained on Google's quantum device.

Finally, additional information which may aid the understanding of the reader is presented in Appendix 1 and Appendix 2.

**Lattice DFT**

We consider the general Hamiltonian for the following many-body fermionic system defined on a lattice $\mathcal{L}$,

$$H = \hat{T} + \hat{H}_{int} + \hat{H}_{ext},$$

(4)

where $\hat{T}$, $\hat{H}_{int}$ and $\hat{H}_{ext}$ are the non-interacting, interacting and external field contributions respectively. DFT enables the efficient solution of this problem, under the assumptions of non-interacting v-representability of an auxiliary system to equation (4) and the linear coupling of $\hat{H}_{ext}$ to the local density, using density functionals rather than wavefunctions. The validity of the DFT approach has applicability for any Hamiltonian which linearly couples to an external field, which for example could be the electric field, but more generally takes the form

$$\hat{H}_{ext} = \sum_{i \in \mathcal{L}} v_i^{ext} \hat{n}_i,$$

(5)

where $v_i^{ext}$ is the external potential. The first Hohenberg-Kohn (HK) theorem establishes a one-to-one mapping between the groundstate electron density, $n_i = \langle \psi_{GS} | \hat{n}_i | \psi_{GS} \rangle$, and the many body groundstate wavefunction, $|\psi_{GS}[n]\rangle$, which is a functional of the density. The second HK theorem states that the groundstate electron density minimises the energy functional. It follows that the expectation value of any observable in the groundstate is uniquely defined as a functional of the groundstate density. For example, the groundstate energy functional is given by

$$E_{GS} = F[n] + \sum_{i \in \mathcal{L}} v_i^{ext} \hat{n}_i,$$

(6)

where $F[n] = \langle \psi_{GS}[n] | \hat{T} + \hat{H}_{int} | \psi_{GS}[n] \rangle$ is known as the universal functional, as it doesn't depend explicitly on the external potential. The exact analytical form of this functional is not known for many fermionic systems of interest and requires the solution of many body problems that do not scale efficiently in the system size on classical computers. Routine approximations of $F[n]$ are made to allow for practical implementations of DFT. The error in the estimation of groundstate observables depends on the quality of those approximations. A necessary reduction is then made from the original problem to an auxiliary non-interacting (KS) Hamiltonian that is guaranteed to reproduce the groundstate properties of the original system only if the exact universal functional is used. This step is valid only if the original system is non-interacting v-representable, i.e the groundstate density of the original interacting system can be obtained from a non-interacting one. Within this scheme, known as the KS approach, equation (6) is written as,

$$E_{GS}[n] = T_{NI}[n] + U_H[n] + E_{XC}[n] + \sum_{i \in \mathcal{L}} v_i^{ext} \hat{n}_i,$$

(7)

where $T_{NI}[n]$ is the kinetic energy of the auxiliary non-interacting system, $U_H[n]$ is the Hartree energy, and $E_{XC}[n]$ is the exchange-correlation energy functional. By minimising equation (7), the Euler-Lagrange equations result in,

$$\frac{\partial E_{GS}}{\partial n_i} = \frac{\partial T_{NI}}{\partial n_i} + \frac{\partial U_H}{\partial n_i} + \frac{\partial E_{XC}}{\partial n_i} + v_i^{ext} = 0,$$

(8)

which has a solution that is the groundstate density. This equation is equivalent to a non-interacting system with an effective potential defined as,

$$V_i^{KS} = v_i^{ext} + \frac{\partial U_H}{\partial n_i} + \frac{\partial E_{XC}}{\partial n_i}$$
$$= v_i^{ext} + V_H[n_i] + V_{XC}[n_i] \,,$$

$$(9)$$

where $V_H[n_i]$ is the Hartree potential and $V_{XC}[n_i]$ is the exchange correlation potential, which is the object that is typically approximated. The effective non-interacting single-particle system is known as the KS Hamiltonian,

$$\hat{H}_{KS} = \hat{T} + \sum_{i \in \mathcal{L}} V_i^{KS} \hat{n}_i \,.$$

$$(10)$$

This system of equations is then solved self-consistently where at each iteration the groundstate density at site index i is constructed from the eigenvectors of the KS system, by summing over the occupied single particle states of the KS eigenvectors (accounting for spin),

$$n_{GS,i} = 2 \sum_{j}^{occ/2} |\phi_j(i)|^2 \,,$$

$$(11)$$

where $\hat{H}_{KS}\phi_j = \varepsilon_j\phi_j$ is the full eigensolution of the KS system. Here we treat the example spin unpolarised lattice DFT, but we note that spin polarised lattice DFT can be treated by separating the Kohn-Sham states into their up and down spin channels.

The groundstate energy of the original many-body system in terms of the KS eigenvalues and density is given by,

$$E_{GS}[n_{GS}] = 2 \sum_{i \leq occ/2} \epsilon_i - \sum_{i \in \mathcal{L}} V_{XC}[n_{GS,i}]n_{GS,i} - E_H[n_{GS}] + E_{XC}[n_{GS}] \,.$$

$$(12)$$

The KS solution is converged self-consistently by iteratively updating the input density by computing equation (11) and mixing it with the previous density estimate,

$$n^{(j+1)} = \alpha n^{(j)} + (1 - \alpha)n^{(j+1)}$$

$$(13)$$

where j is the DFT iteration index and $\alpha$ is a linear mixing parameter that is used for numerical stability. When the groundstate observables no longer change up to a tolerance $\delta$ then the groundstate energy and density are equivalent to those of the interacting problem, if the true XC energy functional is used.

To summarise, the groundstate density minimises the total energy functional which enforces the functional derivative to be zero and results in a variational equation for auxiliary wavefunctions that can construct the groundstate density. This equation takes the form of a single-particle equation which can be treated as a standard eigenvalue problem. All of many-body physics manifests in the exchange correlation potential contribution of an otherwise fully determined effective single particle potential. These equations are solved self consistently, as the XC potential depends on the density, and the density depends on the unknown auxiliary wavefunctions. At self consistency, these wavefunctions no longer change and are used to construct groundstate observables, most notably the density and energy. The algorithmic procedure is presented below in **Table 1.**

**Table 1:** The algorithmic procedure for executing a DFT loop for practical applications.

| **Algorithm 1:** The lattice DFT algorithm |
| --- |
| Choose the inhomogeneous representation (i.e the external potential) of the fermionic system to be used, equation **(4).** |

1. Choose initial density;
2. Construct the KS potential equation (9) where $v_{XC}$ is approximated;
3. Solve for $(\phi_j, \varepsilon_j)$ in the KS system of equations $\hat{H}_{KS}\phi_j = \varepsilon_j\phi_j$ with $H_{KS}$ equation (10);
4. Construct density estimate from the KS eigenvectors, equation (11);
5. Compute energy of the system, equation (12);
6. Check for system convergence;
7. If converged, exit loop and compute groundstate properties.

If not, go back to step 1 and use new estimate of density as the input density, equation (13)

**QEDFT**

In this section we present the quantum-enhanced DFT algorithm for fermionic systems within the lattice DFT formalism presented in the previous section. The main difference between lattice DFT and normal (ab initio) DFT is that the former is discrete, while the latter uses continuous basis sets.

The QEDFT algorithm consists of two parts (a) the computation of the XC potential and energy using a quantum machine and (b) application of the potential and energy within a DFT loop, which essentially follows the steps outlined in the section Lattice DFT. As an example, we discuss QEDFT in its offline mode, where the quantum computer is called during the first stage only and the second stage is a purely classical computation that queries results from the first stage. Our approach is motivated by the previously discussed deficiencies of classical DFT approximations to systematically incorporate the effects of exchange and correlations. In contrast, quantum computations natively include these effects, so building functionals out of their computations has the potential to improve upon classical approximations.

In **Table 2** we describe the algorithmic procedure for computation of the quantum-enhanced XC (QE-XC) potential and energy in this scheme. The initial step of the problem is to choose a homogeneous system of size $L$. The homogeneous Hamiltonian is related to the inhomogeneous Hamiltonian of equation (4) by setting $\hat{H}_{ext} = 0$. Its groundstate energy for density $n = \{n_1, \ldots, _{nL}\}$ is given by the universal functional F[n = $\{n_1, \ldots, n_L\}$], where $\Sigma_i n_i = N$, corresponds to the total number of electrons in the system. The fermionic Hamiltonian of $L$ sites is then encoded into a $M = \mathcal{O}(2L)$ qubits Hamiltonian via a fermion-to-qubit mapping,

$$H^{\mathrm{fermions}} = \sum_k h_k \rightarrow H^{\mathrm{qubits}} = \sum_\alpha \lambda_\alpha P_\alpha \, ,$$

$$(14)$$

where $P_\alpha \in \{I,X,Y,Z\}^{\otimes M}$ are Pauli operators and $\lambda_\alpha$ are real coefficients. This mapping is necessary due to how operations are executed on the quantum device and its choice depends on the system under consideration. A quantum circuit representation $|\Psi_{QC}[n]\rangle$ is then created that can be used by quantum algorithms to predict the groundstate energy of the system for a fixed number of fermions. The variational quantum eigensolver (equation (3)) can be used for this step, where a quantum circuit is parameterised by real parameters that are variationally optimised for. At each allowed fermionic filling, the quantum algorithm is run to compute the groundstate energy,

$$E(n) = \left\langle \Psi_{QC}[n]\right|H^{\mathrm{qubits}}\left|\Psi_{QC}[n]\right\rangle \text{ where } \sum_i n_i = N_e \, , \quad \forall N_e \in \{0, \ldots, 2L\} \, .$$

$$(15)$$

At this stage, there is still the combinatorially many possibilities for the densities at each site of the lattice $\mathcal{L}$ as $n = \{n_1, \ldots, n_L\}$. To further reduce the complexity we employ the local density approximation, where we just scan over states with total density $N_e/L$. This naturally fits in the state preparation of $|\Psi[n]\rangle$ as the total density is a conserved quantity, so the VQE minimization rearranges the local density profile while maintaining $N_e$.

From now on, within the offline QEDFT mode all computations are executed on a classical machine that post-processes the function F(n) with a spacing 1/2L. We introduce the quantum-enhanced local density approximation $\varepsilon_{QELDA}$

for the energy equation (15),

$$\epsilon_{QELDA}\left(\frac{N_e}{L}\right) = \frac{E(n)}{L}, \quad \text{s.t} \quad \sum_i n_i = N_e,$$

(16)

over the domain $\frac{N_e}{L} \in [0,2]$. Therefore, as the system size is increased, the number of grid points that can be accessed by $\epsilon_{QELDA}\left(\frac{N_e}{L}\right)$ increases and the resolution of the local energy increases. The local XC energy can also be obtained via,

$$\epsilon_{QELDA-XC}\left(\frac{N_e}{L}\right) = \epsilon_{QELDA}\left(\frac{N_e}{L}\right) - \epsilon_{HF}\left(\frac{N_e}{L}\right)$$

(17)

where $\epsilon_{HF}\left(\frac{N_e}{L}\right)$ is the local Hartree Fock (HF) energy which can be obtained efficiently on a classical machine. In this representation, the total exchange correlation energy is approximated as a linear combination of the local energy generated by the local density at each site, i.e.

$$\epsilon_{QE-XC}(n) = \sum_{i\in\mathcal{L}} \epsilon_{QELDA-XC}(n_i).$$

(18)

The QE-XC potential is then obtained by taking the numerical derivative of equation (18),

$$V_{QE-XC}(n_i) = \frac{\partial E_{QE-XC}(n)}{\partial n_i}\bigg|_{n=n_{GS}}.$$

(19)

As equation (19) and equation (17) are only known in the domain [0,2] with grid spacing 1/2*L* it is necessary to generate a continuous representation of it so that it can be queried in a DFT loop. This can be achieved by performing a spline interpolation for a fixed polynomial order. As a result of this interpolation, the functional can be used for target systems which are not the ones used to generate it. For example, a functional which is generated on a 2D system of size 3 × 3 can be used for a target system of size 30 × 30. The DFT loop then proceeds as described in section 'Lattice DFT' for any Hamiltonian system given by equation (4), where the form of the XC potential and energy used in the DFT query is given by the continuous representations of equation (18) and equation (17).

| **Algorithm 2:** Obtaining the quantum-enhanced XC potential and energy |
|---|

Choose system of interest and construct its homogeneous fermionic Hamiltonian representation, i.e. equation **(4)** where $\hat{H}_{\text{ext}} = \mathbf{0}$;

1. Encode the fermionic Hamiltonian into qubits via a fermion-to-qubit mapping equation (14);

2. Create a quantum circuit representation for computation of groundstate energy;

3. Use a quantum computer to compute the groundstate energy at each allowed fermionic filling (equation (15));

4. Use a classical computer to obtain the exchange correlation energy equation (18);

5. From the exchange correlation energy, compute the exchange correlation potential equation (19);

6. Interpolate the exchange correlation potential and energy to obtain continuous representations and store on a classical computer;

7. Use exchange correlation potential and energy in the DFT loop (**Table 1**) for any inhomogeneous fermionic Hamiltonian of the form equation (4).

**Table 2:** The algorithmic procedure to generate a classical representation of a quantum-enhanced XC potential and energy.

## QEDFT Examples

In this section, we present some examples for how the QEDFT algorithm can be applied to the Fermi-Hubbard model.

## A quantum density functional theory for the Hubbard model

In this work, we approach the problem of XC functional design using quantum algorithms running on quantum computers.

Here we present a general hybrid-quantum algorithm in the context of materials simulation. It is applied to lattice models where it systematically uses VQE data to parametrise the functional form of the exchange correlation potential that is incorporated within a classical iterative Kohn-Sham scheme. By doing so, we show that in this hybrid quantum-classical DFT approach can be more accurate than the gold standard classical mean field approaches.

By way of example, we deal with materials and/or molecules which can be represented by fermionic lattice models such as the Fermi-Hubbard model. We present this approach in the context of the Inhomogeneous Hubbard Model for varying system sizes. With this technique it is possible to use quantum simulated data from low depth circuits and for small lattice sizes and then encode that information into the exchange correlation functional that be used for much larger system sizes, i.e., it is independent of system size. In doing so, it is also possible to systematically improve the approximation by extrapolating away finite sizes effects, incorporate constraints that must be respected and improve with deeper circuits. For materials systems believed to be appropriately modelled by lattice Hubbard Models this technique can efficiently solve much larger systems than with classically exact methods, in challenging regimes such as defect-driven phenomena and strongly correlated interactions.

Near-term hybrid-quantum algorithms that can compute the ground-state properties for fermionic systems have been produced. Our method uses quantum measurements from a state prepared on a quantum computer, that approximates the ground state of the system to establish a systematic scheme that computes exact exchange correlation functionals. To illustrate the technique, we use the Variational Quantum Eigensolver applied to the homogeneous and inhomogenous Hubbard model as a function of system size, lattice connectivity, electron doping, external potential and interaction strength. We show that low-depth VQE calculations of small system sizes are able to parametrise approximations of the exchange correlation functional, which can be used for systems of equivalent dimension but also much larger sizes. Furthermore, these functionals are used in the variational self-consistent scheme of Kohn Sham to produce the ground state properties of the interacting many-body system to a better approximation than classical mean field approaches in particular parameter regimes. Finally, we also illustrate how our approach can be extended to larger systems with varying connectivity structures that incorporate multiorbital electronic interactions.

**Application to the Hubbard dimer**

Here we will describe each of the necessary steps required for implementing the QEDFT algorithm for the prototypical $1 \times 2$ inhomogeneous Fermi-Hubbard model at half-filling, also referred to herein as the Hubbard dimer.

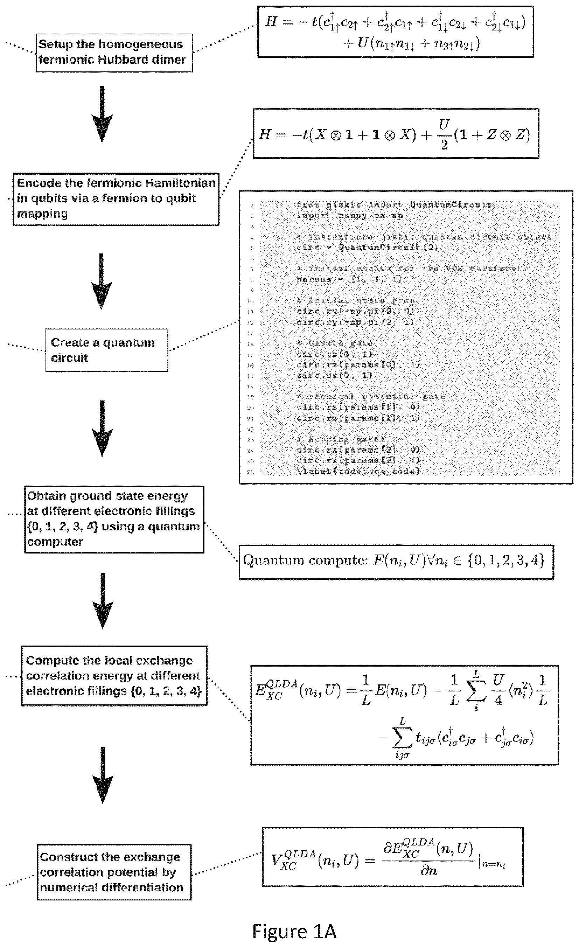

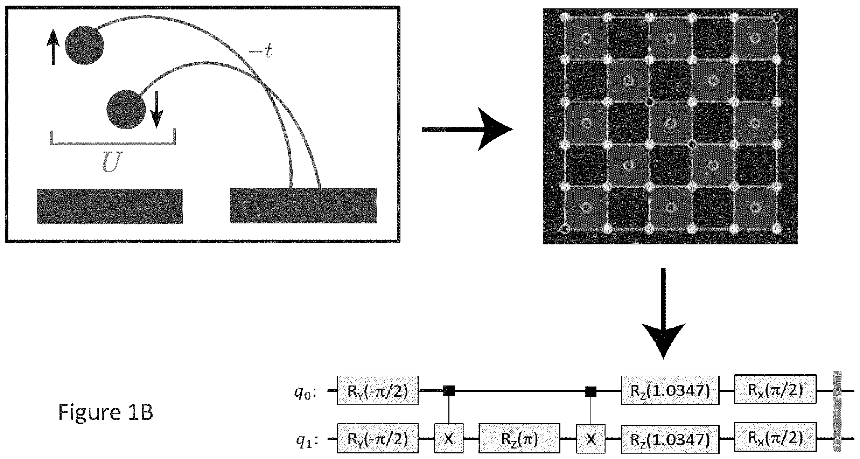

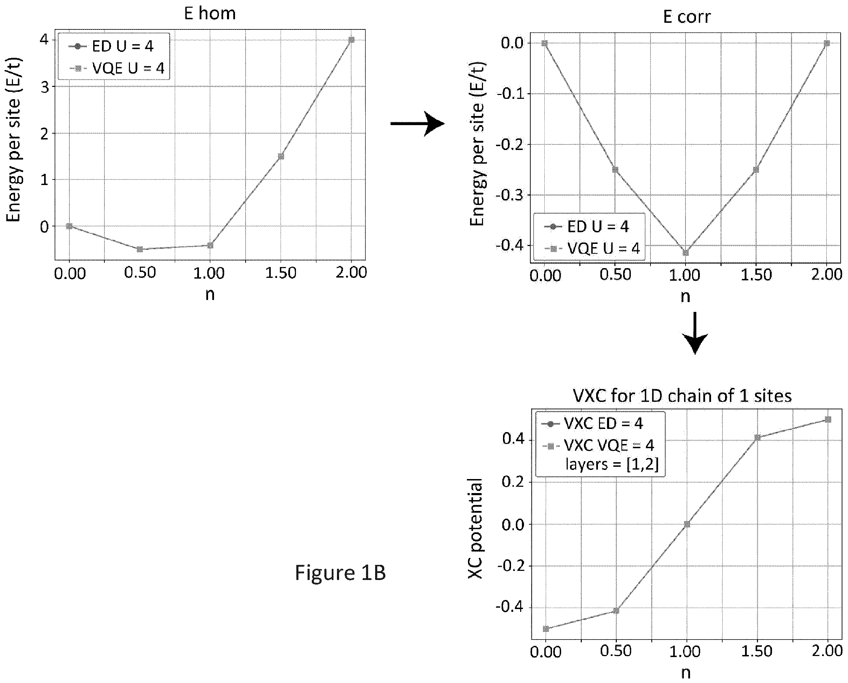

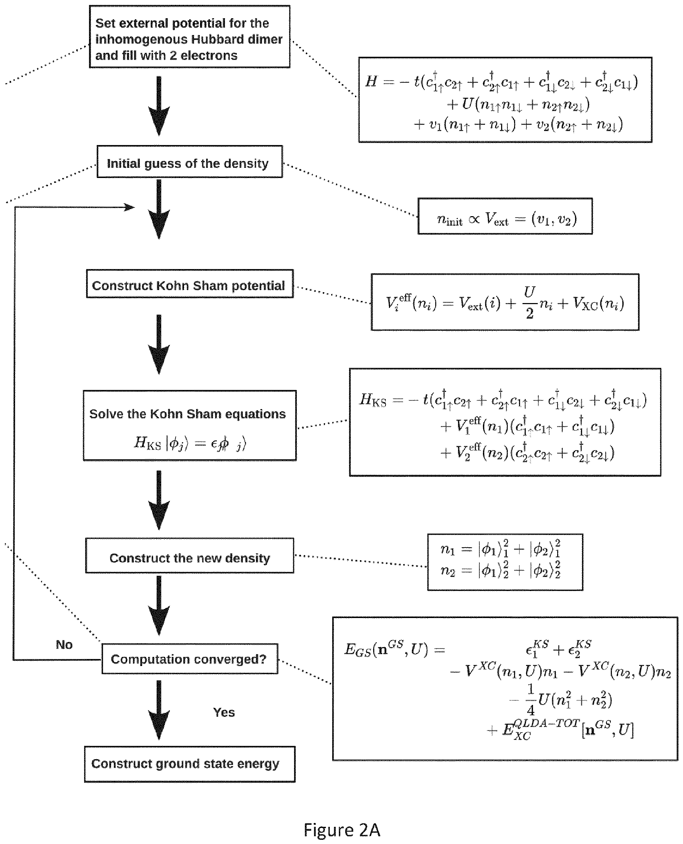

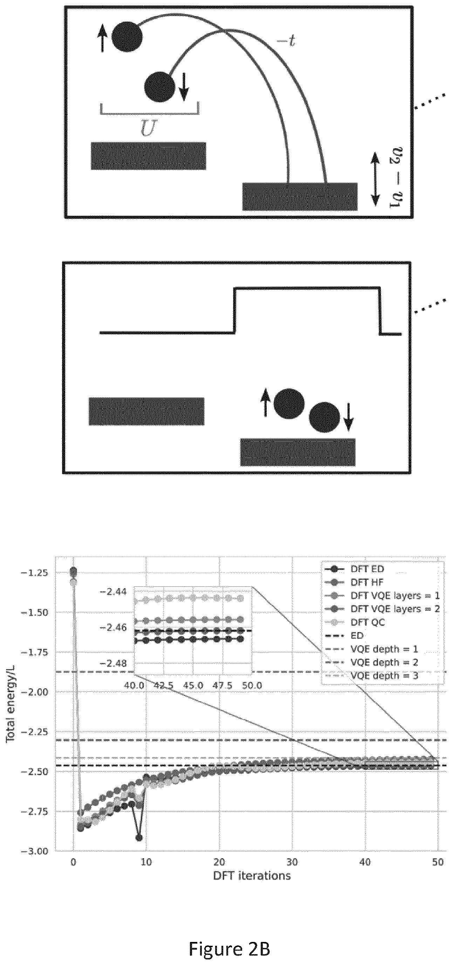

The Hubbard dimer is the simplest model to which this process can be applied. In Figures 1A and 1B we show the process within a specifically designed flow chart for the Hubbard dimer of how to compute the XC potential, while in Figures 2A and 2B we show how to incorporate that functional into a classical DFT loop for the dimer.

Taking the process step by step, we have that:

(a) The ground state energy $E_{dimer}$ of the Hubbard dimer (e.g. the second Hamiltonian),

$$H_{dimer} = -t \sum_{i\sigma}^{1} \left( c_{i,\sigma}^{\dagger} c_{i+1,\sigma} + c_{i+1,\sigma}^{\dagger} c_{i,\sigma} \right) + \sum_{i=1}^{2} U n_{i,\uparrow} n_{i,\downarrow}$$

(20)

This second Hamiltonian is the homogeneous Hubbard Dimer Hamiltonian that is required by the QEDFT algorithm **(Table 2)** which is equivalent to the general equations (4) and (45), and specific dimer equation (32), without the external potential, equation (20) can also be written in the form given by

$$H_H = -t \left( \hat{c}_{1\uparrow}^{\dagger} \hat{c}_{2\uparrow} + \hat{c}_{2\uparrow}^{\dagger} \hat{c}_{1\uparrow} + \hat{c}_{1\downarrow}^{\dagger} \hat{c}_{2\downarrow} + \hat{c}_{2\downarrow}^{\dagger} \hat{c}_{1\downarrow} \right) + U \left( \hat{n}_{1\uparrow} \hat{n}_{1\downarrow} + \hat{n}_{2\uparrow} \hat{n}_{2\downarrow} \right),$$

(21)

where $H_H$ stands for homogeneous Hamiltonian, and the sums of equation (20) are expanded.

(b) The ground state energy $E_{dimer}$ of the Hubbard dimer (the second Hamiltonian), eq. (20) is transformed from a complex fermionic representation to Pauli representation via a fermionic encoding, in this case we choose a Jordan Wigner, which in the compressed representation equals

$$H = -t(X \otimes \mathbf{1} + \mathbf{1} \otimes X) + \frac{U}{2}(\mathbf{1} + Z \otimes Z)$$

(22)

where $X, Z$ are Pauli matrices, which can be decomposed into native hardware gates on a quantum device, and $1 = I$ is the identity operator. These steps are illustrated in the first 3 panels of Figure 1.

Following the algorithmic protocol presented in **Table 2,** the Fermi-Hubbard dimer is first transformed from a complex fermionic representation (in terms of creation and annihilation operators, $c_{i,\sigma}$ and $c_{i,\sigma}^{\dagger}$, and spin density operators, $n_{i,\uparrow}$ and $n_{i,\downarrow}$) to qubit representation (in terms of quantum operators - also referred to as quantum gates - which act on qubits) via a fermionic encoding. Using the Jordan-Wigner encoding and starting from the expanded form of the Hamiltonian in equation (21), the terms in the Hamiltonian are transformed as follows:

$$\hat{c}_i^{\dagger} \hat{c}_j + \hat{c}_j^{\dagger} \hat{c}_i \mapsto \frac{1}{2} \left( X_i X_j + Y_i Y_j \right) Z_{i+1} \dots Z_{j-1}, \quad \hat{n}_i \hat{n}_j \mapsto \frac{1}{4} (I - Z_i)(I - Z_j),$$

(23)

assuming each spin-orbital has been assigned a unique index with a chosen Jordan-Wigner ordering (e.g. all up orbitals, followed by all down with the lattice sites in a snake ordering). In the specific case of the half-filled dimer, it is possible to reduce the 4-qubit representation of the Fermi-Hubbard Hamiltonian into a 2-qubit one as only four amplitudes in the statevector are non-zero. The resulting compressed representation is given by equation (22) above, and is also be written in the form:

$$H_C = -t(X \otimes I + I \otimes X) + \frac{U}{2}(I + Z \otimes Z)$$

$$(24)$$

where $H_C$ stands for the Hamiltonian in a compressed representation, since $\mathbf{1} = I$.

(c) Equation (22) is then solved and normalised by the number of sites using a quantum computer by executing a VQE circuit which can be defined through code similar to the circuit depicted in Figure 1, for which we must choose an ansatz, in this case the Hamiltonian Variational Ansatz. It has been shown in numerical simulations that one layer of the HVA is required to find the groundstate of this model using VQE. Therefore, the state that we prepare using VQE is the following:

$$|\psi(\theta, \phi)\rangle = e^{i\theta(X \otimes I + I \otimes X)} e^{i\phi(I + Z \otimes Z)}|\psi_0\rangle,$$

$$(25)$$

where $\theta$, and $\phi$ are variational parameters, and $|\psi_0\rangle = |-\rangle \otimes |-\rangle$ is the groundstate of the compressed Hamiltonian, $H_C$ with $U = 0$.

The quantum portion of VQE computation requires two important specifications: (1) a fermion-to-qubit mapping and (2) a variational ansatz which comprises an initial state and circuit. Each of these considerations is related to the generated control signals.

The encoding defines how fermion operators are mapped into qubit operators which manifest as gates (quantum operations) in the quantum circuit. In our discussions we have chosen to use the conventional Jordan Wigner mapping which is a standard fermion-to-qubit mapping and associates each fermionic mode with a qubit. For example, if we consider the Hubbard dimer and just the interaction term on the first lattice site, we have that the fermionic Hamiltonian transforms as

$$U \times \hat{n}_{1\uparrow}\hat{n}_{1\downarrow} \rightarrow \frac{U}{4} \times Z \otimes Z,$$

$$(26)$$

where $Z$ is the Pauli Z matrix.

There are different possible VQE ansatze which may be used; in this example the popular Hamiltonian Variational Ansatz (HVA) for VQE simulations is chosen. For HVA, the initial state can be prepared from the $U = 0$ ground state of the Hubbard model, which comprises of a layer of depth $n_x n_y - 1$ Givens rotations acting on adjacent modes, and its decomposition into signals depends on the native gates available on the hardware device. $n_x$; $n_y$ are the system dimensions because here we consider 2d. After the initial state has been created in the circuit, a representation of the VQE circuit is created which consists of a number of parametrised quantum gates. We illustrate this by returning to the Hubbard dimer example and specifically analysing the gate decomposition of the $\frac{U}{4} \times Z \otimes Z$ term in the HVA circuit. The task that we need complete is to represent the following term in the circuit: $e^{i\theta \times U/4 \times Z \otimes Z}$ as a quantum gate, where $\theta$ is a VQE parameter which is initialised and updated using classical optimisers. This precisely probes how this term is translated into a control signal. Ultimately, this depends on the native gates of the device, so there is no general answer on how to do this. However, a realistic practical assumption may be that CNOT and RZ gates are native on the hardware device. If so, then any then the above $e^{i\theta \times U/4 \times Z \otimes Z}$ terms can be decomposed into a sequence of CNOT and RZ gates. Moreover, this is also an operational way in which the aforementioned Givens rotations can be decomposed into signal controls for a device.

(d) For each fermionic filling the groundstate energy at a fixed $U/t$ needs to be computed, i.e $E(N_e, U/t) \forall N_e \in \{0, 1, 2, 3, 4\}$. For $N_e = 2$, VQE with the ansatz in equation (25) can be used. For $N_e = 0, 4$, the number sector is one dimensional and so a quantum computer does not need to be used. The energy for $N_e = 1$ can also be calculated classically using the observation that there are no interacting $U$ terms which reduces the problem to one with hopping terms only. A similar argument can be used in the symmetric case of $N_e = 3$. The calculation of $E(N_e, U/t)$ allows then for the local XC energy to be calculated using:

$$\epsilon_{QEDFT-XC}\left(\frac{N_e}{2}, U/t\right) = \epsilon_{QEDFT}\left(\frac{N_e}{2}, U/t\right) - \epsilon_{MF}\left(\frac{N_e}{2}, \frac{U}{t}\right) \quad \forall N_e\{0, 1, 2, 3, 4\},$$

$$(27)$$

where the QEDFT energy, $\varepsilon_{QEDFT}$, shown in the fourth horizontal panel of Fig. 1, is found by obtaining the following energies on a quantum machine, that is:

$$\epsilon_{QEDFT}\left(\frac{N_e}{2}, U/t\right) = \frac{E(N_e/2, U/t)}{4} \quad \forall N_e \in \{0, 1, 2, 3, 4\}$$

$$(28)$$

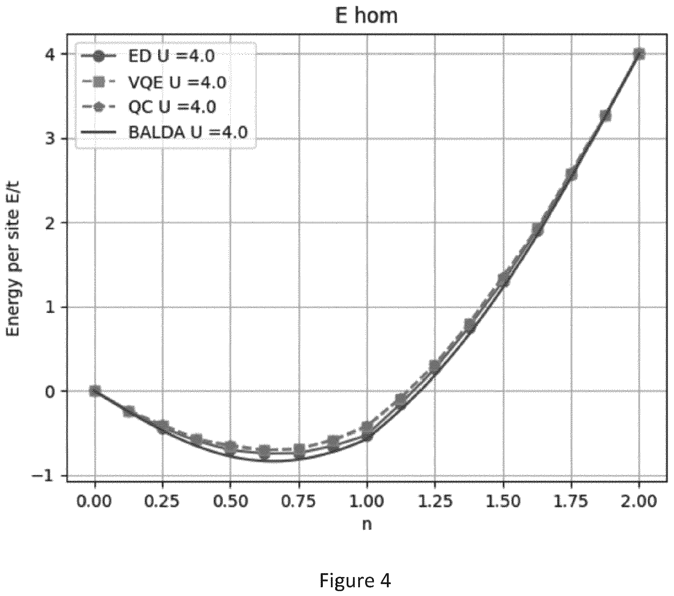

In other words, the Hubbard dimer has 2 lattice sites, so it can at most have 4 fermions, which means the quantum computer must produce 4 data points (5 including the empty state where there are no fermions), these 4 data points are plotted as a function of the fractional fermion filling, as highlighted in Fig. 1 within the fourth horizontal panel. We note here that for larger systems, the approach is the same, but the density mesh is denser, due the number of fillings allowed being greater. A result which emphasises this is presented in Fig. 4, which has 16 data points (representing a $1 \times 8$ system) instead.

In Fig 4 we see that noisy QC data can fall within 1% of in particular parameter regimes of the Hubbard model. This is in contrast to the classical mean field results, which fall outside of this 1% interval.

(e) Using equation (27), the mean field energy, and the energies calculated from the quantum machine, the local exchange correlation energy can be found. The mean field energy can be computed classically by solving the non-interacting homogeneous Fermi-Hubbard model and computing:

$$\epsilon_{MF}\left(\frac{N_e}{2}, \frac{U}{t}\right) = \frac{U}{2}\left(\langle\hat{n}_1\rangle^2 + \langle\hat{n}_2\rangle^2\right) - t\langle\left(\hat{c}_{1\uparrow}^{\dagger}\hat{c}_{2\uparrow} + \hat{c}_{2\uparrow}^{\dagger}\hat{c}_{1\uparrow} + \hat{c}_{1\downarrow}^{\dagger}\hat{c}_{2\downarrow} + \hat{c}_{2\downarrow}^{\dagger}\hat{c}_{1\downarrow}\right)\rangle \quad \forall N_e \in \{0, 1, 2, 3, 4\}.$$

$$(29)$$

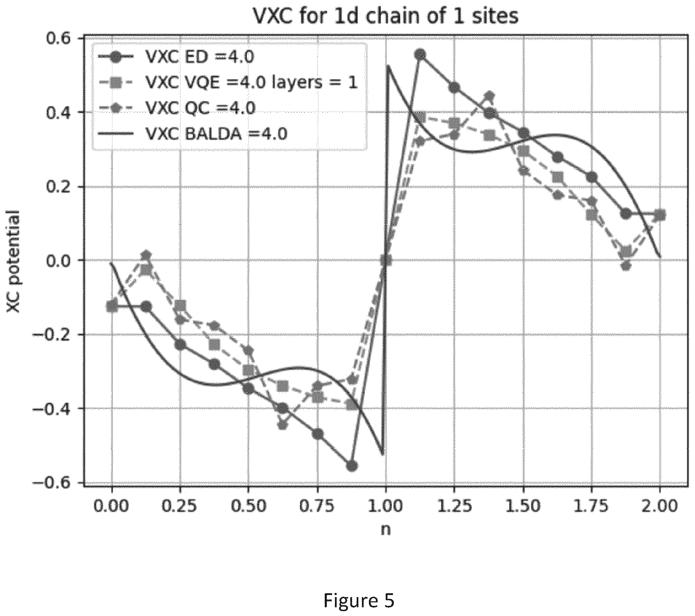

These 4 data points are then used to create the exchange correlation energy, as shown in the 5th panel of Figure 1B. Likewise, Fig. 5 shows the XC potential calculated from Fig. 4, and shows that this approach does reasonably well at capturing qualitative features of the XC potential as compared to exact classical methods (ED and BALDA).

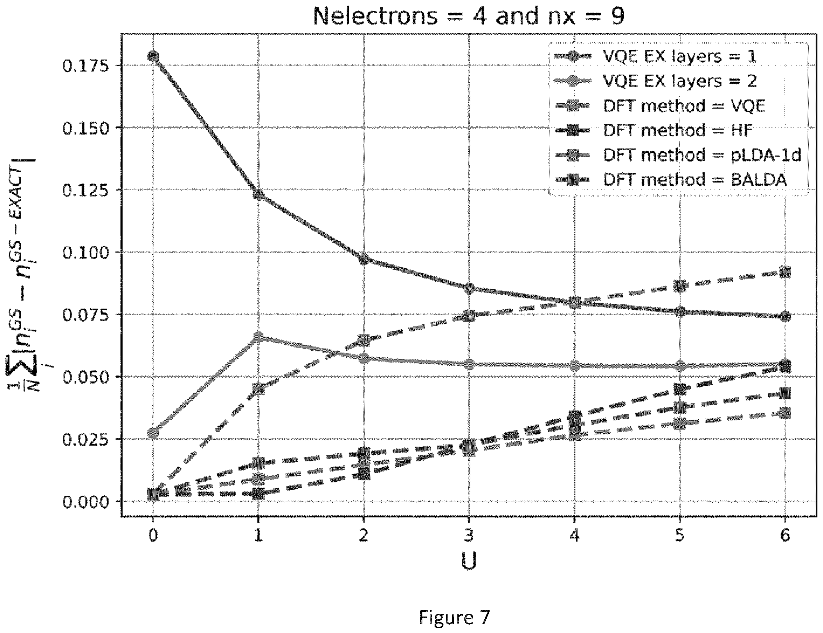

Systematically, we see that for strongly correlated systems ($\frac{U}{t} > 1$) the estimation of the density is also well described, as in Fig. 7.

(f) Then, the QEDFT XC potential is found by taking the numerical derivative of equation (27) with respect to the density, as shown in equation (19).

In other words, the functional is created from these 4 points in the XC energy to give $V_{XC}^{QLDA}(n_i, U)$, as shown in the 6th and final panel of Figure 1B, and further outlined in Section 6.2.1. This representation can be obtained through polynomial splining procedures, for example.

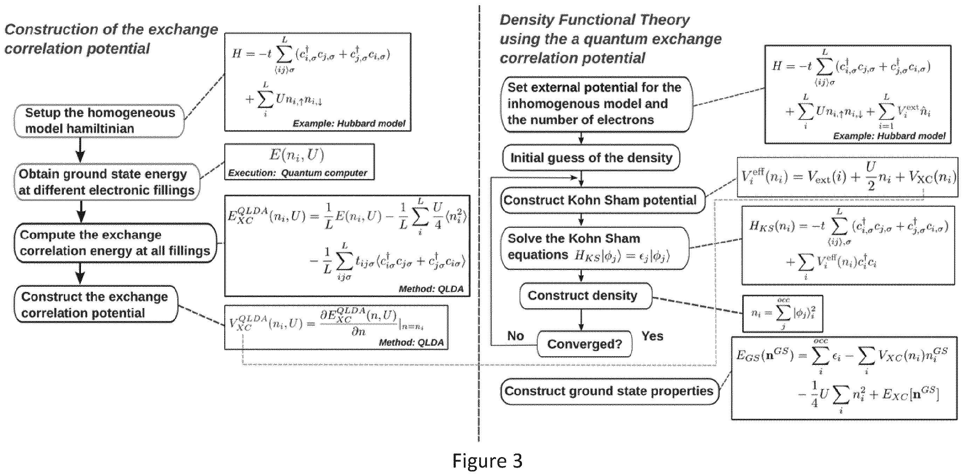

Finally, the QEDFT XC potential is interpolated (not shown in Figure 1) so that it can be queried at any density between 0 and 2. This concludes the first phase of applying QEDFT to the Fermi-Hubbard dimer which creates a classical queryable function that can be used in a DFT computation. This function is then stored and now can be used in a classical DFT loop, which remains the same as the right hand side of Fig. 3 for a specific choice of dimension and external potential $V_{ext}(i)$. That is to say, with reference to equation (10), that the following set of Kohn Sham equations for the Fermi-Hubbard dimer are solved for in a self consistent manner,

$$H_{KS}(n_i) = -t\sum_{\langle ij\rangle}^{L}\left(c_i^{\dagger}c_j + c_j^{\dagger}c_i\right) + \sum_i V_i^{eff}(n_i)c_i^{\dagger}c_i$$

$$(30)$$

where

$$V_i^{\text{eff}}(n_i) = V_{\text{ext}}(i) + \frac{u}{2}n_i + \frac{dF(\mathbf{n})}{dn_i}$$

(31)

and $\dfrac{dF(\mathbf{n})}{dn_i}$ is the unknown multivariate exchange correlation potential approximated to produce an approximation to the ground state energy and density.

Now, having a continuous representation for the XC energy and potential we can return to solving the prototypical $1 \times 2$ inhomogeneous Fermi-Hubbard model at half-filling, i.e., the inhomogeneous Hubbard Dimer given by equation (32) below with lattice DFT according to the algorithm protocol of **Table 1**. Figures 2A and 2B show how to incorporate the functional from the processes in Figures 1A and 1B (and described in a to f above) into a classical DFT loop which solves a model Hamiltonian for the dimer, in this case the inhomogeneous Fermi-Hubbard model.

(g) That is, in more detail, in the first panel of Figs 2A and 2B we start by setting an external potential, which in this case is only defined at two lattice points, i.e., $(v_1, v_2)$, as we are dealing with the dimer. This may be thought of as the model Hamiltonian that the whole QEDFT algorithm solves, i.e., the model Hamiltonian that QEDFT solves is the inhomogeneous Fermi-Hubbard Hamiltonian, in this example case restricted to two sites:

$$\begin{aligned}
H_{IH} = &-t\big(\hat{c}_{1\uparrow}^{\dagger}\hat{c}_{2\uparrow} + \hat{c}_{2\uparrow}^{\dagger}\hat{c}_{1\uparrow} + \hat{c}_{1\downarrow}^{\dagger}\hat{c}_{2\downarrow} + \hat{c}_{2\downarrow}^{\dagger}\hat{c}_{1\downarrow}\big) \\
&+ U(\hat{n}_{1\uparrow}\hat{n}_{1\downarrow} + \hat{n}_{2\uparrow}\hat{n}_{2\downarrow}) \\
&+ v_1(\hat{n}_{1\uparrow} + \hat{n}_{1\downarrow}) + v_2(\hat{n}_{2\uparrow} + \hat{n}_{2\downarrow}),
\end{aligned}$$

(32)

where $U$ is the interaction strength, $t$ is the hopping amplitude, and $\{v_1, v_2\}$ are the chemical potentials for the different sites that constitute the external potential. This form of the inhomogeneous Hamiltonian corresponds to that of equation (4) for two sites.

(h) Firstly, the density must be initialised and a common choice is to make it such that it is proportional to the external potential:

$$n_{\text{init}} \propto V_{\text{ext}} = (v_1, v_2),$$

(33)

where $\{v_1, v_2\}$ are the chemical potentials as mentioned previously. So, the KS potential is then initialised for the two lattice sites:

$$\begin{aligned}
V_1^{KS}(n_1) &= v_1 + \frac{U}{2}n_1 + V_{QEDFT-XC}(n_1) \\
V_2^{KS}(n_2) &= v_2 + \frac{U}{2}n_2 + V_{QEDFT-XC}(n_2)
\end{aligned}$$

(34)

which allows for the full single particle KS Hamiltonian of the system to be expressed as:

$$\begin{aligned}
H_{KS} = &-t\big(\hat{c}_{1\uparrow}^{\dagger}\hat{c}_{2\uparrow} + \hat{c}_{2\uparrow}^{\dagger}\hat{c}_{1\uparrow} + \hat{c}_{1\downarrow}^{\dagger}\hat{c}_{2\downarrow} + \hat{c}_{2\downarrow}^{\dagger}\hat{c}_{1\downarrow}\big) \\
&+ V_1^{KS}(n_{1\uparrow})\hat{n}_{1\uparrow} + V_1^{KS}(n_{1\downarrow})\hat{n}_{1\downarrow} \\
&+ V_2^{KS}(n_{2\uparrow})\hat{n}_{2\uparrow} + V_2^{KS}(n_{2\downarrow})\hat{n}_{2\downarrow},
\end{aligned}$$

(35)

where we have separated the spin channels ($\uparrow$ and $\downarrow$), which we consider splitting into two independent problems as we deal with the spin unpolarised case by dealing with the total density at site $i$ as $n_i = n_{i\uparrow} + n_{i\downarrow}$. The KS system of equations is then diagonalised, i.e., $\hat{H}_{KS}\phi_j = \varepsilon_j\phi_j$, where $\varepsilon_j$ are the KS eigenvalues and $\phi_j$ are the KS eigenvectors. Explicitly, for the Fermi-Hubbard dimer, the KS matrix for a given spin channel is defined by:

$$H_{KS} = \begin{pmatrix} V_1^{eff}(n_1) & -t \\ -t & V_1^{eff}(n_2) \end{pmatrix}.$$

$$(36)$$

That is, in short, an initial guess of the density is made, which is proportional to the external potential, as in the second panel of Figs 2A and 2B.

(i) Finally, the Kohn Sham equations are solved until self-consistency, as depicted in the rest of the panels in Figures 2A and 2B. The spectrum of the KS problem consists of the eigenvalues $\{\varepsilon_1, \varepsilon_2\}$ and eigenvectors $\{\phi_1, \phi_2\}$, that are of dimension $L$. From the KS eigenvectors a new density is constructed, for the half-filled system this is:

$$n_1 = 2|\phi_1(1)|^2,$$
$$n_2 = 2|\phi_1(2)|^2,$$

$$(37)$$

where $\phi_i(j)$ is the $j^{th}$ element of the $i^{th}$ KS eigenvector. This density is then used as the input to the next iteration of the DFT iterative algorithm, i.e. equation (33) is replaced with these values. At each iteration the energy of the parent Hamiltonian, i.e. equation (32), is calculated using:

$$E_{GS}(\mathbf{n}^{GS}, U) = 2\epsilon_1 - V_{QEDFT-XC}(n_1, U)n_1 - V_{QEDFT-XC}(n_2, U)n_2$$
$$- \frac{1}{4}U(n_1^2 + n_2^2) + \epsilon_{QEDFT-XC}(n_1, U) + \epsilon_{QEDFT-XC}(n_2, U).$$

$$(38)$$

The algorithm concludes when the difference between the energy at two successive iterations is less than a chosen tolerance $\delta$. At this point the results of the DFT algorithm for a chosen functional can be compared to the results produced via an exact method, which is possible as the Fermi-Hubbard dimer can be efficiently solved both analytically and numerically due to its small size.

**Levy-Lieb Lattice DFT formulation for the Hubbard Dimer**

Another way of saying this is that the Hubbard Dimer is analysed within the Site Occupation Functional Theory (SOFT) formalism with Kohn Sham self consistency across a number of different approximations, including exact approaches (Exact Diagonalisation (ED)), mean field (Hartree Fock (HF)) and variational methods (Variational Quantum Eigensolver (VQE)). Moreover, we introduce two approaches that can compute the physical features of the universal functional, which is:

$$F[n] = \min \Psi \to n \langle \Psi | \hat{T} + \hat{V} | \Psi \rangle = T[n] + V_{ee}[n] = T[n] + E_H[n] + E_{XC}[n]$$

$$(39)$$

where T[n] is the kinetic energy functional, $E_H[n]$ is the Hartree term and $E_{XC}[n]$ is the exchange correlation energy, corresponding to the first term of equation (6).

The dimer is defined in accordance with equation (32) by:

$$H_{dimer} = H_{hubb} + \sum_{i=1}^{2} v_i \hat{n}_i$$

$$(40)$$

where $H_{hubb}$ is the homogeneous Hubbard Hamiltonian defined in agreement with equation (21) by:

$$H_{hubb} = -t \sum_{i\sigma}^{1} \left( c_{i,\sigma}^{\dagger} c_{i+1,\sigma} + c_{i+1,\sigma}^{\dagger} c_{i,\sigma} \right) + \sum_{i=1}^{2} U \, n_{i,\uparrow} n_{i,\downarrow} \; .$$

$$(41)$$

Now, we define the following variables and set $N = 2$, so that: $\Delta v = v_1 - v_2$, $v_1 + v_2 = 0$, $n_1 + n_2 = 2$ and $\Delta n = n_1 - n_2$. In doing so we can show that:

$$\begin{aligned} \langle H_{dimer} \rangle &= \langle H_{hubb} \rangle + 2v_2(1 - \langle \hat{n}_1 \rangle) \\ &= \langle H_{hubb} \rangle + \Delta v \big( 1 - (1 - \Delta n/2) \big) \\ &= \langle H_{hubb} \rangle + \Delta v \Delta n/2 \end{aligned}$$

$$(42)$$

We can rearrange this formula to obtain the expression for the Hubbard energy, or the energy solely associated with exchange and correlation of the problem, i.e.:

$$\langle H_{hubb} \rangle = \langle H_{dimer} \rangle - \Delta v \Delta n/2$$

$$(43)$$

These expressions enable us to formulate Kohn Sham theory in the same way as discussed above by restricting our study to the $N = 2$ and $S_z = 0$ sector of the Hubbard dimer, which has the following matrix representation:

$$\begin{pmatrix} \Delta v + U & -t & -t & 0 \\ -t & 0 & 0 & -t \\ -t & 0 & 0 & -t \\ 0 & -t & -t & -\Delta v + U \end{pmatrix}$$

$$(44)$$

where we have used the following basis states $\{| \uparrow \downarrow , 0 \rangle, | \uparrow, \downarrow \rangle, | \downarrow, \uparrow \rangle, | 0, \uparrow \downarrow \rangle\}$.

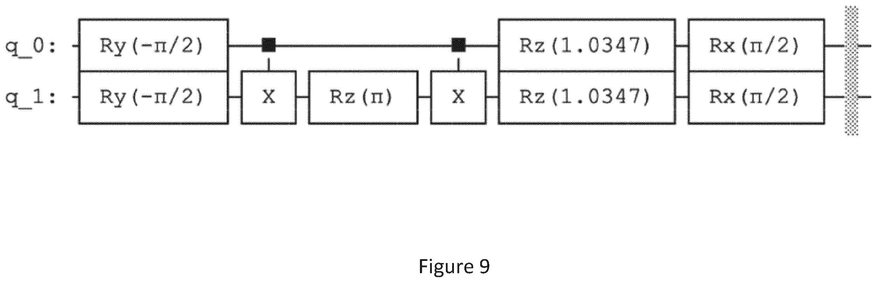

The VQE circuit used here is illustrated in Figure 9 and is based on the compressed representation of the Hubbard dimer at half-filling and $S_z = 0$ with additional single qubit gates to account for the external potential. We have created two subroutine algorithms that compute $F(U/t, \delta n)$ for the Hubbard dimer: (i) qDFT-max and (ii) qDFT- min. Both algorithms utilise the ground state energy computed by VQE which then enters into an outer optimisation loop to find the exchange correlation functional.

The outer optimisation loop (that is analogous to **Table 2)** is executed as a quantum kernel that uses interpolative and extrapolative procedures that allow for continuous representations of the exchange correlation potential whilst reducing finite size effects. In other words, precisely, the quantum kernel protocol is:

a. Obtain the exchange correlation potential using a quantum computer for system size L. This means executing at most 2L quantum computations resulting in a function $V_{XC}(\mathbf{n})$ where $\mathbf{n} = \frac{1}{L}\{0, \ldots, L, \ldots 2L\}$ the discrete fermion fillings.

b. Obtain $V_{XC}(\mathbf{n})$ in a continuous representation of via a classical interpolative procedure, e.g cubic splines. (It is possible to impose constraints on the functional, for example if the fundamental gap, $\Delta = V_{XC}(1^+) - V_{XC}(1^-)$, is known a priori, it can impose two extra constraining points near $\frac{N}{L} = 1$. It is possible to know this a priori from $\Delta = U - \lim_{n \to 1^-} \partial_n \epsilon_{GS}(n < 1)$.

c. Repeat step (a)-(b) for M different system sizes, for example in 2d we could have $\mathbf{L} = \{(n_x, n_y)_1, \ldots, (n_x, n_y)_M\} = \{(2, 2), (3, 3), \ldots, (M, M)\}$.

d. Extrapolate using $\mathbf{L}$ to the thermodynamic limit, if the scaling is possible, otherwise take $V_{XC}(\mathbf{n})$ from the largest system size.

Extrapolating to thermodynamic limit can alleviate finite size effects. In the context of the Bethe Ansatz solution to the Hubbard Model in one dimension 'thermodynamic limit' means the limit $L \to \infty$, $N \to \infty$, and $\frac{N}{V}$ is constant at zero temperature.

**The VQE circuit**

The VQE circuit is given in Figure 9 in a compressed representation. This circuit can be tested and verified by running by running a suitable python code, for example Qiskit modelling code:

```
from qiskit import QuantumCircuit
import numpy as np

# instantiate qiskit quantum circuit object
circ = QuantumCircuit (2)

# initial ansatz for the VQE parameters
params = [1, 1, 1]

# Initial state prep
circ .ry(-np.pi /2, 0)
circ .ry(-np.pi /2, 1)

# Onsite gate
circ .cx (0, 1)
circ .rz( params [0] , 1)
circ .cx (0, 1)

# chemical potential gate
circ .rz( params [1], 0)
circ .rz( params [1] , 1)

# Hopping gates
circ .rx( params [2] , 0)
circ .rx( params [2], 1)
```

**Preliminary Results**

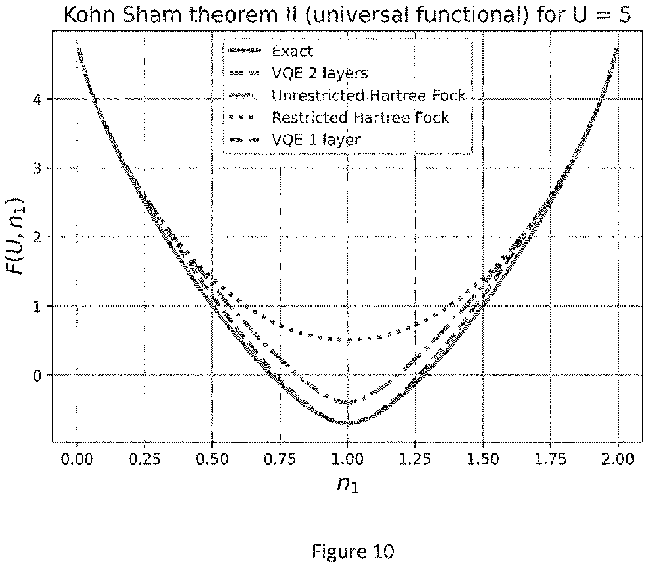

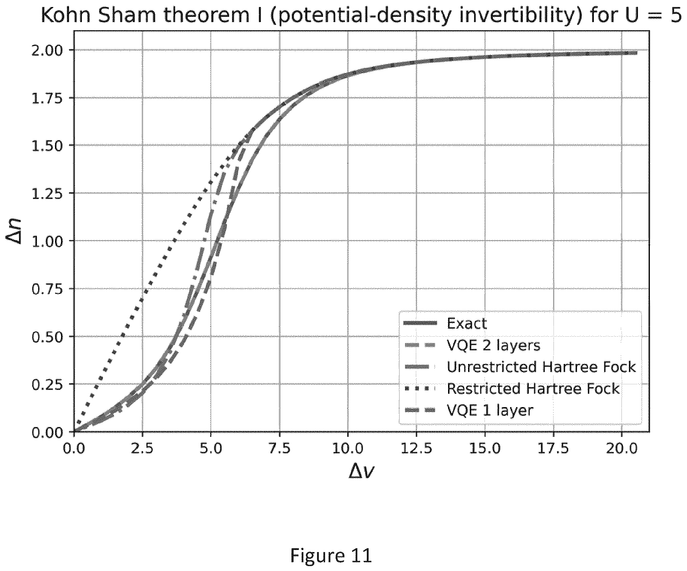

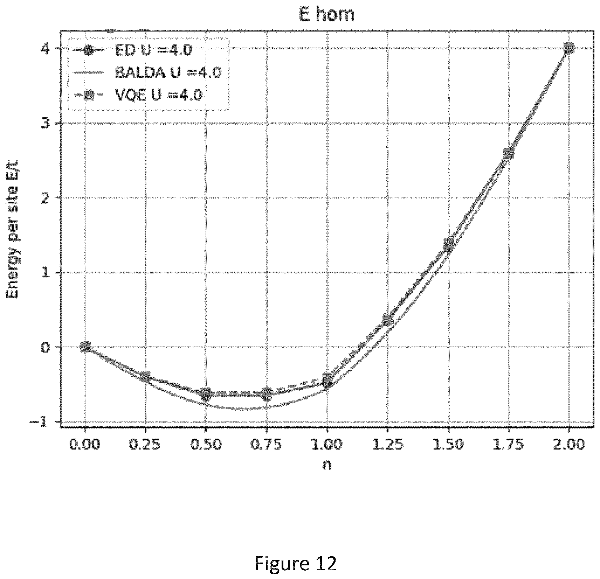

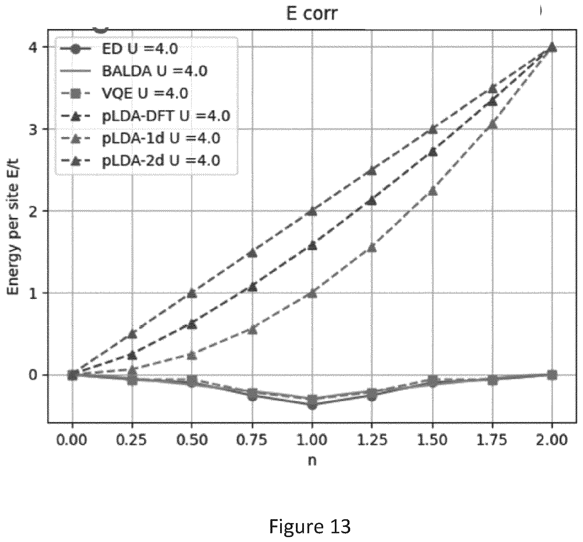

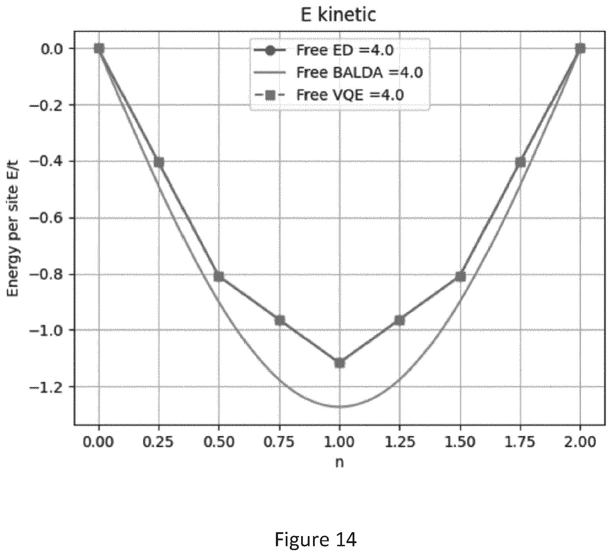

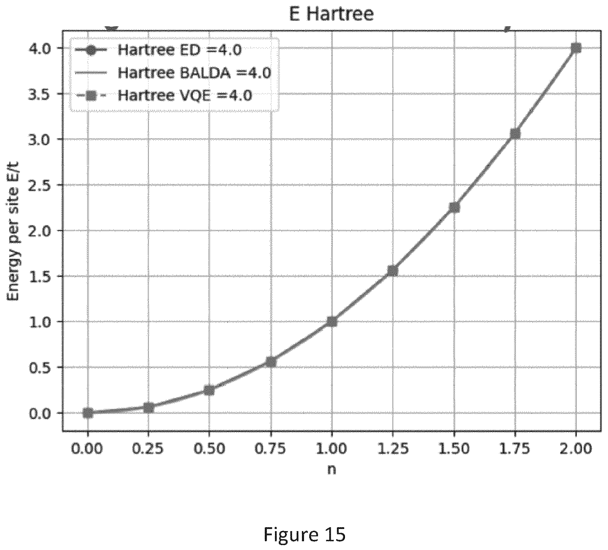

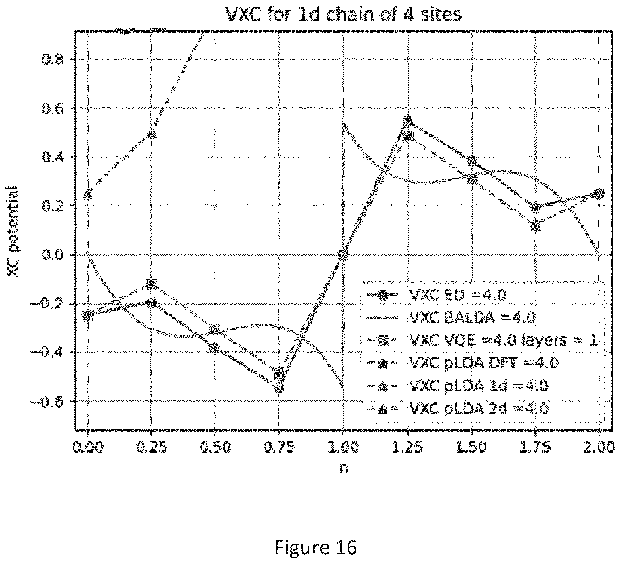

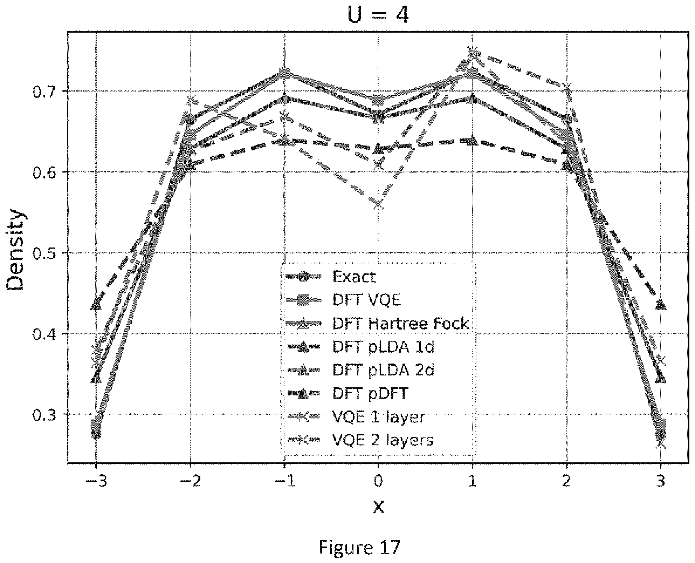

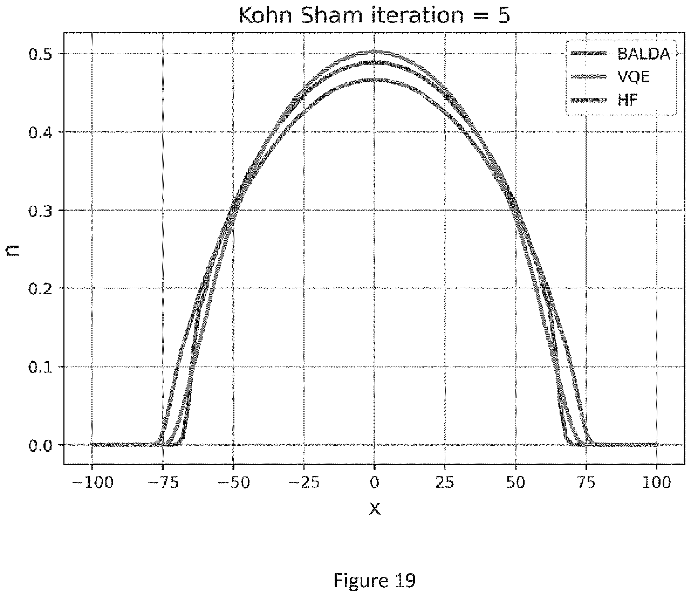

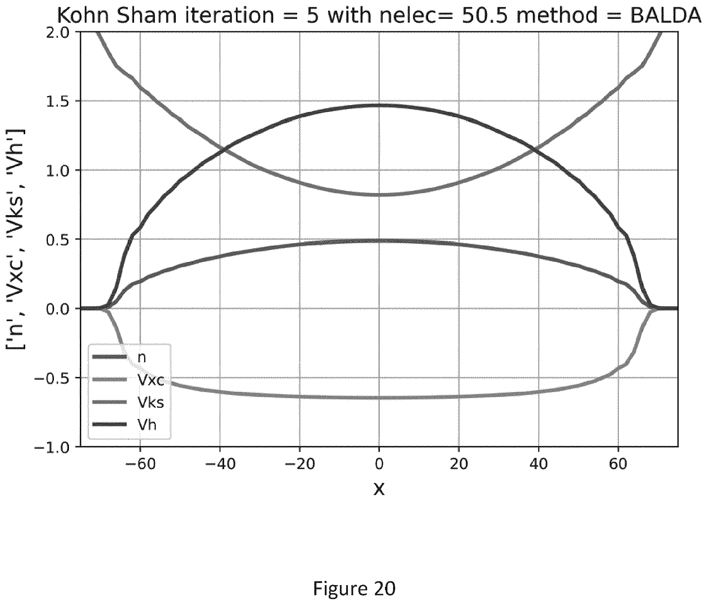

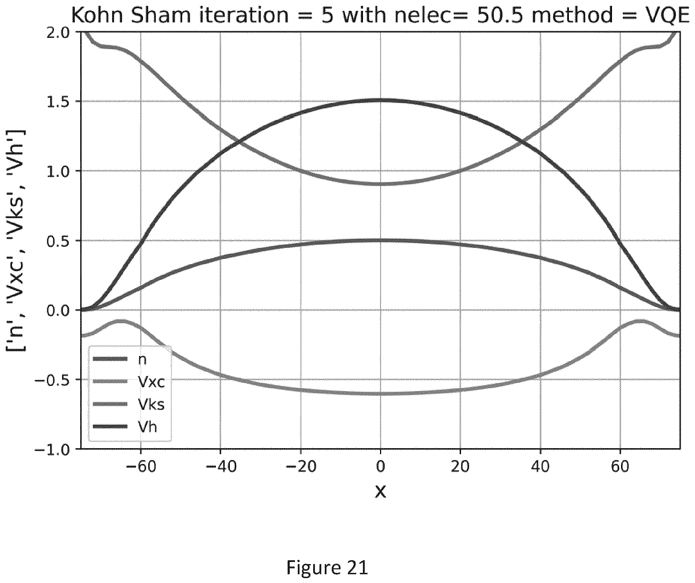

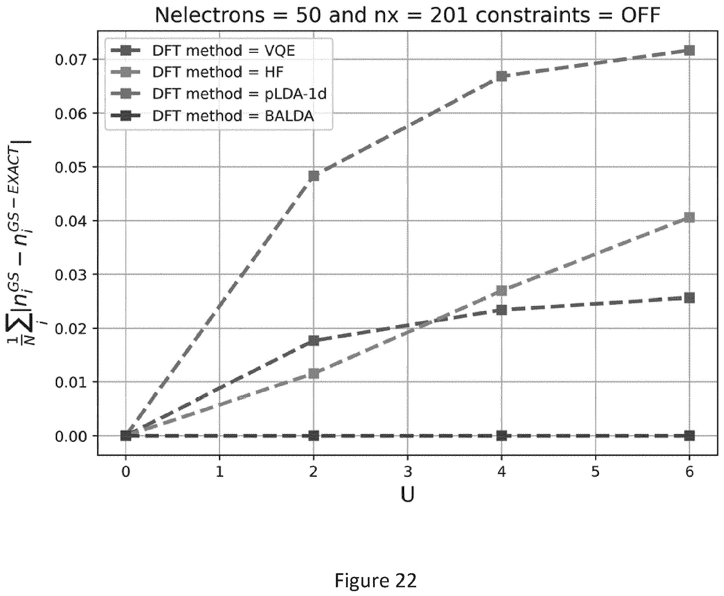

Various results of the process including comparisons against existing methods, are shown in Figures 10 to 22. A core result here is the improvement shown in the hybrid DFT methods set out herein show an unexpectedly beneficial result, despite the simplifications implemented to allow the problem to be tractable on modern NISQ hardware.

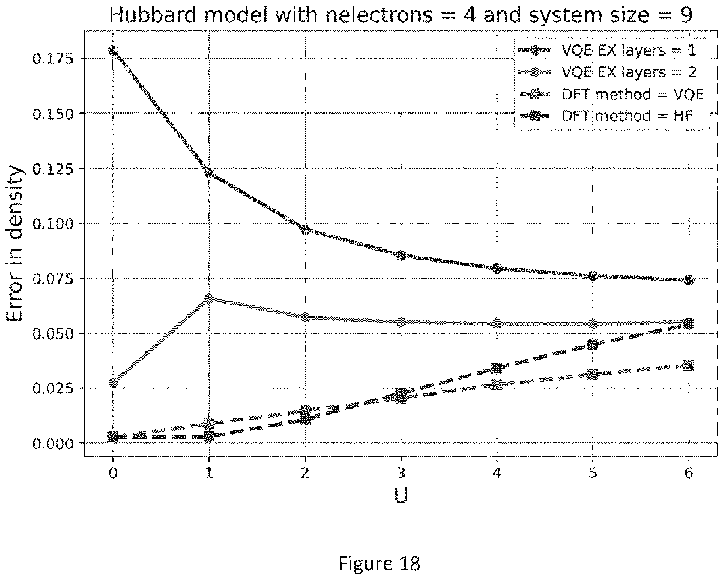

As one example, in Figure 18, the DFT method (VQE line) outperforms all other methods (with the exception of Hartree Fock at low U values. In particular, it is substantially more accurate than the raw VQE algorithm of depth 1.

**More general Fermi-Hubbard models: Extending beyond the dimer**

Figure 3 shows the general flow diagram of how the quantum and classical components interact in the QEDFT algorithm for the Fermi-Hubbard model. On the left hand side we illustrate how the exchange correlation potential is constructed within QEDFT. On the right hand side we show how the DFT loop is implemented within this hybrid scheme. We have highlighted by "Execution: Quantum Computer" the computations which are run on the quantum computer, while the rest of the computations are all run on a classical Computer, in this example.

The dimer is extended by simply adding more sites for a specified geometry, and results in the inhomogeneous Hubbard model (IHM, the generalised inhomogeneous Fermi-Hubbard model) is given by:

$$
H = -t \sum_{\langle ij \rangle, \sigma} \left( c_{i,\sigma}^{\dagger} c_{j,\sigma} + c_{j,\sigma}^{\dagger} c_{i,\sigma} \right) + \sum_{i}^{L} U n_{i,\uparrow} n_{i,\downarrow} + \sum_{i=1}^{L} V_{\text{ext}}(i) \hat{n}_i ,
$$

$$(45)$$

where $V_{\text{ext}}(i)$ is the external lattice potential, $U$ is the interaction strength, $t$ is the hopping parameter and $L$ is the system size. Here $\langle ij \rangle$ indicates a sum over nearest neighbours, and $\sigma$ denotes the spin. In 1 dimension $L = n_x$ where $n_x$ is the number of sites of the lattice along the x-axis; in 2 dimensions $L = n_x n_y$, and so on. Herein, $V_{\text{ext}}(i)$ may also be denoted $v_i^{ext}$.

The Hamiltonian Variational Ansatz for inhomogeneous systems takes account of the densities which appear attached to the external potential in the above equation. These manifest as additional single particle density terms in the Hamiltonian due to the presence of the external potential, that would not be there if one wasn't constraining it to specific values of the external potential, and consequently specific values of density observables due to the Hohenberg-Kohn theorems.

Kohn-Sham (KS) DFT maps equation (45) into the effective Kohn-Sham system, where the many body interaction term is absorbed into a one-body potential $V_i^{\text{eff}}$,

$$H_{KS}(n_i) = -t \sum_{\langle ij \rangle}^{L} \left( c_i^\dagger c_j + c_j^\dagger c_i \right) + \sum_i V_i^{\text{eff}}(n_i) c_i^\dagger c_i \ ,$$

(46)

for a given spin channel $\sigma$, which we now omit as we treat both spin channels equally going forward by setting the density variable as $n_i = n_{i\uparrow} + n_{i\downarrow}$.

The effective KS potential for the generalised Fermi-Hubbard model is written as a combination of the external Hartree and XC potentials

$$V_i^{\text{eff}}(n_i) = V_{\text{ext}}(i) + \frac{U}{2} n_i + \frac{dF(\mathbf{n})}{dn_i}$$

(47)

where $\frac{dF(\mathbf{n})}{dn_i} = V_{QE-XC}(n_i)$ is the unknown multivariate exchange correlation potential the central object of interest which is approximated according to the methods prescribed for equation (19) which are described in **Table 2.** Once $\frac{dF(\mathbf{n})}{dn_i}$ is approximated, the self-consistent Kohn-Sham scheme is initiated and the primary parts of the classical algorithm are highlighted in Section 6.1. Now we introduce quantum techniques that compute $F(\mathbf{n})$ for the extended Fermi-Hubbard model.

The Quantum Local Density Approximation (QLDA) expresses the form of the XC energy functional in a compact representation, shown above in the specific context of the Hubbard Dimer. To extend beyond the Dimer we start again with the general functional form of the object, we have,

$$E_{XC} = E_{XC}(n_1, \ldots, n_L, U)$$

(48)

QLDA approximation to the XC energy functional then is,

$$E_{XC}^{QLDA}(n_i, U) = \frac{1}{L} E_{hom}^{VQE}(n_i, U) - \frac{1}{L} \sum_{ij\sigma}^{L} t_{ij\sigma} \langle c_{i\sigma}^\dagger c_{j\sigma} + c_{j\sigma}^\dagger c_{i\sigma} \rangle - \frac{1}{L} \sum_i^{L} \frac{U}{4} \langle n_i^2 \rangle$$

(49)

where $E_{hom}^{VQE}(n_i, U)$ is the energy of the homogeneous Hubbard model, with $V_{\text{ext}}(i) = 0$, computed using VQE of a specified depth and occupation number. When the hopping parameter 't' doesn't depend on spatial or spin indices (and/or $E_{hom}^{VQE} = E_{QE-hom}$ ), equation (49) can be written in the equivalent form

$$E_{QE-XC}(n_i, U) = \frac{1}{L} E_{QE-hom}(n_i, U) - \frac{t}{L} \sum_{\langle ij \rangle \sigma} \langle \hat{c}_{i\sigma}^\dagger \hat{c}_{j\sigma} + \hat{c}_{j\sigma}^\dagger \hat{c}_{i\sigma} \rangle - \frac{1}{L} \sum_i^L \frac{U}{4} \langle n_i^2 \rangle$$

$$(50)$$

where $E_{QE-hom}(n_i, U)$ is the energy of the homogeneous Fermi-Hubbard model, retrieved from equation (45) by setting $v_i^{ext} = 0$, and computed using VQE of a specified depth and occupation number.

In comparison to $E_{XC}$, above, the total energy has been assumed to be a sum of the average energy in a homogeneous Hubbard chain of size L. In the local density approximation, the total homogeneous energy for the Fermi-Hubbard model is defined as the sum of the average energy in a homogeneous Fermi-Hubbard over the entire chain. The protocol for obtaining this function is sketched in Section 6.2, and once it is known the XC potential can be computed by differentiation, i.e., once equation (50) is computed and an ansatz for the initial density is made, the XC potential can be obtained by equation (19):

$$V_{XC}^{QLDA}(n_i, U) = \frac{\partial E_{XC}^{QLDA}(n, U)}{\partial n} \bigg|_{n=n_i}$$

This potential determines the KS system of equations to be solved. For example, for the 1D Hubbard chain with open boundary conditions, the KS matrix is

$$H_{KS} = \begin{pmatrix} V_1^{eff}(n_1) & -t & 0 & 0 & 0 & 0 \\ -t & V_2^{eff}(n_2) & -t & \ddots & \ddots & 0 \\ 0 & -t & V_3^{eff}(n_3) & \ddots & -t & 0 \\ 0 & \ddots & \ddots & -t & V_{L-1}^{eff}(n_{L-1}) & -t \\ 0 & \ddots & \ddots & \\ 0 & 0 & 0 & 0 & -t & V_L^{eff}(n_L) \end{pmatrix}$$

$$(51)$$

The result of this diagonalisation are the KS eigenvalues, $\{\varepsilon_1, \dots, \varepsilon_L\}$, and the KS eigenvectors, $\{\phi_1, \dots, \phi_L\}$. The KS eigenvectors are used to construct a new approximation to the density via equation (11), which is then mixed with the previous density equation (13) for numerical stability, which can be then used to construct a new KS matrix equation (51).

Thus, for a system of size L the maximum number of VQE calls to approximate $E_{XC}$ is given by 2L. By constructing interpolative and extrapolative techniques, which are briefly touched upon in the section "Numerical results", it is possible to modify the number of these calls, whose upper bound will be $2 \Pi_i L_i$, where $L_i$ is the set containing the number of system sizes considered, e.g. L = {4, 5, 6} would require at most 240 quantum computations to be performed. At convergence, the ground state energy is determined by:

$$E_{GS}(\mathbf{n}^{GS}, U) = \sum_i^{occ} \varepsilon(U)_i^{KS} - \sum_i V^{XC}(n_i, U) n_i^{GS} - \frac{1}{4} U \sum_i n_i^2 + E_{XC}^{QLDA-TOT}[\mathbf{n}^{GS}, U]$$

$$(52)$$

where $\varepsilon_i^{KS}$ are the eigenvalues of the occupied Kohn-Sham system and the ground state density is determined by:

$$n_i^{GS} = \sum_j^{occ} \phi_{ij}^2$$

$$(53)$$

where $\phi_{ij}$ is the $i$'th element of the j'th occupied Kohn Sham eigenvector. The DFT approximation to the groundstate energy from Equation (52) can also be expressed as:

$$E_{GS}(\mathbf{n}^{GS}) = 2 \sum_{i \le \frac{occ}{2}} \epsilon_i^{KS} - \sum_{i \in \mathcal{L}} V^{XC}(n_i) n_i^{GS} - \frac{1}{4} U \sum_{i \in \mathcal{L}} n_i^2 + \sum_{i \in \mathcal{L}} E_{QE-XC}(n_i, U),$$

$$(54)$$

where only the first $N_e$ KS eigenvalues are included in the summation, with $N_e$ corresponding to the fermion filling of the inhomogeneous model equation (45). The entire protocol for the generalised Fermi-Hubbard system is illustrated in Figure 3.

The total exchange correlation energy is given by:

$$E_{XC}^{QLDA-TOT}[\mathbf{n}^{GS}, U] = \sum_i E_{XC}^{QLDA}(n_i, U).$$

$$(55)$$

Herein, multivariate means that:

$$E_{XC} = E_{XC}(n_1, \dots, n_L, U)$$

$$(56)$$

where $E_{XC}(n_1, \dots, n_L, U)$. is a functional of the density at $L$ sites, i.e., it is a function of $L + 1$ variables. The function depends on $L + 1$ variables in potentially a nonlinear way. The local density approximation assumes that the function can be separated linearly, such that:

$$E_{XC}^{QLDA-TOT}[\mathbf{n}^{GS}, U] = \sum_i E_{XC}^{QLDA}(n_i, U)$$

$$(57)$$

i.e., the function is a sum of $L$ terms in a linear way. Including nonlocality means that the function would depend not only on density at site $i$, but also at additional sites, for example in the nearest neighbour case in one dimension it would mean:

$$E_{XC}^{QLDA-TOT}[\mathbf{n}^{GS}, U] = \sum_i E_{XC}^{QLDA}(n_{i-1}, n_i, n_{i+1}, U).$$

$$(58)$$

In a continuous sense, it just means that the functional not only depends on the density at site $i$, but also on the derivative, i.e.:

$$E_{XC}^{QLDA-TOT}[\mathbf{n}^{GS}, U] = \sum_i E_{XC}^{QLDA}(n_i, \partial_x n_i, U)$$

$$(59)$$

if we are in 1 dimension. In the classical DFT literature this approach is similar to the Generalised Gradient Approximation (GGA).

The true XC functional is multivariate and a complicated function of the entire density at all points in space. Knowing exactly the multivariate functional representation for a given model would provide the exact solution for the ground state properties for the homogeneous and all inhomogeneous variations of that model by only solving the classical Kohn-Sham equations, which scale as $L^3$, where $L$ is the system size.

Theoretically this is NP-hard, as it necessitates the solution of the exact homogeneous problem, for example the Hubbard model. However, with a near term quantum computer it may be possible to qualitatively capture important features of the multivariate functional with low-depth and low-quality quantum simulations, which lead to better solutions when this is fed into the DFT loop than classical approximations to the functional can achieve. Practically, the external potential of the inhomogeneous model needs to be varied, in a more general way than it is done in the qDFT-max procedure as implemented for the Hubbard dimer in Appendix 1; however, for the QELDA approach the XC functional can be built entirely from the homogeneous Hubbard model.

Nonlocality can be incorporated into this approximate functional by making the XC energy depend not only on the local density at each site, but also on its derivative. This follows the spirit of where in practical ab initio DFT calculations a very successful approach that supplements ideas based on local density approximations are generalised gradient approximations.

In Figs 1A to 2B we present the detailed steps involved in performing a full QDFT computation for the Hubbard dimer. Specifically, when this process is extended to more complicated scenarios we distinguish these by (1) Hubbard models of larger dimension, as is highlighted generically in Figure 1A, or (2) simulate materials/chemical systems. In the case of (1), we remind ourselves that $L = n_x \times n_y$, where $n_x = 1$ and $n_y = 2$ for the Hubbard dimer. $n_e = 2L$, i.e the maximum number of fermions in the system. Generally, the number of energy data computed by a quantum computer in a system of size $L$ is $2L$ and they are plotted in the same way as Figure 4. This is precisely the main operational difference between running the Hubbard dimer and a system size of larger dimension. For more realistic systems that can be used for materials and/or chemical simulation the general strategies are outlined in the sections "Direct parameterisation of tight binding models" and "Direct representation for periodic systems in a continuous basis" below. Importantly, what needs adaptation here is the connectivity of the underlying lattice model, the values of the parameters which are used to the define the model, and the form of the external potential studied. The type of encoding and VQE ansatz used may also have to be modified, depending on the chosen system of interest. In principle, any systems which have a representation of the form in the "Direct parameterisation of tight binding models" section below, these include for example semiconductors, superconductors, battery materials, photovoltaics, and thermoelectric materials, to name only a few. In specific reference to the plots, the primary difference between the approach for the dimer and more general systems essentially occurs in Figure 1A fourth panel, where the ground state energy at different fillings is computed, where for more general systems the density mesh is denser, as in Figure 4.

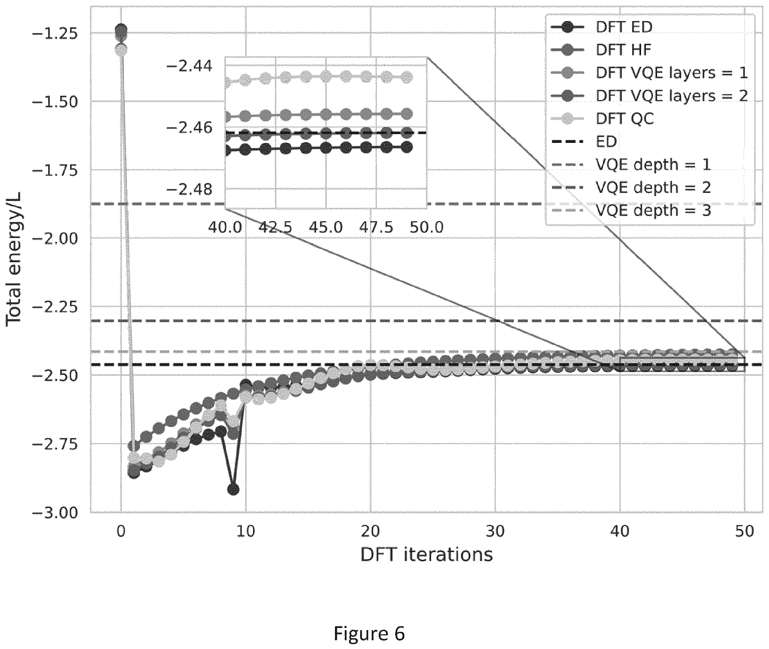

It is surprising that the ground-state approximation produced with a noisy NISQ processor can still produce a DFT output that falls within 1% (e.g. Figure 6) of in particular parameter regimes (e.g. as a specific example, $\frac{U}{t} = 4$, $n_x = 1$, $n_y = 8$, with a quarter filling in an impurity potential) of the Hubbard model. This is in contrast to the classical mean field results, which fall outside of this 1% interval. Additionally, the approach does reasonably well at capturing qualitative features of the XC potential as compared to exact classical methods - in particular, the discontinuity of the XC potential is well captured by VQE methods as shown in Figure 4. The XC potential in the Hartree Fock approximation by contrast, perhaps the most famous single particle method after DFT, is zero everywhere by definition, so it is impossible to infer how Mott physics appears in the functional with Hartree Fock. Advantageously, for strongly correlated systems ($\frac{U}{t} > 1$) the estimation of the density is also well described.

It is also worth highlighting that in the weakly correlated regime that classical mean field theory (Hartree-Fock) can be used as a more accurate solver, as can be seen in Figure 7.

In Figures 1 to 2A we present the detailed steps involved in performing a full QDFT computation for the Hubbard dimer. We have commented in the discussion after the Hubbard dimer walkthrough how to generalise this approach to more complicated systems, in particular also materials simulations. In specific reference to the plots, the primary difference between the approach for the dimer and more general systems essentially occurs in Fig. 1 fourth panel, where the ground state energy at different fillings is computed, where for more general systems the density mesh is denser, as in Figure 4.

- A quantum computer was used to obtain the energies of the homogeneous Hubbard model using VQE, as highlighted in left part of Fig. 3. The result of this is a discrete function, such as in Figure 4
- This data is then used to construct the exchange correlation potential, shown in Figure 5.
- Finally the XC potential in Figure 5 is queried 50 times inside a DFT loop for an inhomogeneous model (see Fig. 6) with a random external potential (a test case) and illustrates then convergence of the ground state energy from various methods compared to the exact answer as computed using Exact Diagonalisation.

To help this discussion, we outline where the quantum computation occurs in the overall algorithm, which is sketched in Fig. 3. A particular instance of the qDFT implementation works as follows:

- Using a quantum computer, approximate (possibly to very low accuracy) the ground state energies as a function of electron filling for a chosen homogeneous interacting model. This is the point where the quantum computer is called and uses an algorithm to compute the ground state energy. For this instance it is the only part of the whole method which is performed by a quantum computer. However, for the numerics which we have performed to test and validate our methods, we have used the Varitional Quantum Eigensolver (VQE) and its Hamiltonian Varitional Ansatz (HVA) as the quantum algorithm of choice. This is illustrated in the left part of Fig. 3.

The call to the quantum computer necessitates a quantum algorithm to approximate the ground state energy of a system. This approximation, however, does not necessarily need to be very accurate, as it is not used as the final output but fed into the DFT loop in order to steer the DFT algorithm towards a more accurate solution (see discussion, below, on intuition about why the combination of classical DFT with VQE works better). Therefore, any quantum algorithm that is capable of approximating the ground state energy - even crude approximations such as the very low-depth VQE achievable on current, noisy quantum hardware - can be used. We anticipate that better approximations would be advantageous when these are available, but we have demonstrated that our hybrid algorithm still produces very good solutions even when only low accuracy approximations are available from the quantum part of the method. For example, in the case of the Hubbard model we use VQE and the HVA and this determines how the circuit is designed. This is a common choice for computing the ground state energy of the Hubbard model.

- Once the energy of the homogeneous model is computed, the exchange correlation energy is computed. In Fig. 3 we show how this is achieved within the Local Density Approximation of DFT for the homogeneous Hubbard model. We emphasise that this approximation is not strictly necessary but is used to demonstrate practically how to obtain the exchange correlation energy.
- The exchange correlation energy is then differentiated to produce the exchange correlation potential.
- This potential is then used inside the classical DFT algorithm which is entirely run on a classical computer, which is illustrated on the right part of Fig. 3

***Direct parametrisation of tight binding models:***

Materials/molecular Hamiltonians have the following representation

$$H = \sum_{\sigma} \sum_{\lambda_1, \lambda_2} t_{\lambda_1, \lambda_2} c^{\dagger}_{\lambda_1, \sigma} c_{\lambda_2, \sigma} + \sum_{\sigma, \sigma'} \sum_{\lambda_1, \lambda_2, \lambda_3, \lambda_4} V^{\text{Coulomb}}_{\lambda_1 \lambda_2 \lambda_3 \lambda_4} c^{\dagger}_{\lambda_1, \sigma} c^{\dagger}_{\lambda_2, \sigma'} c_{\lambda_3, \sigma'} c_{\lambda_4, \sigma}$$
$$+ \sum_{\lambda_1} V^{ext}_{\lambda_1} \big( c^{\dagger}_{\lambda_1, \uparrow} c_{\lambda_1, \uparrow} + c^{\dagger}_{\lambda_1, \downarrow} c_{\lambda_1, \downarrow} \big),$$

$$(60)$$

where $\lambda$ is the site or orbital index and represents the quantum numbers of the fermions, excluding spin which is explicitly labelled. $V^{\text{Coulomb}}_{\lambda_1 \lambda_2 \lambda_3 \lambda_4}$ is the four index Coulomb tensor, $V^{\text{ext}}_{\lambda_1}$ is the single index external potential, and $t_{\lambda_1, \lambda_2}$ is the two-index pure hopping terms is an off diagonal matrix. $V^{\text{ext}}_{\lambda_1}$ is set by the material or molecule under examination, while the other parameters can be computed from Ab Initio calculations. Once the homogeneous Hamiltonian is known (through setting $V^{\text{ext}}_{\lambda_1} = 0$), its local exchange correlation energy can be computed similarly as described in Fig. 3 using quantum algorithms. The obtained functional can then be used for larger system sizes with inhomogeneities. We note that depending on the material of choice, the Hamiltonian defined above is general, so that 1d, 2d, and 3d systems can be described; as well as systems with long range hoppings, interactions (e.g. a multiorbital Slater-Kanamori system) and varying connectivities. In principle, every material that has a unique homogeneous Hamiltonian would also have a unique XC potential.

Similar approaches, i.e. extending lattice models to continuous systems, have been applied within classical algorithms, using different approximate functionals, the first application of DFT within the lattice context to continuous systems. In these works, well known values for $t_{\lambda_1, \lambda_2}$ are used to model the hopping processes, while $V^{\text{Coulomb}}_{\lambda_1 \lambda_2 \lambda_3 \lambda_4}$ is approximated to an onsite Hubbard interaction. We also note that it is also possible to truncate the above Hamiltonian to a subset of preferential active states within the Hilbert space, as described in. The advantage of doing so can dramatically reduces the quantum algorithm resource requirements. Technically, in this approach there emerges the well-known issues of incorporating double corrections.

***Direct representation for periodic systems in a continuous basis:***

In this case, we first define the ground state energy in terms of the continuous density $n(\mathbf{r})$

$$E[n] = T[n] + H[n] + E_{XC}[n] + \int n(\mathbf{r})V_{ext}(\mathbf{r})$$

$$(61)$$

The basis dependent (e.g. plane-wave, localised) Kohn Sham equations are

$$\left(-\frac{\nabla^2}{2} + V_{\text{ext}}(\mathbf{r}) + V_H(\mathbf{r}) + V_{XC}(\mathbf{r})\right)\phi_j(\mathbf{r}) = \epsilon_j\phi_j(\mathbf{r})$$

$$(62)$$

where $V_{\text{ext}}(r)$ is the external potential, $V_H(r)$ is the Hartree potential, $V_{XC}(r)$ is the exchange correlation potential, which needs to be approximated, and $\{\varepsilon_j, \phi_j\}$ are the Kohn-Sham eigenvalues and eigenvectors. Here we note the difference to the formulation in Fig. 7, which is discrete. The external potential in this case is defined by:

$$V_{\text{ext}}(\mathbf{r}) = \sum_I \frac{Z_I}{|\mathbf{R_I} - r|}.$$

$$(63)$$

In this approach to solve the equation, the approximation to $V_{XC}(r)$ originates in using a quantum algorithm to compute

$$E_{XC}[n] = E_{GS}[n] - \left(T[n] + H[n] + \int n(\mathbf{r})V_{ext}(\mathbf{r})\right),$$

$$(64)$$

where $E_{GS}[n]$ is computed on a quantum computer, for a given number of electrons and a Hamiltonian of the form above. Note that we have that for a given system we have subtracted away the contribution from the exchange correlation energy.

As a subroutine, the Variational Quantum Eigensolver algorithm is used to compute the exact exchange correlation energy contribution of the constituent fermions in the input molecular system. Importantly, for the exemplary application of *myotonic dystrophy, we* also incorporate into the algorithm how best to represent the input molecular Hamiltonians in order to minimize the number of quantum resources required to efficiently simulate the full target system.

By using a quantum computer to determine the unknown classical XC functional, the effects of exact exchange and correlation are automatically incorporated, a well-known shortfall of classical functional approximations that results in inaccurate ground state property calculations, most notably the over delocalisation of electrons for a number of well-known molecular and periodic systems. We also note the benefit this approach has in terms of scalability over direct quantum mechanical treatments, such as classical configuration interaction approaches that suffer from the exponential scaling of the Hilbert space, or the direct implementation of quantum algorithms for the complete target system; here, once the functional is stored classically it can be used for any target system within the classical Kohn Sham framework, and agnostically incorporated with classical software packages, which scales polynomially in the system size and is an efficient classical algorithm.

REPRESENTATIVE RESULTS

**Numerical results**

For the VQE simulations, we implemented the Hamiltonian variational ansatz from [D. Wecker, M. B. Hastings and M. Troyer. "Progress towards practical quantum variational algorithms". In: Phys. Rev. A 92 (4 2015), p. 042303] which has been shown to be promising for solving the Fermi-Hubbard model. The starting state was taken to be the groundstate of the $U = 0$ Fermi-Hubbard model from [C. Cade et al. "Strategies for solving the Fermi-Hubbard model on near-term quantum computers". In: Physical Review B 102.23 (2020), p. 235122] which can be prepared efficiently on a quantum computer using Givens rotations in [Z. Jiang et al. "Quantum Algorithms to Simulate Many-Body Physics of Correlated Fermions". In: Phys. Rev. Appl. 9 (4 2018), p. 044036] and the commuting sets were auto-generated using a greedy graph colouring algorithm which considers each vertex of a graph in sequence and assigns each the first colour that is available. The quantum circuit simulations for VQE were run using the Julia package 'Yao' from [X.-Z. Luo et al. "Yao.jl: Extensible, Efficient Framework for Quantum Algorithm Design". In: Quantum 4 (Oct. 2020), p. 341]. Since all the simulations generated the exact VQE state, i.e. no measurements or noise, we were able to use Yao's automatic differentiation framework to speed up calculations of the gradient to be used with the L-BFGS optimiser suggested in [D. C. Liu and J. Nocedal. "On the limited memory BFGS method for large scale optimization". In: Mathematical Programming 45 (1989), pp. 503-528] in NLopt from [S. G. Johnson, The NLopt nonlinear-optimization package, https://github.com/stevengj/nlopt, 2007].

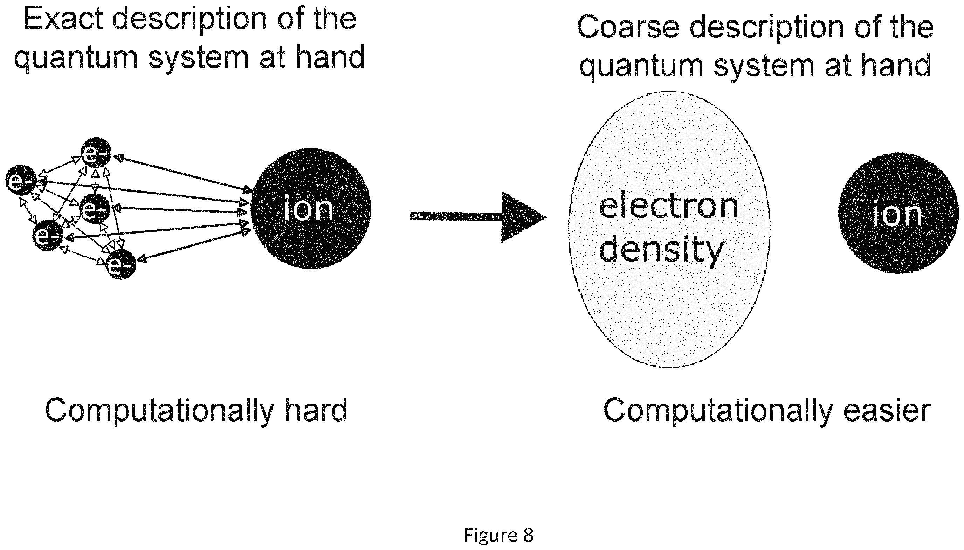

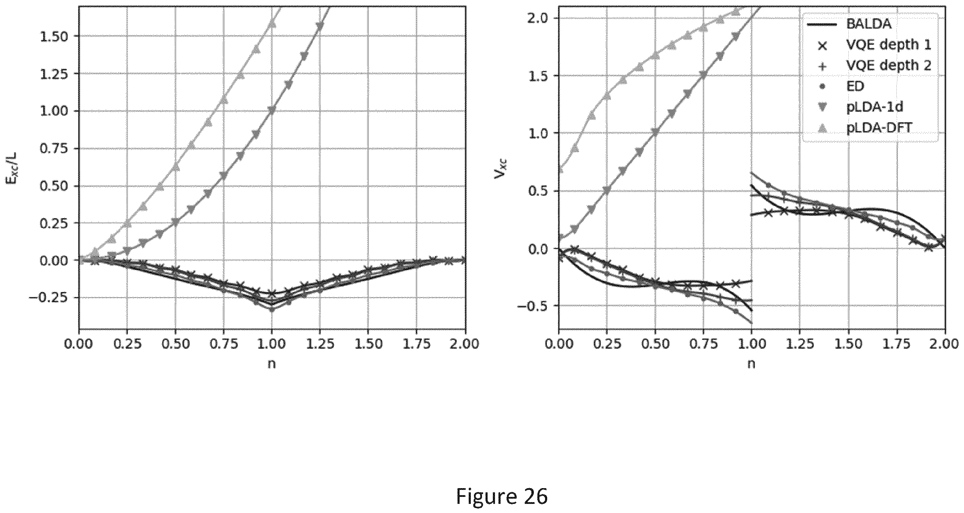

In general, the results presented in this section are analogous to those presented in the sections above, in 'Preliminary Results', and in Figures 1 to 22. The XC energies and potentials used to produce Figures 26 to 46 were found using the procedures outlined above, in the equations, and algorithms of Figures 1 to 3. The results presented below are intended to illustrate further the advantages of the methods disclosed herein, and to provide further examples of systems to which said methods can be applied, and it would be readily appreciated by persons skilled in the art that the following are possible applications and further examples.

The QEDFT XC functional for the Fermi Hubbard model

In Figure 26 we present the XC energy and XC potential for the $1 \times 12$ homogeneous Fermi-Hubbard model. We note that the XC potential has a discontinuity at $n = 1$ due to the cusp in the exchange correlation energy, commonly known as the derivative discontinuity (DD). The DD is a known property of the exact XC potential which a number of classical approximations fail to reproduce and has consequences for the description of properties relating to the fundamental gap of the system as shown in [M. J. P. Hodgson et al. "How Interatomic Steps in the Exact Kohn-Sham Potential Relate to Derivative Discontinuities of the Energy". In: The Journal of Physical Chemistry Letters 8.24 (2017). PMID: 29179553, pp. 5974-5980]. All functionals presented in Figure 26 exhibit this discontinuity, with the exception of the 'pseudo' functionals, and higher depth VQE functionals approximate this discontinuity with higher accuracy in regions both near and away from where the discontinuity occurs. The "pseudo-DFT" functional, $E_{XC}(n) = 2^{-4/3}Un^{-4/3}$ is based on local density functional designs applied in ab-initio methods, specifically for the homogeneous electron gas, and evidently is not a good approximant for the Fermi-Hubbard system. Similarly, the "pseudo-1D" functional, $E_{XC}(n) = 0.25Un^2$, originates from the 1D equivalent. Not only do these functionals omit the DD but they also qualitatively arrive at values which are not comparable for either the XC energy or the XC potential. As these pseudo functionals are so erroneous - which we find is also the case in 2D - we do not proceed in analysing them further or implementing them as part of the DFT study. We emphasise that the BALDA functional is valid in the limit of an infinite chain, but indeed the results for the $1 \times 12$ chain approximate this behaviour. The Hartree Fock results are not discussed here because by definition the XC potential is zero at all interaction values. While the results are only illustrated for VQE variational layer depths of 1 and 2, we anticipate that for larger depths the accuracy of the XC functional will improve with respect to the ED functional. The QEDFT approach stands to have an advantage when scaling to larger system sizes, as the ED functional is limited by the classical compute resources which cannot scale to large system sizes.

The purpose of generating these correlation functionals is to query them inside the DFT algorithm, which means that it is necessary to obtain a continuous representation as a function of the number of electrons. The continuous lines in Figure 26 represent cubic spline fits for each functional, where the XC potential is defined piecewise $V_{XC}^{\text{left}}$ for $n \leq 1$ and $V_{XC}^{\text{right}}$ for $n > 1$. By replacing the discrete points with a continuous spline we introduce an additional source of error, of different nature than the error introduced by approximating the XC functional. The first type of error is present in other works, for example [M. Ijäs and A. Harju. "Lattice density-functional theory on graphene". In: Phys. Rev. B 82 (23 2010), p. 235111], however instead of a spline there a linear regression is used to fit a model functional. In the presence of low amounts of noise, however, the errors due to cubic splines are usually not significant, as we will show for data obtained from hardware. It could be possible to improve the quality of the functional with more sophisticated interpolation and extrapolation procedures that are not investigated here.

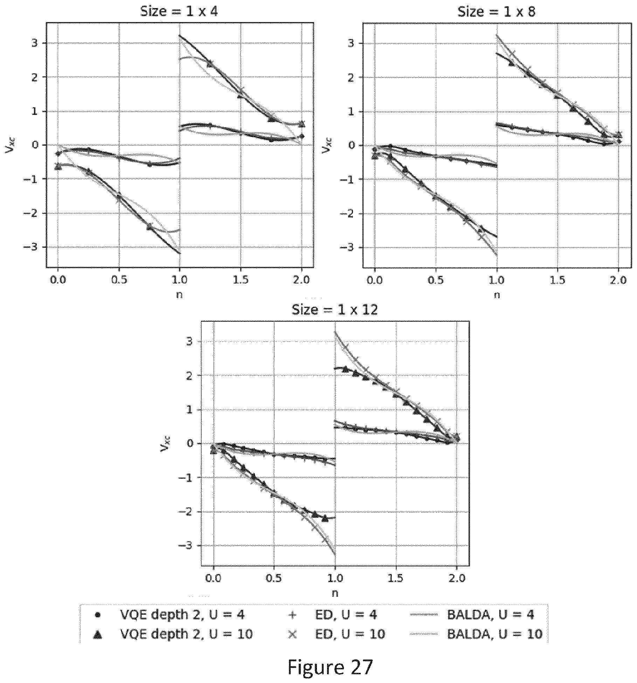

In Figure 27 we illustrate the different forms of the XC potential (obtained from a VQE ansatz circuit of depth 2) as we vary the model parameters, compared against the ED and BALDA results. These comparisons can provide insight into the limitations of using VQE to generate functionals, as the accuracy of VQE decreases for larger interaction strengths $\frac{U}{t}$ and system sizes as in [C. Cade et al. "Strategies for solving the Fermi-Hubbard model on near-term quantum computers". In: Physical Review B 102.23 (2020), p. 235122]. Firstly, we see that the VQE results qualitatively agree with the ED results as $\frac{U}{t}$ is increased with a loss in quantitative accuracy, attributed to the limitations of our VQE instantiation (as the initial state preparation corresponds to $U = 0$) and the Hamiltonian variational ansatz at larger $U$ values. The physical effect of increasing $\frac{U}{t}$ widens the discontinuity in the functional at $n = 1$, whose effects are absent in classical approximations such as Hartree Fock. Moreover, increasing the system size also reduces the quality of the result, as we can see in comparing the $1 \times 12$ results to the $1 \times 8$ results. The main driver of this discrepancy is that at fixed depth the VQE algorithm loses quality for larger system sizes, due to the requirement that a larger number of variational layers (and hence circuit parameters) are needed to converge to the optimal VQE parameters. On the other hand, the continuous representation is constructed on a denser mesh, suggesting that if the VQE depth is sufficient to within a desired error tolerance of the ED (Exact Diagonalisation) functional, then finite size physical errors, as well as those related to the interpolation scheme, can be reduced.

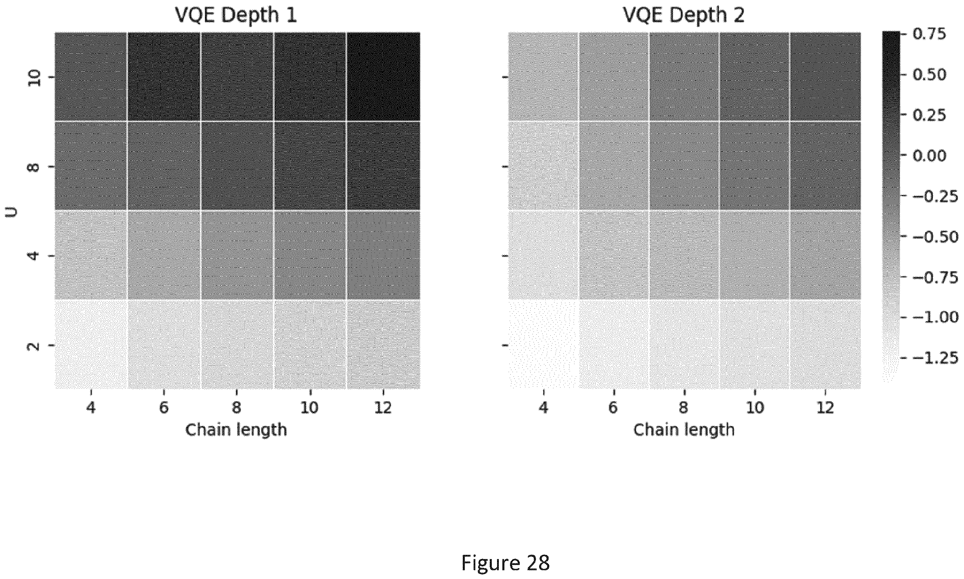

To quantify these effects further, in Figure 28 we provide a quantitative illustration of the error in the XC functional across varying interaction strengths and system sizes. To isolate errors from the splining procedure, we present the Frobenius norm error (on a $\log_{10}$ scale) between the VQE XC potential functionals and the exact functional with respect to the discrete values that are computed and not sampled along the continuous spline. We confirm that VQE functionals decrease in accuracy for bigger systems sizes with larger $U/t$ parameters and that increasing the number of VQE layers can significantly improve the quality of the underlying functional, especially in the high $U/t$ ($\approx 10$) and large system size ($\approx 1 \times 12$) parameter regime of the 1D Hubbard model. When $U/t$ is smaller, i.e $\leq 4$, it is sufficient to use a VQE functional of depth 1 for all system sizes up to $1 \times 12$, as the overall error metric is on a similar scale as that for the VQE functionals of depth 2. Indeed, our results suggest that the XC functional in 1D is more sensitive to increases in the interaction strength than similar increases in the system size, indicating that there exists legitimate regions of applicability for low-depth VQE functionals in DFT computations. This bias is due to the structure of the ansatz circuit for VQE, that uses as a starting state the non-interacting groundstate at $U = 0$, potential improvements could be had by varying this ansatz.

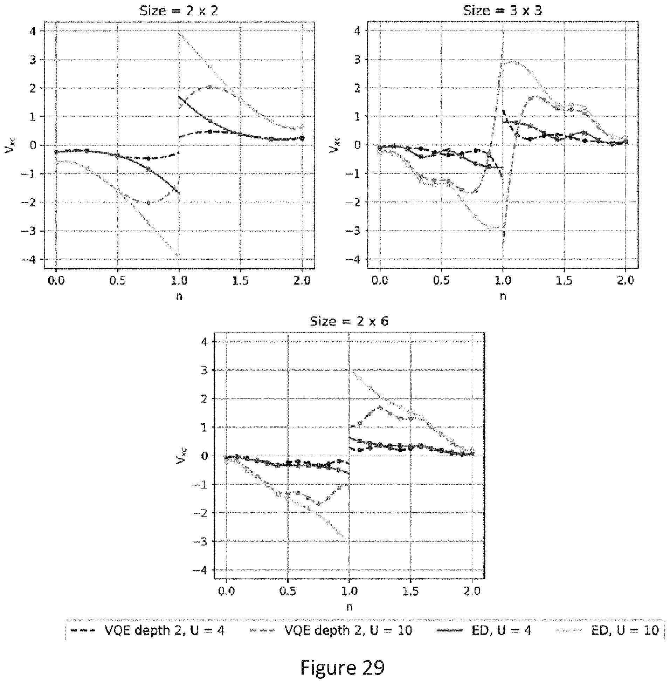

In Figure 29 we extend the analysis to 2D systems and re-examine the effect of size and interaction strength on the VQE functionals. Like in 1D, we see that increasing $U/t$ in 2D also increases the discontinuity in the ED functional, which is mirrored in the VQE functional, albeit with considerably less accuracy than in 1D at larger interaction strengths. As the Bethe ansatz applies in 1D, we do not compare to the BALDA functional in 2D. We highlight that the curvature of the functional in 2D attains a different quality and is visibly different in character for different dimensionalities, e.g. $3 \times 3$ exhibits steps near $n = 1.5$ and $n = 2$, while $2 \times 6$ is mostly monotonic. Notably, the VQE depth 2 functional for the $2 \times 6$ system struggles to reproduce this monotonicity but nevertheless tracks the ED functional closely until eventually incurring significant error near half-filling. This degradation of the functional near half-filling is most striking and is present in all systems illustrated in Figure 29, heavily affecting the splined representation near $n = 1$ for the $3 \times 3$ system. However, despite these shortcomings near half-filling at $U/t = 10$, there is considerably better agreement at $U/t = 4$, indicating that low-depth functionals can visibly approximate the ED functionals for lower values of the interaction strength.

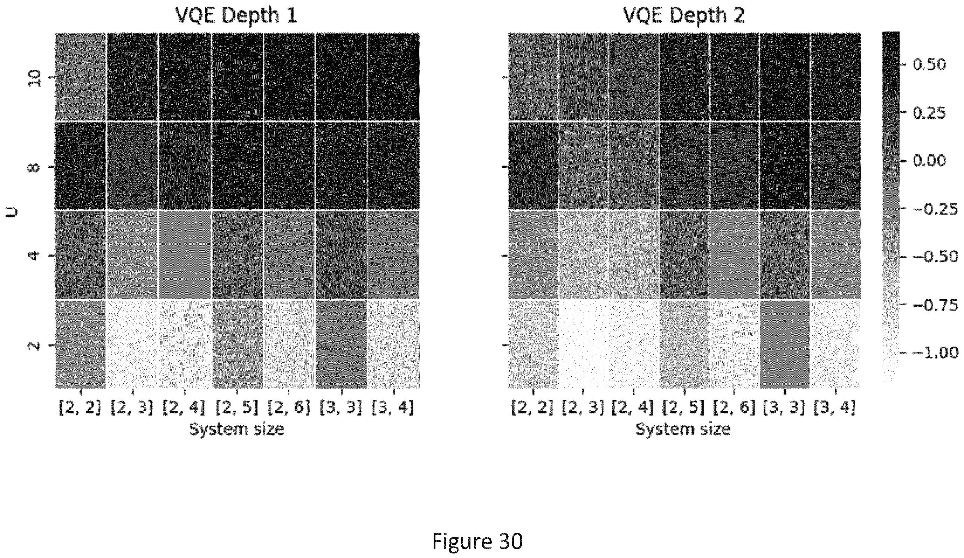

Indeed, in Figure 30 we examine the effect that VQE depth has across a range of 2d system sizes and interaction strengths on the $\log_{10}$ Frobenius norm error between the VQE XC potential functionals and the ED functional. Similar to 1D, it is clear that the leading indicator for quality in the VQE functional is the variational depth at which the quantum simulation is performed. While we observe that the 2d results do not see the same quality of improvements at VQE depth 2 as the 1D systems show, most notably in the $U \geq 8$ regions, we nevertheless do see consistent improvements over depth 1. This suggests that 2D VQE functionals may benefit, especially at larger interaction strengths, from using slightly larger circuit depths, or varying the circuit ansatz as discussed above. Similar to the 1D case, we see that for $U$ values in the region of $U \leq 4$ it is sufficient to use VQE functionals of depth 1 in favour of depth 2 as they retain a similar accuracy.

QEDFT applied to the 1D Fermi Hubbard model

In Figures 31A and 31B we present QEDFT, DFT, VQE, and exact results for the $1 \times 12$ Fermi-Hubbard model with an external quadratic potential $v_i^{ext} = (i-1)^2/L$ with L = $1 \times 12$. The model parameters are $U = 4$, $t = 1$, and $N_e = 6$ (i.e quarter-filling). The DFT algorithm runs for 500 iterations using a density mixing of $\alpha = 0.95$ and the initial density ansatz is chosen to be proportional to the external potential. The exact results are found using exact diagonalisation of the full system.

Figures 31 show the convergence of the energy and the corresponding final density for all methods. Considering the energetics, the QEDFT method clearly achieves better accuracy than both Hartree-Fock DFT alone (Hartree Fock - which by definition has no discontinuity) and pure quantum VQE alone and converges after ~ 100 iterations to $\delta$, the DFT self-consistency criteria, within machine precision. Figures 31 also highlight that despite only having access to qualitatively accurate XC energy functionals via VQE simulations it is possible to outperform popular classical approximations and/or purely quantum approaches. The accuracy of the QEDFT methods nest in order of depth, with the QEDFT depth 2 functional being the closest to the true ground state energy, excluding BALDA and ED functionals, as shown in **Table 3.** Similarly, this trend is observed for pure quantum VQE alone, but with a much larger relative error, though still smaller than Hartree Fock which is the worst overall approximation. Notably the QEDFT depth 2 functional attains a comparable absolute error (~ 0.2) to the classical state-of-the-art functional approximation (BALDA) which is theoretically valid in the thermodynamic limit, but routinely used for finite sized simulations. The ED DFT method converges to the true solution with

$10^{-3}$ accuracy, highlighting the utility of the LDA approximation for this system but also emphasising that it is still approximate in its estimation of groundstate properties.

Similarly, the density also converges to machine precision in the DFT self-consistency criteria. Figures 31 highlight that that the pure quantum VQE methods struggle to reproduce the sharp central peak at the centre of the chain generated by the external quadratic potential and is also considerably erroneous near the boundaries. The QEDFT approach captures these features well over the entire chain and are qualitatively indistinguishable from the state-of-the-art classical methods. Moreover in **Table 3** the errors of the groundstate density from QEDFT are comparable to all of the classical methods. Notably the QEDFT density at depth 1 is more accurate than at depth 2 and we attribute this difference to the interpolation scheme used for the XC energy and potential, which we expect to disappear at higher depths. From this result we see that QEDFT not only can predict the groundstate energy accurately, it is also well suited to predicting the groundstate density, and in particular it is far superior than using pure VQE alone.

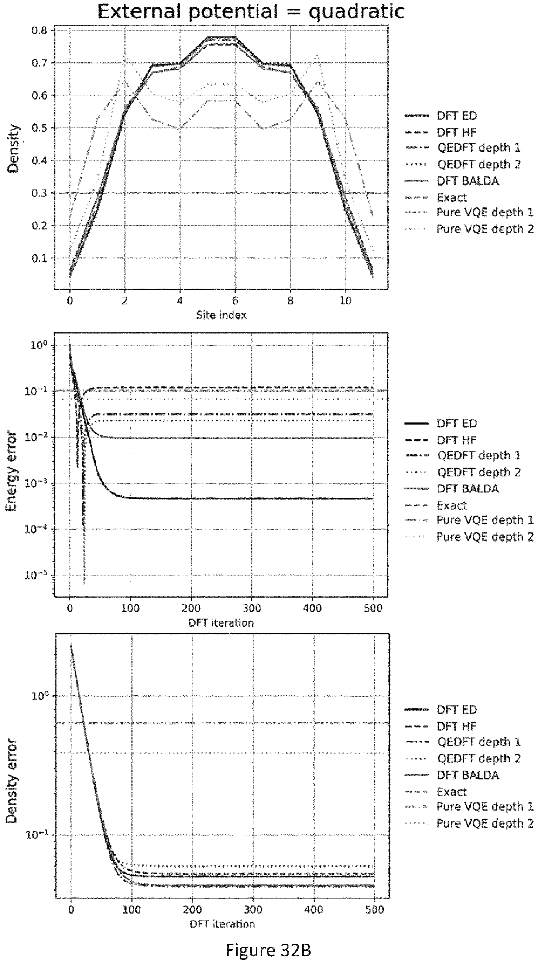

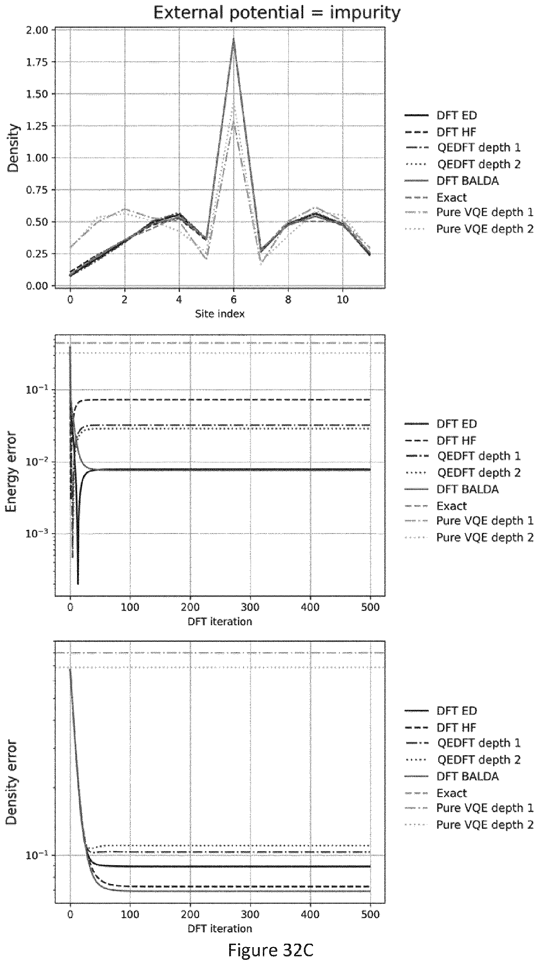

We extend our analysis to understand how QEDFT deals with the impact of changing the type of external potential for the $1 \times 12$ Fermi-Hubbard model. In Figures 32 we highlight the DFT groundstate properties and how they converge for *(i)* no external potential (32A), *(ii)* a confining quadratic potential (32B), and *(iii)* a single impurity potential placed near the centre of the chain (32C). In the top panel of Figures 32A-C we show the groundstate density prediction across all potentials for all methods. The observations we made for the quadratic potential also apply to the case of no potential as well as an impurity in the centre of the system, i.e., the densities from QEDFT are qualitatively always more accurate than pure quantum VQE and comparable to the classical state-of-the-art. We emphasise this by computing the groundstate density error, highlighted in the bottom panel on a semi-logarithmic scale, which clearly shows the order of magnitude difference between pure quantum VQE approaches and the QEDFT approach.

| Table 3 | | |
|---|---|---|
| Method | $\Delta n$ | $\Delta E$ |
| Pure VQE depth 1 | 0.635 | 0.10 |
| Pure VQE depth 2 | 0.388 | 0.067 |
| QEDFT depth 1 | 0.043 | 0.031 |
| QEDFT depth 2 | 0.06 | 0.023 |
| DFT HF | 0.052 | 0.11 |
| DFT BALDA | 0.044 | 0.009 |
| DFT ED | 0.050 | 0.0005 |

**Table** 3: The Frobenius error norm in density $\Delta n = \sqrt{\left(\sum_i^L \left| n_{i,approx} - n_{i,EXACT} \right|^2\right)}$ and error in energy $\Delta E = | E_{approx} - E_{EXACT} |$ after 500 DFT iterations with respect to the exact groundstate.

We draw our attention to the case of no potential, which is equivalent to the homogeneous problem from which the XC functionals are generated, and emphasise that even here the QEDFT approach yields better groundstate density estimates than quantum VQE alone. That is to say, by first doing a quantum VQE computation and then doing a QEDFT computation using the VQE data we are able to arrive at a more accurate description of the groundstate density. The same is not true for the groundstate energies, but the quantum VQE energies and QEDFT energies are almost equivalent, as shown in the middle panel of Figures 32A-C. While for the case of the impurity potential, the QEDFT energies are significantly more accurate than both HF and quantum VQE, essentially following the same conclusions as those we reached for the quadratic potential. These results suggest a wide range of applicability of the QEDFT approach for general homogeneous and inhomogeneous 1d Fermi-Hubbard Hamiltonians, where the QEDFT functionals can provide an avenue for using small quantum machines to improve over pure quantum VQE as well as classical approximations.

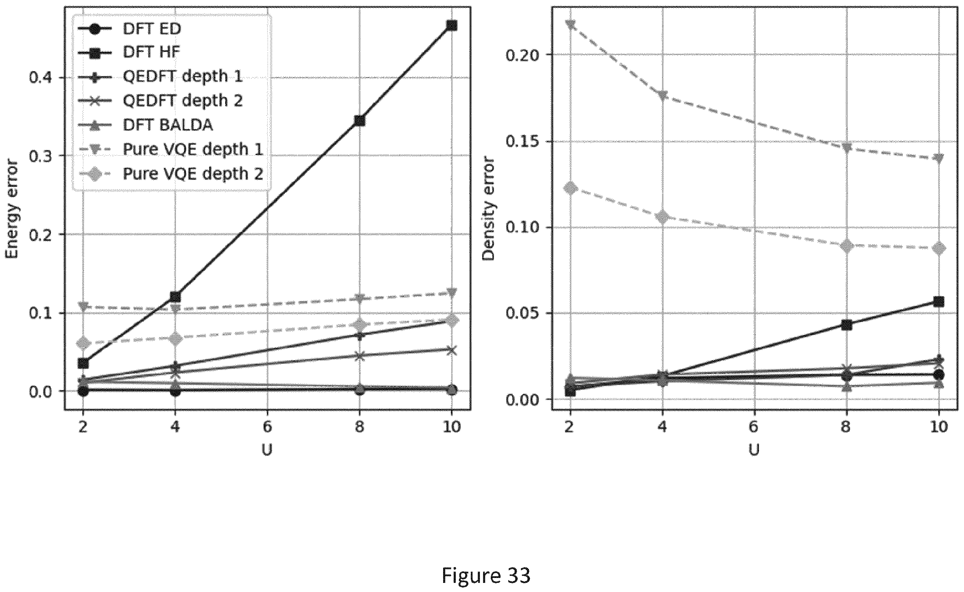

The strength of interactions $U/t$ determines how correlated groundstate of the Fermi-Hubbard model is, which is largely captured within the XC potential $V_{XC}$. As $U/t$ is increased, classical HF theory breaks down in its ability to describe groundstate properties as it explicitly sets the XC potential to zero and reduces the Fermi-Hubbard problem into a purely single particle problem in the absence of exchange and correlation. In Figure 33 we examine the effect of increasing $U/t$ on the groundstate energy and density for the $1 \times 12$ system at quarter-filling within the same confining quadratic potential. This is a direct probe of how well the QEDFT functional can encapsulate XC effects.

In Figures 31A, 31B, and 32A-C we noticed that while QEDFT always outperforms quantum VQE in accuracy for both energy and density metrics, it competed with all classical methods, importantly including HF for the density. In Figure 33 we see that when $U/t \geq 4$, QEDFT consistently is more accurate than HF for both the energy and the density. Indeed, as *U/t* increases, the HF approximation becomes less well justified due to the presence of correlations and at *U/t* = 8 the QEDFT is more accurate than HF. On the other hand, when *U/t* is small the presence of interactions is weaker and the validity of HF is evidenced by its capacity to predict the groundstate density, despite the energy being vastly overestimated. Importantly, this acts as a demonstration that QEDFT functionals, even those which are generated from low-depth VQE circuits, are able to reproduce essential features of correlation that are needed to describe the Mott insulating phase of the Fermi-Hubbard model. Moreover, as *U/t* is increased, the accuracy of the QEDFT functional in predicting the energy deteriorates at a rate that is higher than pure quantum VQE and at *U/t* = 10 the energy error for quantum VQE depth 2 is the same as QEDFT at depth 1. However, the energies relative to HF widen as a function of *U/t* and the QEDFT at depth 2 is always lower than any other quantum method. We also note that these results are sensitive to the choice of external potential but in all cases that we have examined these trends remain the same.

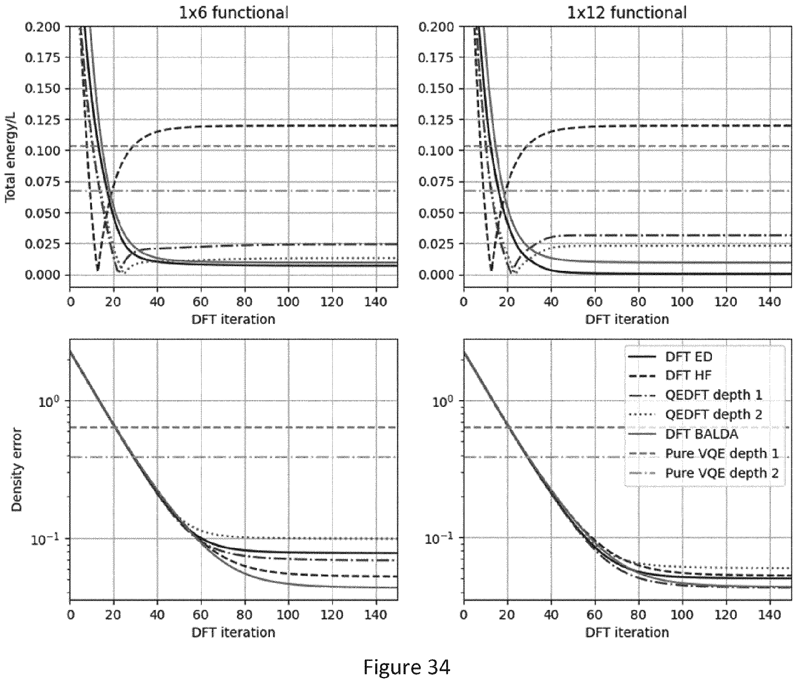

Another pertinent question related to the scalability of the QEDFT method is if the performance of the functional improves as a function of system size. We probe this question by using functionals generated on 1 × 6 and 1 × 12 homogeneous systems applied to 1 × 12 inhomogeneous models (quadratic potential). In the top panel of Figure 34 we see that the error in the energy when using different functionals manifestly improves for the ED functional, indicating that the quality of the solution gets better by using DFT functionals which are generated on larger grids. We note that the DF HF and DFT BALDA solutions in both of these cases are the same, as they are not sensitive to system size. Notably, the QEDFT energetics do not demonstrably improve, rather slightly worsen, when increasing the functional size. We attribute this behaviour to the worsening of the VQE algorithm with system size at fixed depth, and expect this to be resolved by increasing the ansatz depth. However, in the bottom panel of Figure 34 we find that the total error of the density undeniably improves as the system on which the functional is used increases. There is a systematic reduction of the error for each approximation, with BALDA being the most accurate method to predict the density. We note that the quality of these results will be dependent on the inhomogeneous models studied and their parameter settings, but largely we have identified that the above trends hold across the various systems we have examined.

We now turn our attention to much larger many-body systems of interest and study how functionals generated on much smaller homogeneous models can be used to model significantly larger inhomogeneous systems. In Figures 35A and 35B we show the results for a 1 × 200 instance of the Fermi Hubbard model using a functional generated by a 1 × 12 quantum computation. In these simulations we use the BALDA functional as the exact benchmark, as performing ED simulations for these system sizes is not possible. We also note that we do not perform quantum VQE simulations, as the system size is also far too large to do a statevector simulation. The system parameters are *U/t* = 10 at quarter-filling ($N_e$ = 100) within a confining quadratic potential $v_i^{ext} = (i - L)^2/L$. This model is known to exhibit regions of coexisting phases between metallic (compressible) and Mott insulating (incompressible) characteristics as shown in [G. Xianlong et al. "Bethe ansatz density-functional theory of ultracold repulsive fermions in one-dimensional optical lattices". In: Physical Review B 73.16 (2006), p. 165120]. A typical example of such a scenario is illustrated in Figures 35A and 35B in the BALDA density profile at the centre of the trap, where a plateau emerges where the density is constrained to $n_i$ = 1. This plateau is determined by the competition between $v_i^{ext} + Un_i/2$ and $V_{XC}$ in the KS potential, which are separated by the XC potential discontinuity at $n_i$ = 1 (Figure 31). Importantly, the QEDFT methods capture the coexistence of the localised incompressible region with that of the metallic compressible phase, noting that this functional is generated using a 1 × 12 system. While QEDFT depth 2 is far more robust in this regard we remark that QEDFT depth 1 is also capable of capturing qualitative features of these phases, despite bearing resemblance to the HF solution within the interior of the trap. Notably, these features are entirely absent from the HF result, demonstrating that the low depth QEDFT functionals are qualitatively superior in their descriptive capabilities of coexisting phases that are of a correlated nature. The noise in the density and energy is a known issue with applying BALDA in locally incompressible regions due to the discontinuity in the XC functional that results in convergence issues for DFT, as shown in [G. Xianlong et al. "Bethe ansatz density-functional theory of ultracold repulsive fermions in one-dimensional optical lattices". In: Physical Review B 73.16 (2006), p. 165120]. Nevertheless, we observe that the groundstate QEDFT energies are closer to that of BALDA than Hartree Fock, which further support the capabilities of low depth QEDFT functionals applied to larger systems.

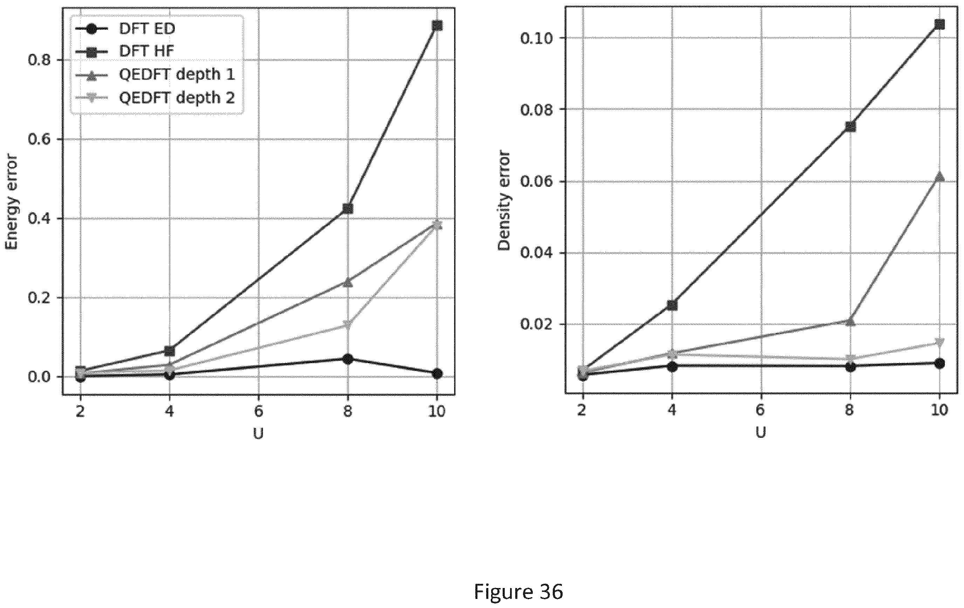

As in the case of the smaller systems, we present the effect of varying the interaction strength *U/t* on the 1 × 200 Fermi-Hubbard system confined to the same quadratic potential at quarter-filling in Figure 36. The errors are measured relative to the BALDA DFT method. In contrast to the 1 × 12 results presented in Figure 33, here we see that for all values of *U/t* that the QEDFT density is more accurate than the HF density, which is similarly true for the energy. The trends worsen as a function of *U/t,* even for the ED functional, and the energy errors are noticeably larger for large *U/t* compared to the 1 × 12 system. We attribute this to the known issue of sampling near unity in the compressible phase with DFT methods that use functionals with XC discontinuities. At *U/t* = 10 we see for QEDFT depth 1 that there is a jump in the density error, which we associate with the emergence of the Mott plateaus near the centre of the trap. For lower values of *U/t,* the QEDFT functionals are reliably comparable to the BALDA solution and never exceed a 3% error.

QEDFT applied to the 2d Fermi Hubbard model

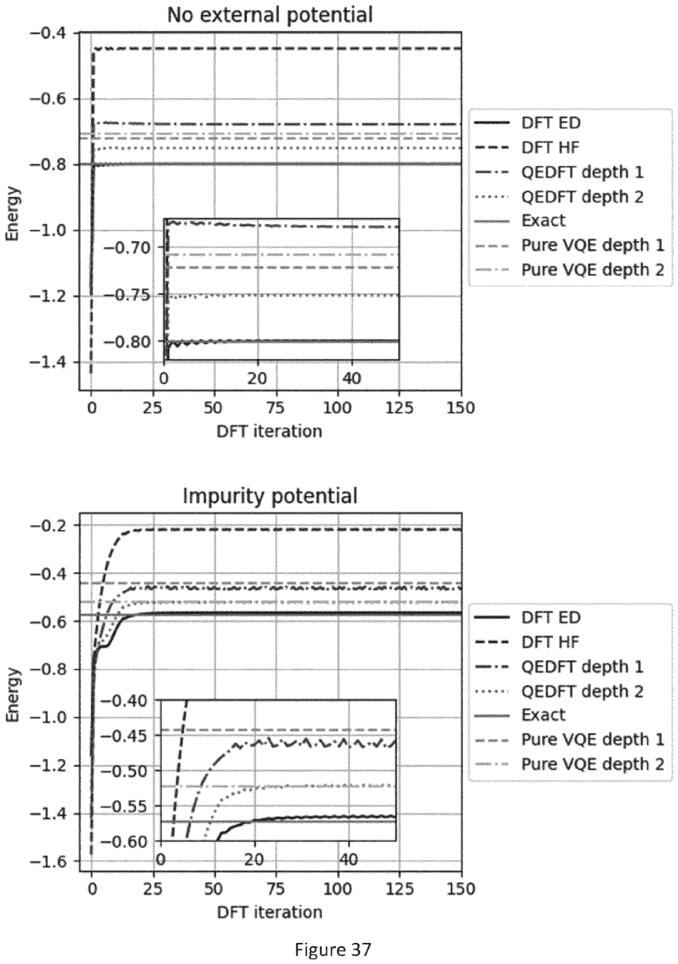

We now proceed with the DFT analysis of Fermi-Hubbard models in 2D, beginning with the energy convergence of the DFT energy, presented in Figure 37 for the $3 \times 3$ system with $U/t = 10$ at quarter filling. We consider the homogeneous model as well as the inhomogeneous system with an impurity potential embedded in a random background at the (1,2) site. For the homogeneous model we observe that the convergence of DFT energy is initially unstable, as evidenced by the jagged spikes for the first 50 iterations. This behaviour is present in all functionals studied, indicating that this instability is not associated with the particular form of the XC potential but rather the stability of the single particle Kohn-Sham approach for the chosen parameter regime. A high density mixing parameter ($\alpha = 0.95$) ensures that early instabilities cannot propagate towards erroneous fixed points.

For the homogeneous model it is clear that in all cases using a VQE approach is superior to Hartree Fock, which has an error in the converged energy of $\approx 44\%$, in contrast to the most accurate VQE method (QEDFT at depth 2) $\approx 6\%$. Indeed, we further see that QEDFT at depth 2 attains an accuracy closer to the exact solution than pure VQE alone, which is not achieved at QEDFT depth 1. In contrast to the 1D results, this suggests that going beyond depth 1 QEDFT functionals is not only beneficial, but also necessary, to improve upon using pure VQE. Finally, the ED functional produces the groundstate energy with an error of $\approx 0.1\%$, indicating that the local density approximation when sampled at the converged density produces a good, but not exact, estimate of the groundstate energy for 2D systems also. Considering the inhomogeneous model with the impurity centred at (1, 2), we reach the same primary conclusion that QEDFT outperforms the HF solution and is on par with, and sometimes more accurate than, pure VQE. In particular, the HF energy is $\approx 63\%$ away from the exact solution, while the QEDFT depth 2 energy is $\approx 8\%$ away. We also see that the inhomogeneities introduce more noticeable instabilities in the DFT algorithm, as evidenced by the jaggedness of the energy convergence around a fixed value. While not severe in this case, a combination of more accurate XC interpolation methods and higher density mixing parameters should remove convergence to multiple fixed points. In summary, the energetic accuracy of low variational depth QEDFT functionals worsens for 2D systems as contrasted against 1D systems, but nevertheless show promise as in all cases QEDFT outperforms HF solutions, and in most cases pure VQE.

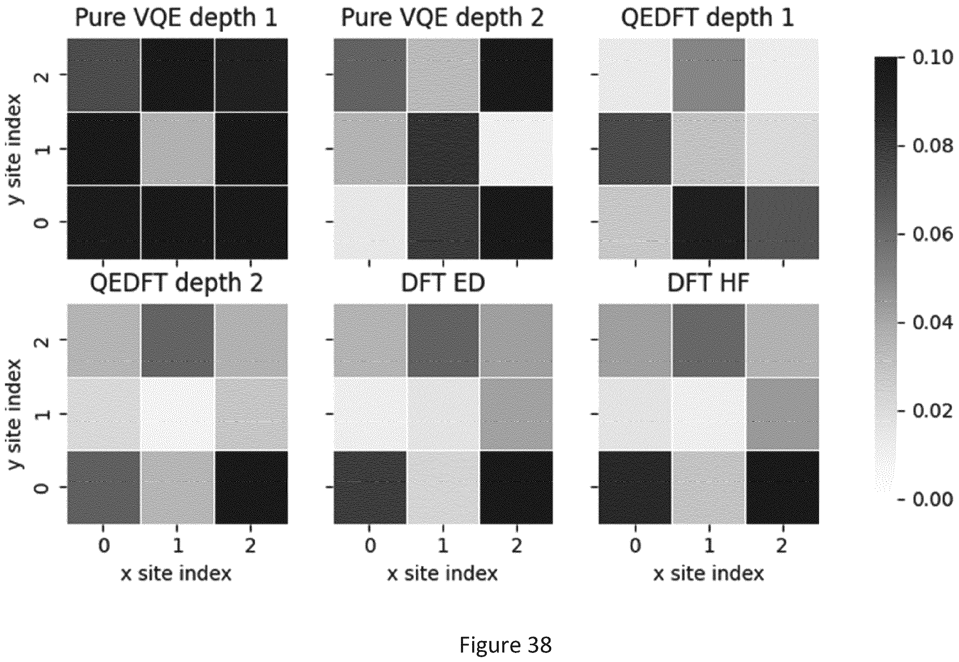

In Figure 38 we analyse converged groundstate densities for the inhomogeneous $3 \times 3$ system by presenting the site density percentage error with the groundstate density,

$$\Delta n_{ij} = \frac{\left| n_{ij}^{\mathrm{approx}} - n_{ij}^{\mathrm{exact}} \right|}{n_{ij}^{\mathrm{exact}}}.$$

$$(65)$$

We arrive at a set of slightly different conclusions for the DFT groundstate energies as compared to the energies. For the densities, the DFT methods are always more reliable than pure VQE, in particular depth 1 does not even reliably find the impurity location at site (1, 2). Indeed, pure VQE produces errors per site that are mostly all above 0.1%, while the QEDFT functionals often obtain errors per site that are less than 0.1%. QEDFT at depth 2 highly resembles the DFT ED and DFT HF solutions, indicating that low-depth QEDFT methods can compete with classical state-of-the-art approaches for predicting the groundstate density in 2D. Moreover, despite potential errors arising in the interpolation of the XC potential due to rogue data points, i.e., as seen in Figure 29 for the $3 \times 3$ case, we see that the DFT algorithm can be robust against these, depending on where the density is sampled. If the curvature of the underlying function is non-trivial, which it is for the $3 \times 3$ system, improvements can be made by using denser meshes to assist the interpolated XC potential and prevent DFT from driving the density to a result that is further away from the exact one but are not necessary for this inhomogeneous model. Again, we arrive at a similar set of conclusions for the converged DFT density as we did for the one 1D models studied in Figures 32, albeit there we used a global density metric rather than a per site one.

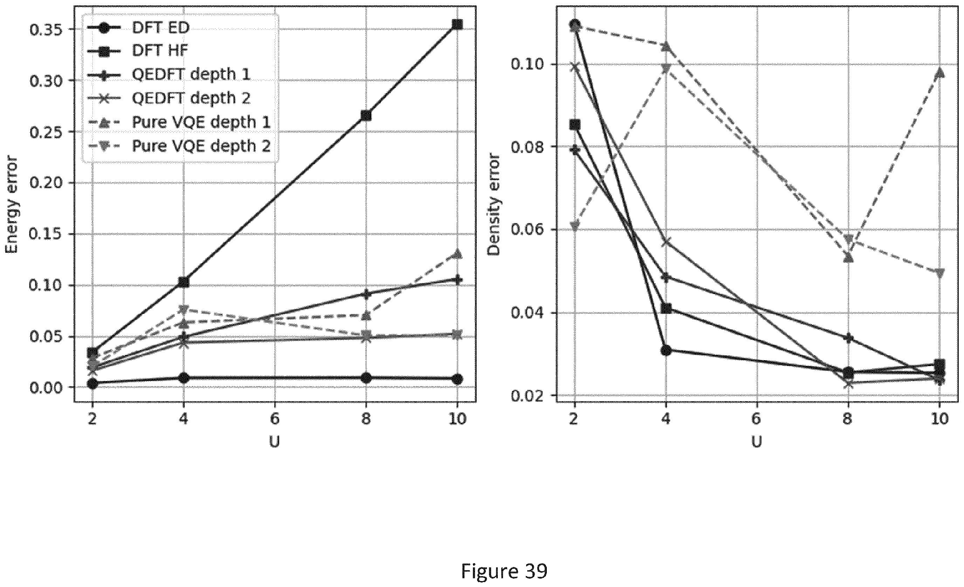

To quantify this error further we study the effect of varying $U/t$ on the absolute errors with respect to the groundstate energies and densities for the quarter-filled $3 \times 3$ inhomogeneous impurity system, illustrated in Figure 39. The results for the energetics are reminiscent of those presented for 1D systems in Figure 33. Firstly, in all cases the QEDFT VQE functional at depth 2 provides more accurate energetics that pure VQE alone, in addition to classical HF. As $U/t$ increases the energy error increases for all methods, with the exception of QEDFT at depth 2, as this remains the same. Moreover, we see that the DFT ED functional for all $U/t$ provides excellent estimates of the exact groundstate energies, which indicates as the QEDFT functional depth increases then so will its accuracy. The density results, however, deviate significantly from what we observed in 1D. Indeed, as $U/t$ increases the density error decreases, which opposite to the trend we observed for the 1D quadratic potential. Even at low $U$, the DFT ED functional does not reproduce the exact solution, indicating that the LDA approximation for the 2D Fermi Hubbard model at low $U/t$ is not as reliable as in 1D for predicting the groundstate density. However, as $U/t$ increases the density error decreases for all DFT methods even for the pure VQE methods. Generally speaking, the DFT methods, and in particular the QEDFT functionals, always provide more reliable densities than pure VQE alone across all values of $U/t$, except at $U/t = 2$, where pure VQE at depth 2 is the most accurate approach.

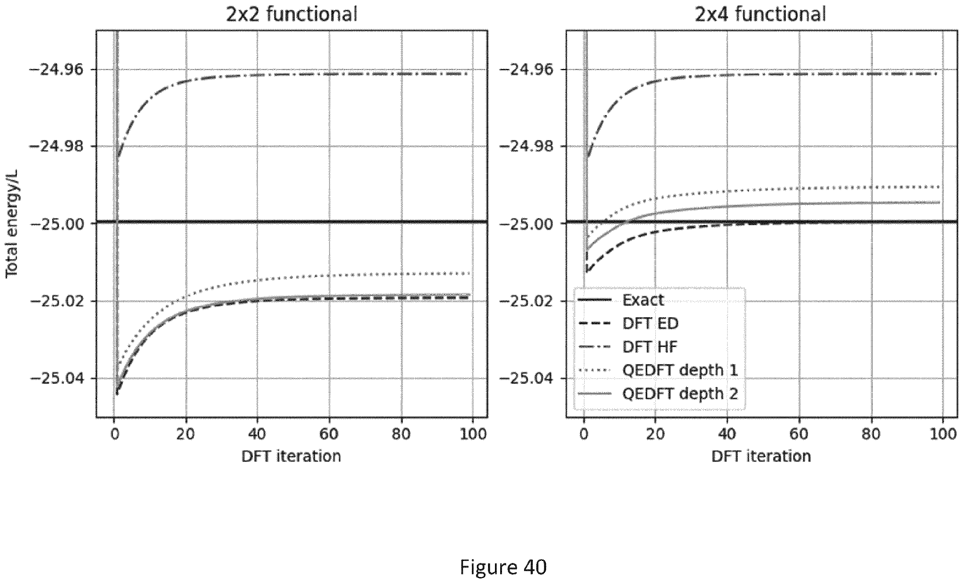

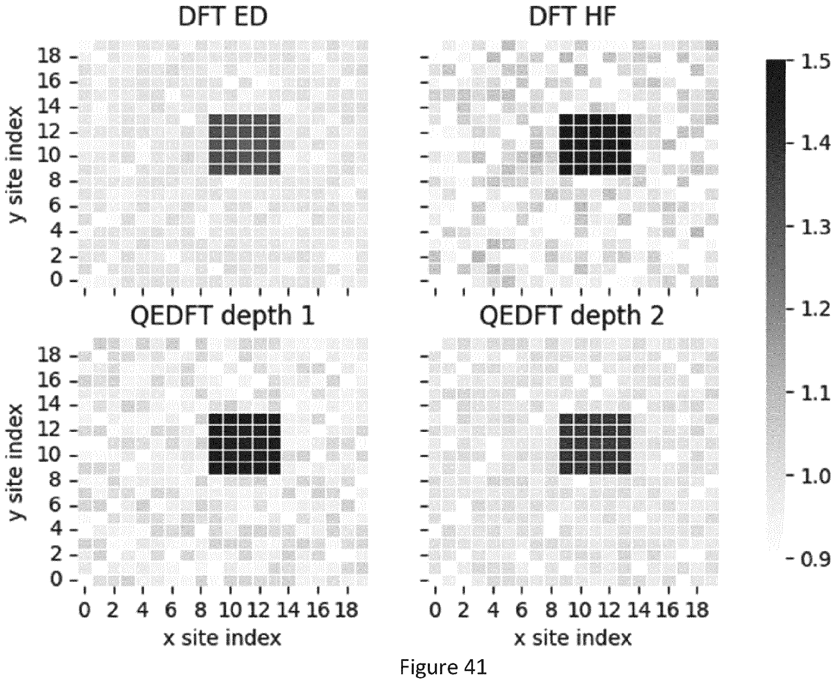

We now revisit examining in 2D how well QEDFT scales as the functionals used are increased in the system size. In Figure 40 we show that studying the inhomogeneous $2 \times 4$ Fermi-Hubbard system with an impurity at site (1,2) improves considerably when going from a functional generated on a $2 \times 2$ lattice, as compared to using a functional from a $2 \times 4$ system. Notably, the DFT ED results converge to the exact method using the $2 \times 4$ functional, demonstrating the utility of how the functional can scale with system size. Moreover, the QEDFT energetics also converge to values which are closer to the exact value, but are not as close as DFT ED. The pure VQE energetics are not shown, as they incur an error of approximately $\sim 20\%$, as opposed to the DFT methods, which are all within the region of less than $\sim 0.2\%$. For 2D we did not observe a significant improvement related to the density in 2D, in contrast to the results for 1D, despite the scaling in the energetics being more systematic in the QEDFT approach. We now extend our analysis to a $20 \times 20$ inhomogeneous Fermi-Hubbard system for $U/t = 4$ at quarter-filling. The inhomogeneities are generated by a cluster of impurities at the centre of the lattice in the presence of a weak background disordered potential. Exact solutions for this system would require diagonalising a matrix of dimensions $2^{20 \times 20} \times 2^{20 \times 20}$, which is not possible with classical computers. DFT, on the other hand, only requires the diagonalisation of a matrix with dimensions $(20 \times 20) \times (20 \times 20)$ which can be easily achieved. Figure 41 shows the DFT densities for the $20 \times 20$ using the $3 \times 3$ QEDFT XC functional (Figure 29). It is clear that all of the DFT methods are able to distinguish where the impurities are located. The absence of an exact method for large 2D systems (unlike 1D systems where the BALDA functional exists, as well as DMRG) means there is no ground truth comparison, and we currently rely on basing our judgement on the behaviour of functionals from smaller 2D systems, as well as in 1D. We see that the QEDFT functional of depth 2 is more in likeness to the ED result, both in contrast to the classical HF functional and QEDFT depth 1. Indeed, these results are similar to those we obtained for the densities of the $1 \times 200$ Fermi-Hubbard system (Figure 36), where QEDFT of depth 1 approximates the HF solution better than the exact solution. In that case, this deficiency limited the functional in describing Mott physics in the system's groundstate. Finally, we see that QEDFT depth 2 is close to DFT ED in Figure 41, and despite the lack of an exact solution for the ground state density, it is at least suggestive that QEDFT at depth 2 is incorporating effects that are not present in the HF solution.

## Hardware Example - Google

To analyse the performance of QEDFT on quantum hardware, we were able to make use of previous Fermi-Hubbard VQE experiments that had been carried out on Google's 23-qubit Rainbow chip as reported in [S. Stanisic et al. "Observing ground-state properties of the Fermi-Hubbard model using a scalable algorithm on a quantum computer", in: Nature Communications 13.1 (2022), p. 5743]. The three instances of the model that were considered in the paper and that we shall consider here are: the $1 \times 4$ Fermi-Hubbard model with VQE depth 2; the $1 \times 8$ Fermi-Hubbard model with depth 1; and the $2 \times 4$ Fermi-Hubbard model with depth 1. For each of these models we generate a QEDFT XC potential, which we refer to as the QEDFT-HW functional, and we then use it inside a DFT loop for the original homogeneous system and complementary inhomogeneous systems. We do not change the preset DFT parameters, unless specified. Note that the ansatz that was run on the hardware is not identical to the simulated ansatz discussed at the beginning of the numerical results section, which is why the hardware may sometimes produce different results to simulated VQE, up to noise.

We first examine the effect that system size has on the XC potential for the 1D systems in Figure 42. The $1 \times 4$ system shows the most promising results, where the hardware results consistently fall between the simulated VQE depth 2 and DFT ED XC potentials, and we do not observe the presence of large amounts of noise. However, as the system size is doubled to $1 \times 8$ we immediately notice the manifestation of noise in in the hardware functional, which has effects on both the raw data as well as the subsequent interpolation, in particular in the density region near 1. We also see that the density discontinuity is amplified, which will have an effect on the DFT algorithm when sampling the density near 1. Finally, the presence of hardware noise is most noticeable in the $2 \times 4$ example, where the QEDFT-HW functional deviates from the simulated results for the entire density region between 0 and 2. For this case we see that the cubic spline interpolation begins to fit the noise, rather than filter through it, indicating the sensitivity of this approach in the presence of larger amounts of noise.

We analyse the effect of this noise by implementing first the QEDFT functional for the $1 \times 8$ model for different homogeneous and inhomogeneous $1 \times 8$ Fermi-Hubbard models. We choose this model as it represents the case where the XC potential possesses significant hardware noise, in order to assess how this manifests in the DFT results. The results are shown in Figures 43A-C. Despite the presence of noise, the hardware data achieves more accurate results than brute force simulated VQE at depth 1 and 2 in all scenarios, for both the energy and density. The QEDFT-HW (where HW stands for 'hardware') energetics consistently achieve accuracies relative to the true energy between $10^{-1}$ and $10^{-2}$, which is significantly better than the classical HF results. While the QEDFT-HW densities do not outperform the classical HF densities, they are certainly similar in accuracy to the QEDFT simulated functionals, which can be improved by including more variational layers. Indeed, even though there is the clear presence of noise in the QEDFT-HW functional, manifest in both the raw data as well its interpolation, we show that its subsequent application in a DFT simulation is largely oblivious to these details for the model parameters we have considered.

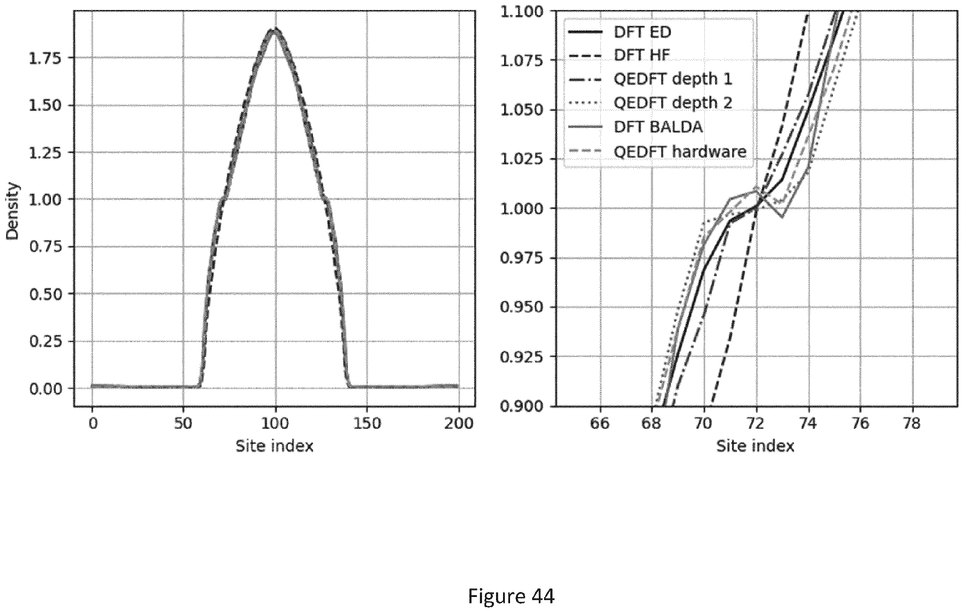

To take this observation even further, we apply the $1 \times 8$ QEDFT-HW functional to the much larger $1 \times 200$ inhomogeneous Fermi-Hubbard model in the presence of an external quadratic potential at $U/t$ = 4. The QEDFT-HW functional captures the presence of Mott plateaus near n = 1, as shown in Figure 44 and previously discussed in relation to Figure 36, which is clearly not present in the classical single particle HF solution. This highlights that not only is the QEDFT-HW functional capable of reproducing the groundstate properties for small Fermi-Hubbard systems, they can be used for much larger systems, and reproduce physical features in strongly correlated regimes that classical single particle solutions omit.

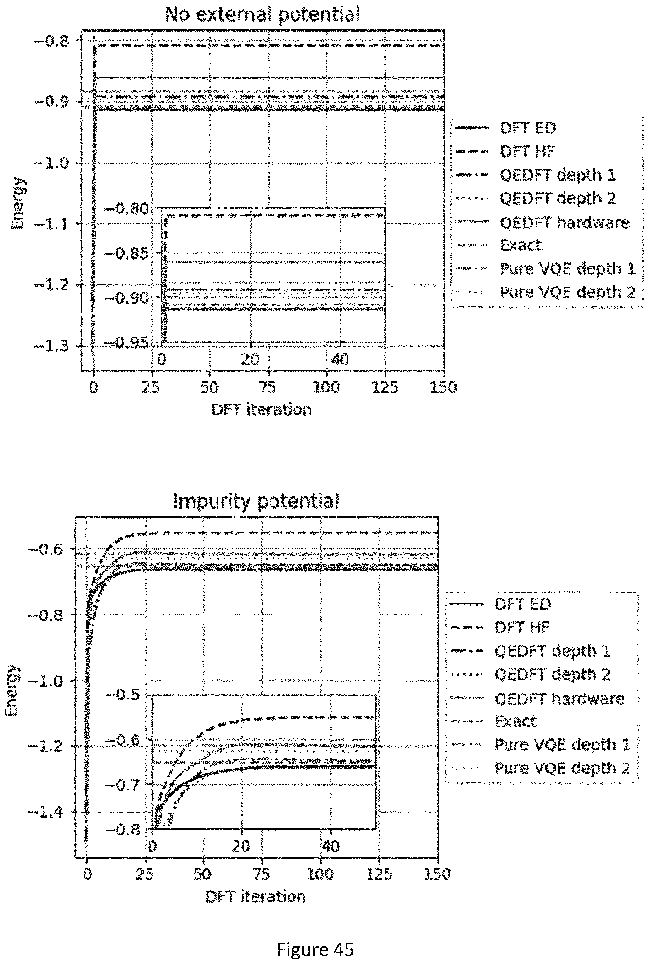

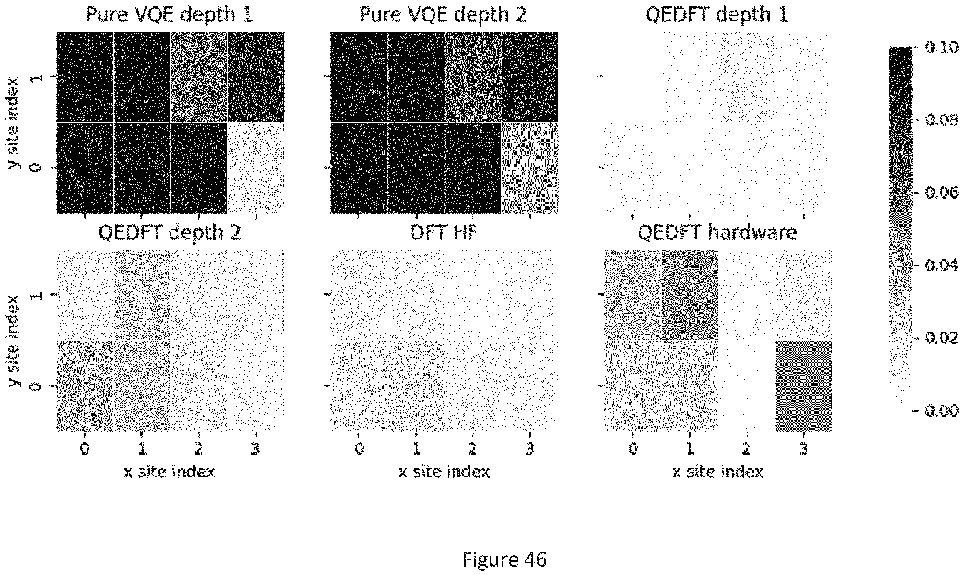

We now study the $2 \times 4$ Fermi-Hubbard system using the hardware functional without an external potential and in the presence of a single impurity at the (1,1) site embedded in a disordered external potential. The results are shown in Figure 45 and Figure 46. For both the homogeneous and inhomogeneous models we see that that all DFT methods are stable as the algorithm converges without any instabilities, in contrast to the $3 \times 3$ simulations. The origin of these differences is possibly related to the behaviour of the XC functional near half-filling as we presented in Figure 29, which is noticeably worse for the square $3 \times 3$ lattice than for rectangular lattices. Indeed, this indicates that errors originating from the splining of the XC functional, as shown for the $2 \times 4$ case in Figure 42, have less of an impact on the stability of the DFT algorithm than absolute error of the raw data to the exact reference values. Most notably we find that the DFT hardware energetics, while consistently an improvement upon classical HF, is either slightly worse or essentially no better than exact emulations of pure VQE. As there is no hardware data for the direct implementation of VQE for these target problems we cannot directly compare DFT VQE to pure VQE on hardware but we anticipate DFT VQE to outperform the latter as we consistently observed in emulation.

The hardware data is also used to assess the accuracy of DFT for the groundstate density of the inhomogeneous $2 \times 4$ problem in Figure 46. Interestingly, the hardware functional produces groundstate densities a lot closer than pure VQE emulation, having a per-site percentage error of less than 0.1% for all sites, while most sites predicted with the pure VQE emulation have errors greater than 0.1%. As was observed for the emulations of the $3 \times 3$ model, the DFT methods based on VQE are comparable to classical HF, which is also true for the hardware functional in this case. Notably, the QEDFT depth 1 densities produce results that are closer to the true solution than QEDFT depth 2. Indeed, this is also reflected in the accuracy of the energetics and is related to the quality of the interpolation method performed. Due to the finite data set over which the XC functional is constructed, it is possible to introduce error at higher depths within the interpolated region if the spacing is not large enough, with respect to lower-depth results. Therefore, we anticipate that classical improvements to the interpolation protocol of QEDFT should make it possible to obtain groundstate properties closer to the true results.

Conclusion

Density Functional Theory is the de facto standard for simulating molecular and materials groundstate properties on traditional computers. Its overwhelming popularity and success relies on choosing appropriate classical approximations to the unknown exchange correlation energy functional on a system-by-system basis. For many cases, especially those with strongly correlated electrons, the classical approximations are inadequate, and sophisticated approaches beyond DFT are needed that are not efficiently scalable on traditional computers. Simulating materials is regarded as a promising application for quantum computers and has so far largely been viewed through the lens of hybrid quantum/-classical algorithms to make the most of near-term noisy quantum resources. In this work we have introduced a new hybrid quantum/classical approach called *quantum-enhanced DFT* (QEDFT) that combines DFT with quantum computation. We have presented this formalism in generality within the specific framework of lattice DFT and applied it to various instances of the 1D and 2D Fermi-Hubbard models. Low depth VQE simulations and hardware experiments are used to construct approximations to the XC energy functional, which are then available to be used as part of the DFT algorithm.

In the majority of cases considered, QEDFT outperforms pure quantum VQE alone, with respect to the accuracy of the groundstate energy and density of both the 1D and 2D inhomogeneous Fermi-Hubbard model. It also provides energetics which are more accurate than classical Hartree Fock theory. QEDFT captures exact properties of the XC potential functional, such as the derivative discontinuity at half-filling, which are not present in many well-known classical approximations. The derivative discontinuity and groundstate properties from QEDFT show impressive robustness to hardware noise and shallow variational layers. DFT is an iterative, self-consistent approach that can be prone to numerical instabilities, but we see that choosing conservative parameter settings, such as high DFT density mixing, help drive the QEDFT algorithm into a unique, converged solution. A hallmark of the QEDFT method is that it does not rely on highly accurate quantum computations to produce groundstate properties of either inhomogeneous or homogeneous lattice systems. Another quintessential feature is that QEDFT can be applied to large many-body condensed matter systems using only small quantum hardware. We achieve this by generating continuous representations of the QEDFT XC potential using classical interpolation schemes. In particular, we have shown this by solving $1 \times 200$ and $20 \times 20$ Fermi-Hubbard models using QEDFT, demonstrating that shallow VQE functionals of at most two variational layers can unambiguously capture Mott plateaus in the groundstate densities of large inhomogeneous models.

These techniques can be applied outside of the bounds of the situations discussed above, for example in applying QEDFT beyond Fermi-Hubbard models to realistic atomic systems by broadening the scope of the inhomogeneous Hamiltonians under consideration. In particular, modifications to the procedure may include additional effects to enhance the descriptive capabilities of QEDFT functionals to capture a rich variety of important physical processes such as multiorbital couplings, beyond nearest-neighbour interactions, lattice connectivity, and electron-phonon interactions, to name only a few important effects. Another important consideration for realistic systems is the effect of the underlying basis set used to represent continuous inhomogeneous models. This choice is known to have significant technical consequences for how to setup the DFT problem and has recently been explored in the context of quantum simulation in [A. V. Ivanov et al. "Quantum computation for periodic solids in second quantization". In: Phys. Rev. Res. 5 (1 2023), p. 013200]. Other possibly modifications, such as to the basis set, can be made when building QEDFT XC functionals that can be used for real atomic systems, preferably the optimal representation of the homogeneous problem is used to construct it. Additional effects, including non-local effects from the density into the QEDFT functional, in the spirit of the classical GGA approximation, suggest themselves as complementary ways in which the QEDFT method can be modified for more general application.

In conclusion, QEDFT presents a new way in which to bring together DFT with quantum simulation, that is applicable across a broad range of physical fermionic Hamiltonians describing real atomic, molecular, and materials systems. It has the benefits of being noise resilient and only requires modest quantum resources on small quantum machines that can produce accurate groundstate properties of systems not able be simulated directly. We have implemented QEDFT for the prototypical Fermi-Hubbard lattice model and demonstrated that it is a powerful hybrid quantum/classical approach that can compete with state-of-the-art classical methods and outperform conventional variational quantum algorithms. QEDFT holds the potential to optimise the utility of near-term quantum machines for accurate simulations of many-body quantum systems such as materials and molecules.

## **Clauses**

The disclosure also extends to the following clauses.

Clause 1. A method of performing improved Kohn-Sham Density Functional Theory, DFT, calculations, for simulation of a material, chemical, or biological system, comprising the steps of:

(i) constructing a model Hamiltonian of the system to be simulated;
(ii) inputting an electron density of the system to be simulated;
(iii) constructing a Kohn-Sham potential by:

(1) providing a second Hamiltonian based on the model Hamiltonian;
(2) calculating a ground state energy of the second Hamiltonian, as a function of electron density, using a quantum algorithm executed by a noisy intermediate-scale quantum, NISQ, processor;
(3) computing a quantum-obtained exchange correlation energy functional, as a function of electron density, from the NISQ processor-obtained ground state energy of step (2);
(4) obtaining a quantum-obtained exchange correlation potential functional by differentiation of the quantum-obtained exchange correlation energy functional from step (3);
(5) using the quantum-obtained exchange correlation potential functional from (4) to construct the Kohn-Sham potential; and

(iv) executing a quantum Kohn-Sham DFT algorithm iteration using the electron density from (ii) and the Kohn-Sham potential from (iii) to compute an updated electron density and an updated total energy of the system to be simulated, optionally wherein computing an updated electron density comprises computing $n_{GS,i} = 2\sum_{j}^{occ/2}\left|\phi_j(i)\right|^2$ and mixing it with the previous density estimate, $n^{(j+1)} = \alpha n^{(j)} + (1 - \alpha)n^{(j+1)}$, where $j$ is a DFT iteration index and $\alpha$ is a linear mixing parameter.

Clause 2. A method according to clause 1, wherein providing the model Hamiltonian in step (i) includes either:

providing an inhomogeneous Hamiltonian; or
providing a homogeneous Hamiltonian, wherein the homogeneous Hamiltonian is the inhomogeneous Hamiltonian with an external potential removed.

Clause 3. A method according to clause 1 or clause 2, wherein when the model Hamiltonian provided in step (i) is inhomogeneous then the second Hamiltonian provided in step (1) is homogeneous, further wherein the model Hamiltonian has an external potential and the second Hamiltonian is based on the model Hamiltonian with the external potential removed.

Clause 4. A method according to any one of the preceding clauses, wherein calculating the ground state of the second Hamiltonian on an NISQ processor further comprises using a fermion-to-qubit encoding to encode qubits of the NISQ processor.

Clause 5. A method according to clause 4, wherein the fermion-to-qubit encoding is a Jordan Wigner encoding that maps fermionic creation and annihilation operators of the second Hamiltonian to Pauli strings comprising Pauli X, Y and Z operators.

Clause 6. A method according to any one of the preceding clauses, wherein calculating the ground state of the second Hamiltonian on an NISQ processor further comprises enacting qubit operations generated by unitary qubit operators on the qubits of the quantum information processor.

Clause 7. A method according to any one of the preceding clauses, wherein the model Hamiltonian is the Hubbard model with an external potential term, and the second Hamiltonian is the Hubbard model.

Clause 8. A method according to any one of the preceding clauses, wherein execution of the quantum algorithm by a noisy intermediate-scale quantum, NISQ, processor further comprises executing a Variational Quantum Eigensolver, VQE, algorithm using the NISQ processor.

Clause 9. A method according to clause 8, wherein the VQE algorithm is implemented as a VQE circuit which is lower depth than VQE circuits required to find the ground state of the model Hamiltonian directly.

Clause 10. A method according to clause 8 or clause 9, wherein the VQE algorithm uses a Hamiltonian Variational Ansatz, HVA.

Clause 11. A method according to any of the preceding clauses, wherein obtaining a quantum-obtained exchange correlation potential functional in step (4) further comprises using a quantum kernel method for continuous representation of the exchange correlation energy functional as a function of electron density.

Clause 12. A method according to clause 11, wherein the quantum kernel method comprises interpolative and/or extrapolative procedures for obtaining the exchange correlation energy functional as a function of electron density.

Clause 13. A method according to clause 11 or clause 12, wherein the quantum kernel method further comprises using a Local Density Approximation, LDA, to construct a continuous representation of the exchange correlation energy functional.

Clause 14. A method according to any of the preceding clauses, wherein the material system to be simulated is a strongly correlated electron system.

Clause 15. A method according to any of the preceding clauses, wherein the Kohn-Sham DFT algorithm is a Kohn-Sham Lattice Density Functional Theory, LDFT, algorithm.

Clause 16. A method according to any of the preceding clauses, wherein the NISQ processor requires fewer qubits to calculate the ground state of the second Hamiltonian than the number of qubits required to calculate the ground state of the model Hamiltonian of the system to be simulated directly.

Clause 17. A method according to any of the preceding clauses, wherein executing a quantum Kohn-Sham DFT algorithm iteration in step (iv) comprises the steps of:

> (a) taking the electron density from (ii) and the Kohn-Sham potential from (iii) as an input to construct Kohn-Sham equations;
> (b) solving the Kohn-Sham equations;
> (c) providing the updated electron density, and the updated total energy of the system to be simulated, based on solutions of the Kohn-Sham equations in step (b); and
> (d) checking if a convergence condition is met, further wherein the condition for convergence is evaluated based on the electron density input in step (ii) and the updated electron density.

Clause 18. A method according to clause 17, wherein evaluating the convergence condition in step (d) comprises calculating:

> (a) a density difference metric from the electron density input in step (ii) and the updated electron density; and/or
> ($\beta$) an energy difference metric from a total energy of the system to be simulated, based on the electron density from step (ii), and the updated total energy of the system to be simulated,

and comparing the density difference metric from (a) and/or the density difference metric from ($\beta$) with a threshold condition for each difference metric.

Clause 19. A method according to clause 18, wherein further quantum Kohn-Sham DFT algorithm iterations, are performed by providing the updated electron density from (c) as the input in step (ii) if the convergence condition in (d) is not met such that steps (ii) to (iv) continue until the convergence condition for the electron density, and/or the convergence condition for the total energy of the system to be simulated, is met in step (d).

Clause 20. A method according to clause 18, wherein a further Kohn-Sham DFT iteration is performed in a classical manner, the classical Kohn-Sham DFT iteration comprising the steps (ii) to (iv), wherein step (ii) receives the updated electron density from (c) as an input and step (iii) replaces steps (1) to (5) by providing a classically obtained exchange correlation potential functional as an input to construct the Kohn-Sham potential.

Clause 21. A method according to clause 20, wherein further classical Kohn-Sham DFT iterations are performed until the convergence condition for the electron density, and/or the convergence condition for the total energy of the system to be simulated, is met in step (d).

Clause 22. A method according to clause 20, wherein further quantum or classical Kohn-Sham DFT algorithm iterations are performed, including at least one further quantum Kohn-Sham DFT iteration, until the convergence condition for the electron density, and/or the convergence condition for the total energy of the system to be simulated, is met in step (d).

Clause 23. A method according to clause 20 or clause 22, wherein further quantum Kohn-Sham DFT iterations are performed at periodic intervals with classical Kohn-Sham DFT algorithm iterations in between, until the convergence condition for the electron density, and/or the convergence condition for the total energy of the system to be simulated, is met in step (d).

Clause 24. A method according to any one of clauses 18 to 23, wherein the updated electron density, and the updated total energy of the system to be simulated, from step (c) are used to construct ground state properties of the model Hamiltonian describing the material system which is to be simulated.

Clause 25. A method according to any of the preceding clauses, further comprising, prior to steps (i) to (iv), the step of performing at least one classical Kohn-Sham DFT algorithm iteration using a classical exchange correlation potential functional.

Clause 26. A hybrid quantum-classical apparatus for simulation of a material, chemical, or biological system, by way of enacting the method of any one of clauses 1 to 25.

Clause 27. A hybrid quantum-classical apparatus according to clause 26 comprising:

a noisy intermediate-scale quantum, NISQ, processor; and
at least one classical processor,

wherein the NISQ processor is used to calculate the ground state energy of the second Hamiltonian provided in step (2).

Clause 28. A hybrid quantum-classical apparatus according to clause 27, wherein the at least one classical processor is used to perform the quantum Kohn-Sham DFT iterations in step (iv).

Clause 29. A hybrid quantum-classical apparatus according to clause 27 or clause 28, as dependent on clause 18 wherein the at least one classical processor is used to perform the classical Kohn-Sham DFT iteration.

Clause 30. A hybrid quantum-classical system according to any one of clauses 27 to 29, further wherein the at least one classical processor performs steps comprising at least one of:

a fermion-to-qubit mapping of the second Hamiltonian into a parameterised quantum circuit;
updating VQE parameters based on the variational principle;
obtaining a continuous representation of the exchange correlation energy functional or the exchange correlation potential functional;
storing an exchange correlation potential functional locally or transmitting it to a remote location; or
storing an exchange correlation energy functional locally or transmitting it to a remote location.

Clause 31. A hybrid quantum-classical apparatus according to any one of clauses 26 to 30, further wherein at least one classical processor is part of a control apparatus for the NISQ processor.

Clause 32. A non-transient computer readable medium comprising instructions which cause a computer to enact the method steps of any one of clauses 1 to 31.

APPENDIX 1

**The Hubbard dimer**

Quantum Hybrid Density Functional Theory

- Density Functional Theory (DFT) is a very successful method for studying weakly correlated electronic systems.
- It encounters numerous pitfalls for strongly correlated electronic systems, where it can often qualitatively fail in its predictions.

Lattice Density Functional Theory (LDFT)

The inhomogeneous Hubbard model is given by:

$$H = -t \sum_{\langle ij \rangle \sigma} \left( c_{i,\sigma}^{\dagger} c_{j,\sigma} + c_{j,\sigma}^{\dagger} c_{i,\sigma} \right) + \sum_{i}^{L} U n_{i,\uparrow} n_{i,\downarrow} + \sum_{i=1}^{L} v_i \hat{n}_i \,,$$

(66)

DFT maps this Hamiltonian into a single particle one with an effective potential:

$$H_{KS}(n_i) = -t \sum_{\langle ij \rangle}^{L} \left( c_i^{\dagger} c_j + c_j^{\dagger} c_i \right) + \sum_i V_i^{\text{eff}}(n_i) c_i^{\dagger} c_i \,,$$

(67)

where:

$$V_i^{\text{eff}}(n_i) = V_{\text{ext}}(i) + \frac{u}{2} n_i + \frac{dF(\mathbf{n})}{dn_i}$$

(68)

where $\dfrac{dF(\mathbf{n})}{dn_i}$ is the unknown exchange correlation potential. A current goal of this work is to approximate $\dfrac{dF(\mathbf{n})}{dn_i}$ with VQE.

The algorithm for LDFT is:

$$\eta^0 = \{n_0, n_1, \dots, n_L\}$$
$$\Downarrow$$
$$\text{Diagonalise } H_{KS}(1) | \psi_{\text{GS}} \rangle = \epsilon_{GS} | \psi_{GS} \rangle$$
$$\Downarrow$$
$$\mathbf{n}^i = \alpha \mathbf{n}^{i-1} + (1 - \alpha) \mathbf{n}_{GS}^i$$
$$\Downarrow$$
$$\text{No} \Leftarrow \left| \epsilon_{GS}^i - \epsilon_{GS}^{i-1} \right| < \epsilon$$
$$\Downarrow \text{Yes}$$
$$\text{Converged}$$

(69)

The Hubbard Dimer in LDFT

$$H_{dimer} = H_{hubb} + \sum_{i=1}^{2} v_i \hat{n}_i$$

(70)

where:

$$H_{hubb} = -t \sum_{i\sigma}^{1} \left( c_{i,\sigma}^{\dagger} c_{i+1,\sigma} + c_{i+1,\sigma}^{\dagger} c_{i,\sigma} \right) + \sum_{i=1}^{2} U n_{i,\uparrow} n_{i,\downarrow}$$

$$(71)$$

Now, we define the following variables and set N = 2, so that: $\Delta v = v_1 - v_2$, $v_1 + v_2 = 0$, $n_1 + n_2 = 2$ and $\Delta n = n_1 - n_2$. In doing so we can show that:

$$\langle H_{dimer} \rangle = \langle H_{hubb} \rangle + 2v_2(1 - \langle \hat{n}_1 \rangle)$$
$$= \langle H_{hubb} \rangle + \Delta v \left( 1 - (1 - \Delta n/2) \right)$$
$$= \langle H_{hubb} \rangle + \Delta v \Delta n/2$$

$$(72)$$

We can rearrange this formulae to obtain the expression for the Hubbard energy, or the energy solely associated with exchange and correlation of the problem, i.e.:

$$\langle H_{hubb} \rangle = \langle H_{dimer} \rangle - \Delta v \Delta n/2$$

$$(73)$$

These expressions enable us to formulate Kohn Sham theory. By restricting our study to the $N = 2$ and $S_z = 0$ sector of the Hubbard dimer, which has the following matrix representation:

$$\begin{pmatrix} \Delta v + U & -t & -t & 0 \\ -t & 0 & 0 & -t \\ -t & 0 & 0 & -t \\ 0 & -t & -t & -\Delta v + U \end{pmatrix}$$

$$(74)$$

where we have used the following basis states $\{|\uparrow\downarrow, 0\rangle, |\uparrow, \downarrow\rangle, |\downarrow, \uparrow\rangle, |0, \uparrow\downarrow\rangle\}$.

**Formulation of certain subroutines**

formulated starting from the Hohenberg-Kohn variational principle which states that the total energy of the ground state is given by:

$$E[V_{\text{ext}}(\mathbf{r})] = \min_{n(\mathbf{r})} \left[ F[(\mathbf{r})] + \int V_{\text{ext}}(\mathbf{r}) n(\mathbf{r}) \right]$$

$$(75)$$

We obtain the formulation of a first algorithm, qDFTmin, by isolating $F[(\mathbf{r})]$ and solving it for the Hubbard dimer as a function of $V_{\text{ext}}(\mathbf{r})$. Therefore, we show that using a quantum computer and given an external potential it is possible to find the corresponding universal functional energy, from which the XC potential can be found.

On the other hand, we obtain a second algorithm, qDFTmax, from Lieb's formulation of DFT, which states that the universal functional can be obtained from maximising over the external potential for a given density, i.e.,

$$F[n(\mathbf{r})] = \max_{V_{\text{ext}}(\mathbf{r})} \left[ E[V_{\text{ext}}(\mathbf{r})] - \int V_{\text{ext}}(\mathbf{r}) n(\mathbf{r}) \right].$$

$$(76)$$

APPENDIX 2

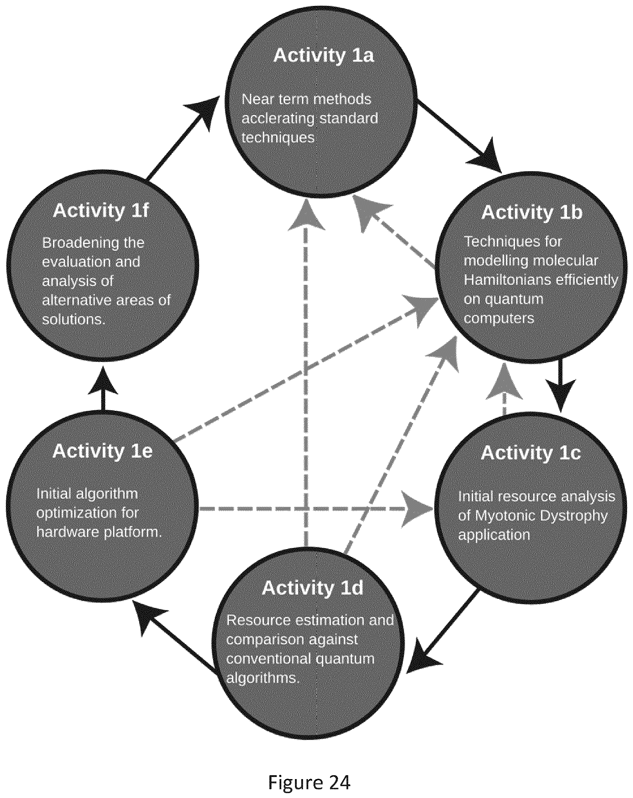

Recent work by the inventors discussed publicly here for the first time has shown that high fidelity XC potentials can be obtained from low depth, low quality VQE computations on quantum processors for the prototypical Fermi-Hubbard lattice model. The XC potentials can then be used inside a classical DFT loop executed on target systems which need not be the input system. Our results indicate that this approach can be an effective way of constructing XC potentials that include the effects of exchange and correlation, overcoming salient issues with classical functional designs, while simultaneously taking advantage of low depth and compact quantum circuits which can be run on NISQ devices. Here we build on these results by extending this formalism to fermionic Hamiltonians that describe the molecular systems relevant to this proposal. To do this, the following procedure is followed (with reference to Fig. 24):

- Identify the minimal representations of fermionic Hamiltonians for myotonic dystrophy applications via classical simulation (optimizing the representation is related to Activity 1b). We note that the input systems need not be the eventual target system, but are used as proxies to generate high quality approximations to the XC energy and potential that are used to simulate the ground state properties of the target system (related to Activity 1c).

- Construct quantum circuits that measure the XC energy from which the XC potentials are generated and permanently stored.

- Extend the implementation of the algorithm presented in Figure 1 to deal with molecular Hamiltonians. This includes providing an interface to classical ab initio software, which builds upon existing software suites developed internally for materials simulation.

Activity 1b: Techniques for modelling molecular Hamiltonians efficiently on quantum computers

It is imperative to map the classical representation of the molecular Hamiltonians into quantum circuits which can fit on the resources afforded by near term quantum devices. We use recent advancements in compact representations via embedding techniques, such as Density Matrix Embedding Theory (DMET), for molecular systems to reduce the number of qubits required in the quantum simulation. In these approaches, the classical molecular Hamiltonian is partitioned into two regions: one which is classically efficient and the other which uses direct quantum treatment. To reduce the gate depth, allowing for more accurate quantum computations of the XC energy, the connectivity of the parent molecular Hamiltonians can be optimized within the DMET approach by considering novel fragment to both mappings based on the hardware layout, a constraint which is not considered in purely classical approaches. Moreover, efficient fermionic encodings that exploit molecular locality and measurement strategies are needed to further reduce this depth. All of the above considerations have been extensively investigated by the inventors and discussed publicly here for the first time in recent works and we incorporate these optimisations into the overall framework as shown in Figure 1 and implement the extensions of the DMET code needed to model molecular Hamiltonians, specifically tailoring their features to myotonic dystrophy inhibitor molecules.

Activity 1c: Initial resource analysis of Myotonic Dystrophy application.

In this work package we determine the quantum computing resources required to implement the algorithms developed in activities 1a / 1b. Important resources to consider here are the number of qubits used, the number of 2-qubit quantum gates, and the quantum circuit depth. Recent work by the inventors discussed publicly here for the first time has developed a comprehensive resource estimation toolkit in the context of materials modelling, which enables the user to input a material's atomic structure, and outputs the quantum complexity of modelling that material via VQE or time-dynamics simulation. Here we perform a similar analysis for "standard" quantum algorithmic approaches in the context of myotonic dystrophy. In particular, we explore the fundamental physical representations of the fermionic systems involved in the myotonic dystrophy process, e.g. the importance of the chosen active space and how that relates to the computation of ground state properties. This activity requires the development of novel software tools as well as underpinning theory and numerical work. We additionally compare against the resources used by classical methods for addressing the relevant systems for myotonic dystrophy.

Activity 1d: Resource estimation and comparison against conventional quantum algorithms.

This activity follows on from 1c by estimating resources used by the novel quantum algorithms developed in this project. Resource estimations for the quantum algorithm presented in Figure 1 are made against conventional quantum algorithms for predicting ground state properties of the target system, such as direct application of the VQE. Our metric of choice for these estimates is the circuit depth and number qubits required to predict the ground state properties of the different target systems under consideration, before and after the optimisations from the above activities are considered.
We show how a collection of connected techniques, from local molecular embeddings, compact fermion encodings, native connectivity layouts of the parent Hamiltonians, to efficient measurement strategies, can impact the requirements needed to perform a NISQ computation for the application of myotonic dystrophy. Understanding the resource requirements for our novel approaches again involves a combination of theory, numerical work, and software development.

Activity 1e: Initial algorithm optimization for hardware platform.

We perform an initial study of how the algorithm in Figure 1 can be implemented and optimised. This platform has a number of features which make it well-suited to the proposed application domain, such as the ability to rearrange qubits and hence achieve effective all-to-all connectivity. In this activity, we determine the most efficient approach to represent and implement our algorithms to take advantage of these features. For example, this includes decomposing the operations that need to be performed in VQE in terms of hardware-native operations. As part of this, we undertake a finer-grained resource analysis that uses an accurate representation of the hardware's properties to determine our algorithms' true complexity and the level of performance that can be expected in later phases.

Activity 1f: Broadening the evaluation and analysis of alternative areas of solutions.

The algorithm presented in Figure 1 can also be applied agnostically to other target molecular systems; this is especially true regarding the generated XC functionals and the underlying relationship between the parent molecular Hamiltonians and their encodings with hardware layouts. We explore the possibility of recycling these fully quantum XC potentials for other relevant human healthcare applications, which are potentially even higher-value than myotonic dystrophy. We also explore how quantum XC potentials can be incorporated into other quantum algorithms, such as time dynamical simulations that can probe excited state molecular properties.

Activity 3: Development of techniques for QM/MM integration

The majority of QM/MM simulations use DFT for the QM part of the computation, primarily due to their computational cost and approximate accuracy. However, there are many scenarios where the accuracy is subject to uncontrolled and large errors that originate from the underlying exchange correlation functional approximation. While it has recently become possible to incorporate highly accurate fully correlated ab initio wavefunction based methods in lieu of DFT for this part of the computation, these are prohibitively expensive as classical algorithms and are thus only applicable to very small system sizes. It is therefore desirable to develop an approach which retains the accuracy of ab initio wavefunction based methods but scales like DFT, and one way in which this can be realised is through more accurate exchange correlation potential approximations.

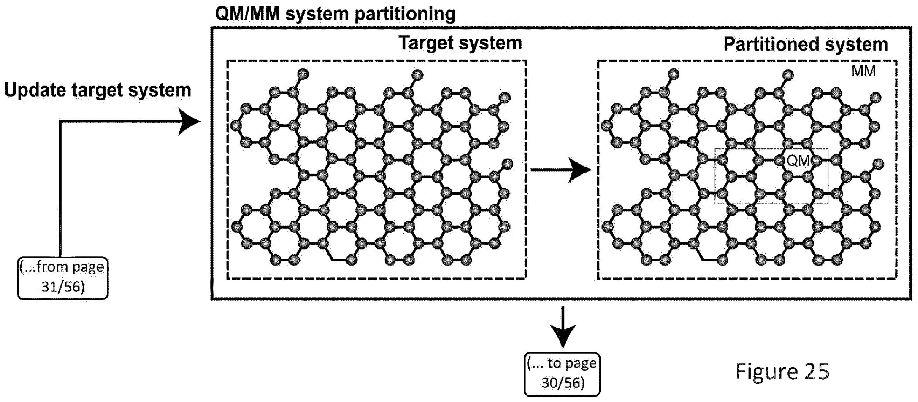

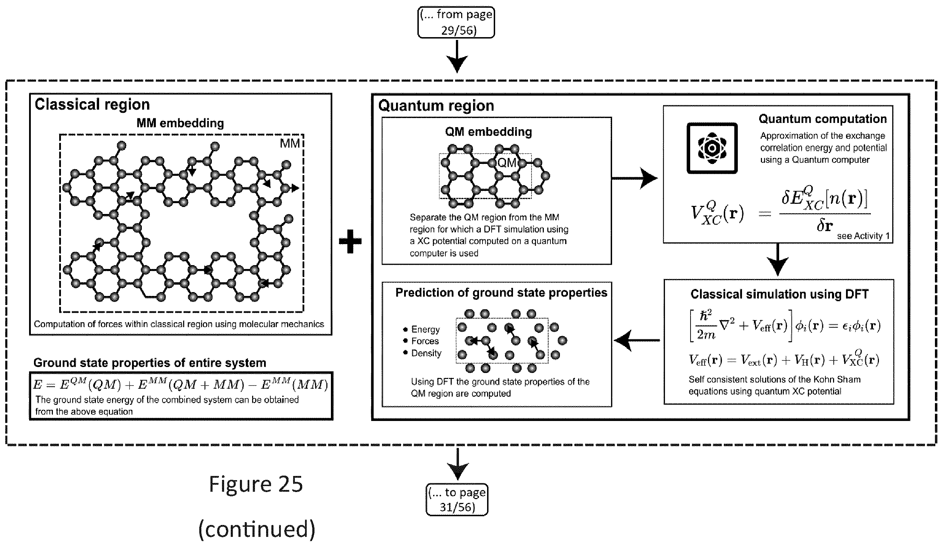

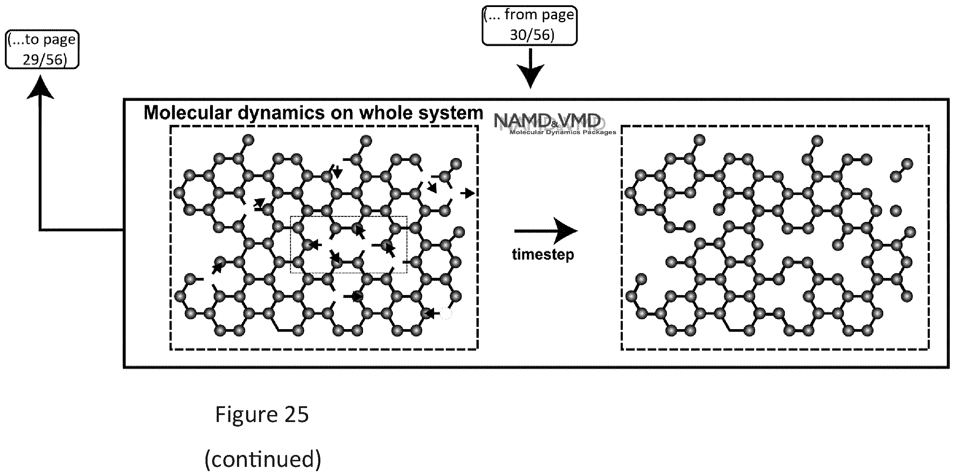

Therefore, we use the quantum algorithms developed in Activity 1 and show how they can be incorporated into the classical QM/MM framework described above, with a focus on leveraging specific features of near term quantum devices. Firstly, we explore in detail how the choice and size of the classical and quantum regions within the QM and MM regions of the simulation affect the quantum resource estimates of a black-box protocol for quantum enhanced single point hybrid QM/MM simulations. Moreover, we develop novel techniques that exploit the inherent advantages of using a quantum computer as a quantum solver. Ultimately, we use these advancements to assess the utility of these techniques for predicting a full reaction profile that incorporates dynamic correlation in a single point QM/MM calculation. The overall framework is illustrated in Figure 25, charting the algorithmic workflow of how the data is passed back and forth between the hybrid quantum classical algorithm and highlighting where the intervention of the quantum computer occurs.

From this starting point, the work is well positioned to develop the theoretical evaluation of the quantum algorithm resource estimations, the novel techniques needed to exploit the quantum solvers for the QM/MM calculation, as well as establishing the computational interface between the classical and quantum computer, having developed a sizeable suite of proprietary software tools for classical-quantum algorithms in-house.

Activity 3a: Theoretical evaluation and resource estimation of "black box" use of quantum solver within QM/MM.

The black box use of a quantum solver within QM/MM can be viewed as taking an input molecular structure, as illustrated in Figure 25, and performing molecular dynamics simulations that incorporate the dynamical correlation effects until the system is equilibrated. When considering the factors which affect the evaluation of this black box we focus on (1) the choice of the classical/quantum partition, i.e. the considered active space, and (2) the manner in which the system is combined after the separate MM (classical) and QM (quantum) simulations are complete. We achieve this by:

- Theoretically develop the lower and upper bounds on the combined classical and quantum resource requirements (number of required qubits, program depth, classical program iterations) needed to efficiently represent the QM region inside the QM/MM framework as a quantum circuit using the approach shown in Figure 25.

- The recombination of the QM and MM regions can be achieved by incorporating the QM and MM interactive effects at different levels of theory. In Figure 25 we highlight the naive "subtractive" approach but we also explore the effect of the more advanced techniques, such as "additive" corrections have on the final quantum resources.

- Perform a scaling study of how the number of active sites in the QM region affects the quantum resources using the QM/MM method with DFT functionals as opposed to the direct application of VQE on the QM active space for the computation of reaction profiles.

- Incorporation of classical simulation codes (GROMACS) with proprietary software packages for quantum resource estimation, such as those discussed in our previous work on quantum resource estimation for materials simulation

Activity 3b: Novel techniques for QM/MM integration using the features of quantum solvers.

As described in Activity 1, we use hybrid quantum-classical variational algorithms to construct approximations to the DFT XC functional. In particular, the performance of these solvers are sensitive to a number of factors, notably (1) the representation of the Hamiltonian for the QM region, (2) the fermionic encoding chosen to map the physical modes into qubits, and (3) the measurement strategy used to obtain ground state properties of the molecular system.

We develop novel techniques that consider each of these factors that aim to maximise the quantum resources available on near term devices. To achieve this, we:

- Assess the effect of projector embedded methods, and further incorporate methods such as DMET, for the secondary truncation of the quantum active space within the QM region on quantum resource estimates. In this case, a further partitioning of the quantum subspace can be made at the QM level, such that the DFT computation is partitioned into "easy" and "difficult" computable regions, and the quantum computer only treats the "difficult" region, while the classical computer treats the "easy" region. Heretofore unpublished work by the inventors has indicated that these embedding schemes can significantly reduce the quantum resources for strongly correlated multi-orbital Hubbard hamiltonians and we extend these techniques to the realistic example of Myotonic Dystrophy applications.

- We explore the effect that different fermionic encoding schemes have on the description of the QM region, such as comparing the Jordan Wigner encoding to compact encoding schemes discussed by the inventors (see e.g. European patent application EP 23175487.0).

- We create optimal measurement strategies that parallelise quantum computations in two ways. For example, the inventor's work on materials simulation highlights the importance of tailoring the measurement strategy for observables of choice, where parallelisation across qubits can reduce the overall quantum runtime. Additionally, we assess the potential of using parallel-VQE, a recent parallel quantum solver framework proposed by the inventors, in improving the quality of results via executing parallel computations across the same chip, at the same time.

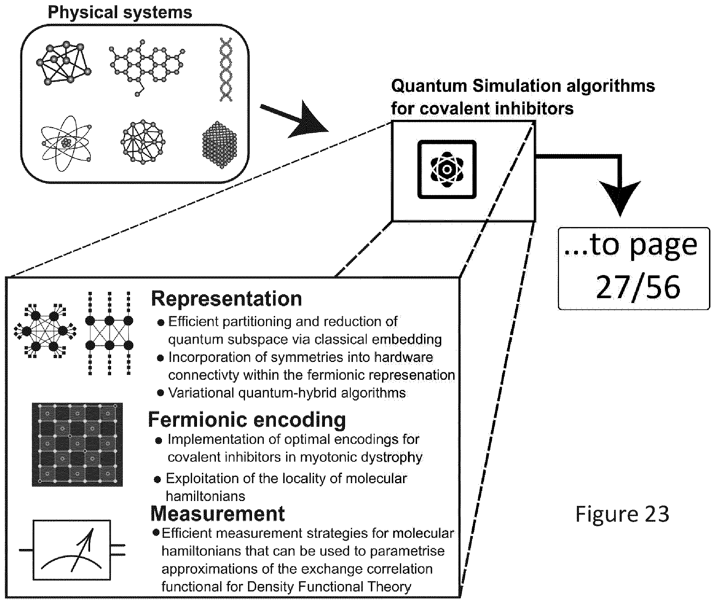

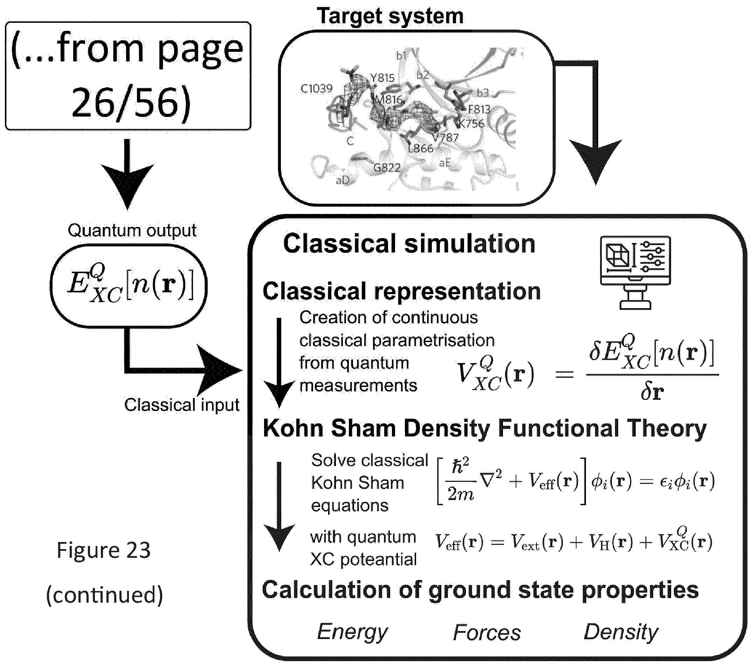

Figure 23 is a schematic of a quantum-classical hybrid algorithm to accelerate and improve over standard classical methods for the exemplary application of methods disclosed herein to the simulation of covalent inhibitors in Myotonic Dystrophy applications. Physical systems inform quantum simulation algorithms for covalent inhibitors which include three main steps: representation, fermionic encoding, and measurement. These are made up of additional tasks as shown below and in Figure 23.

Representation

- Efficient partitioning and reduction of quantum subspace via classical embedding
- Incorporation of symmetries into hardware connectivity within the fermionic representation
- Variational quantum-hybrid algorithms including the one outlined herein and in **Table 2.**

Fermionic encoding

- Implementation of optimal encodings for covalent Inhibitors in myotonic dystrophy
- Exploration of the locality of molecular Hamiltonians

Measurement

- Efficient measurement strategies for molecular Hamiltonians that can be used to parameterise approximations of the exchange correlation functional tor Density Functional Theory

The quantum calculated XC (exchange correlation functional) can then be used to direct classical Kohn-Sham DFT for a full target system (that is, for example, covalent inhibitors in Myotonic Dystrophy). This relates to the DFT loop of QEDFT as shown in **Table 1,** and comprises: classical representation; KS DFT; and calculation of ground state properties. Classical representation is the step of creation of continuous classical parameterisation from quantum measurements. KS DFT comprises solving the KS equations self consistently using the quantum XC potential as detailed previously in this document. Some examples of ground state properties that may be useful to calculate include energies, forces, and densities associated with the target system.

As discussed in activity 3a above, Figure 25 illustrates the 'black box' use of a quantum solver within QM/MM can be viewed as taking an input molecular structure and performing molecular dynamics simulations that incorporate the dynamical correlation effects until the system is equilibrated, as an example algorithmic workflow of quantum computation within a quantum/materials/molecular modelling calculation. This involves a loop with three main steps:

1. QM/MM system partitioning →2. A 'black-box' Quantum Solver →3. Molecular Dynamics on the whole system (then 3. updates the target system in 1.).

Step 1. involves partitioning the target system into two regions, MM and QM.

In step 2 the black-box quantum solver gives the MM embedding to a classical region which computes the forces in the classical, or MM, region using molecular mechanics. Likewise, the quantum region, or QM embedding, is passed to a hybrid quantum-classical algorithm of the type disclosed herein, i.e. QEDFT. A DFT simulation employing an XC potential computed on a quantum computer is used, in accordance with the methods described herein, and shown in Figure 23, in line with Table 1 and Table 2. That is, the QM region is separated from the MM region. The QM region is the whole system to be solved (analogous to the inhomogeneous Fermi-Hubbard Hamiltonian example). A quantum computer is used to approximate the exchange correlation energy and potential according to equation (19). The quantum XC potential may be passed to a classical simulation using DFT which solves the KS equations self consistently according to equations (10), (11), and (12) to obtain a ground state energy. After convergence, ground state properties, such as energies, forces, and densities, of the QM region may be computed.

The ground state properties of the combined QM/MM system may be obtained by combining the results of the classical region (MM) and the quantum region (QM) using:

$$E = E^{QM}(QM) + E^{MM}(QM + MM) - E^{MM}(MM).$$

$$(77)$$

Step 3. may then perform molecular dynamics on the whole system, possible with a time step or other method of time evolution. The resulting system may then be fed back into step 1 as the target system and steps 1-3 may repeat until the system is equilibrated (e.g. step 3 does not produce a change in the system) or another suitable condition is satisfied.

**Claims**

**1.**    A method of performing improved Kohn-Sham Density Functional Theory, DFT, calculations, for simulation of a material, chemical, or biological system, comprising the steps of:

(i) constructing a model Hamiltonian of the system to be simulated;
(ii) inputting an electron density of the system to be simulated;
(iii) constructing a Kohn-Sham potential by:

(1) providing a second Hamiltonian based on the model Hamiltonian;
(2) calculating a ground state energy of the second Hamiltonian, as a function of electron density, using a quantum algorithm executed by a noisy intermediate-scale quantum, NISQ, processor;
(3) computing a quantum-obtained exchange correlation energy functional, as a function of electron density, from the NISQ processor-obtained ground state energy of step (2);
(4) obtaining a quantum-obtained exchange correlation potential functional by differentiation of the quantum-obtained exchange correlation energy functional from step (3);
(5) using the quantum-obtained exchange correlation potential functional from (4) to construct the Kohn-Sham potential; and

(iv) executing a quantum Kohn-Sham DFT algorithm iteration using the electron density from (ii) and the Kohn-Sham potential from (iii) to compute an updated electron density and an updated total energy of the system to be simulated.

2. A method according to claim 1, wherein providing the model Hamiltonian in step (i) includes either:

providing an inhomogeneous Hamiltonian; or
providing a homogeneous Hamiltonian, wherein the homogeneous Hamiltonian is the inhomogeneous Hamiltonian with an external potential removed.

3. A method according to claim 1 or claim 2, wherein when the model Hamiltonian provided in step (i) is inhomogeneous then the second Hamiltonian provided in step (1) is homogeneous, further wherein the model Hamiltonian has an external potential and the second Hamiltonian is based on the model Hamiltonian with the external potential removed.

4. A method according to any one of the preceding claims, wherein:

calculating the ground state of the second Hamiltonian on an NISQ processor further comprises using a fermion-to-qubit encoding to encode qubits of the NISQ processor, optionally wherein the fermion-to-qubit encoding is a Jordan Wigner encoding that maps fermionic creation and annihilation operators of the second Hamiltonian to Pauli strings comprising Pauli X, Y and Z operators; and/or
calculating the ground state of the second Hamiltonian on an NISQ processor further comprises enacting qubit operations generated by unitary qubit operators on the qubits of the quantum information processor; and/or
the model Hamiltonian is the Hubbard model with an external potential term, and the second Hamiltonian is the Hubbard model.

5. A method according to any one of the preceding claims, wherein execution of the quantum algorithm by a noisy intermediate-scale quantum, NISQ, processor further comprises executing a Variational Quantum Eigensolver, VQE, algorithm using the NISQ processor, optionally wherein:

the VQE algorithm is implemented as a VQE circuit which is lower depth than VQE circuits required to find the ground state of the model Hamiltonian directly; and/or
the VQE algorithm uses a Hamiltonian Variational Ansatz, HVA.

6. A method according to any one of the preceding claims, wherein obtaining a quantum-obtained exchange correlation potential functional in step (4) further comprises using a quantum kernel method for continuous representation of the exchange correlation energy functional as a function of electron density, optionally wherein the quantum kernel method comprises interpolative and/or extrapolative procedures for obtaining the exchange correlation energy functional as a function of electron density, optionally wherein the quantum kernel method further comprises using a Local Density Approximation, LDA, to construct a continuous representation of the exchange correlation energy functional.

7. A method according to any one of the preceding claims, wherein:

the material system to be simulated is a strongly correlated electron system; and/or
the Kohn-Sham DFT algorithm is a Kohn-Sham Lattice Density Functional Theory, LDFT, algorithm; and/or
the NISQ processor requires fewer qubits to calculate the ground state of the second Hamiltonian than the number of qubits required to calculate the ground state of the model Hamiltonian of the system to be simulated directly.

8. A method according to any one of the preceding claims, wherein executing a quantum Kohn-Sham DFT algorithm iteration in step (iv) comprises the steps of:

(a) taking the electron density from (ii) and the Kohn-Sham potential from (iii) as an input to construct Kohn-Sham equations;
(b) solving the Kohn-Sham equations;
(c) providing the updated electron density, and the updated total energy of the system to be simulated, based on solutions of the Kohn-Sham equations in step (b); and

(d) checking if a convergence condition is met, further wherein the condition for convergence is evaluated based on the electron density input in step (ii) and the updated electron density.

9. A method according to claim 8, wherein evaluating the convergence condition in step (d) comprises calculating:

(a) a density difference metric from the electron density input in step (ii) and the updated electron density; and/or
($\beta$) an energy difference metric from a total energy of the system to be simulated, based on the electron density from step (ii), and the updated total energy of the system to be simulated,

and comparing the density difference metric from (a) and/or the density difference metric from ($\beta$) with a threshold condition for each difference metric, and optionally wherein the updated electron density, and the updated total energy of the system to be simulated, from step (c) are used to construct ground state properties of the model Hamiltonian describing the material system which is to be simulated.

10. A method according to claim 9, wherein further quantum Kohn-Sham DFT algorithm iterations, are performed by providing the updated electron density from (c) as the input in step (ii) if the convergence condition in (d) is not met such that steps (ii) to (iv) continue until the convergence condition for the electron density, and/or the convergence condition for the total energy of the system to be simulated, is met in step (d).

11. A method according to claim 9, wherein a further Kohn-Sham DFT iteration is performed in a classical manner, the classical Kohn-Sham DFT iteration comprising the steps (ii) to (iv), wherein step (ii) receives the updated electron density from (c) as an input and step (iii) replaces steps (1) to (5) by providing a classically obtained exchange correlation potential functional as an input to construct the Kohn-Sham potential.

12. A method according to claim 11, wherein further classical Kohn-Sham DFT iterations are performed until the convergence condition for the electron density, and/or the convergence condition for the total energy of the system to be simulated, is met in step (d).

13. A method according to claim 11, wherein further quantum or classical Kohn-Sham DFT algorithm iterations are performed, including at least one further quantum Kohn-Sham DFT iteration, until the convergence condition for the electron density, and/or the convergence condition for the total energy of the system to be simulated, is met in step (d).

14. A hybrid quantum-classical apparatus for simulation of a material, chemical, or biological system, by way of enacting the method of any one of claims 1 to 13, optionally wherein the hybrid quantum-classical apparatus comprises:

a noisy intermediate-scale quantum, NISQ, processor; and
at least one classical processor, optionally wherein at least one classical processor is part of a control apparatus for the NISQ processor,

wherein the NISQ processor is used to calculate the ground state energy of the second Hamiltonian provided in step (2), and/or further wherein the at least one classical processor performs steps comprising at least one of:

a fermion-to-qubit mapping of the second Hamiltonian into a parameterised quantum circuit;
updating VQE parameters based on the variational principle;
obtaining a continuous representation of the exchange correlation energy functional or the exchange correlation potential functional;
storing an exchange correlation potential functional locally or transmitting it to a remote location; or
storing an exchange correlation energy functional locally or transmitting it to a remote location,

optionally further wherein the at least one classical processor is used to perform the quantum Kohn-Sham DFT iterations in step (iv), and optionally wherein, when dependent on claim 9, the at least one classical processor is used to perform the classical Kohn-Sham DFT iteration.

15. A non-transient computer readable medium comprising instructions which cause a computer to enact the method steps of any one of claims 1 to 14.

$$H = -t(c_{1\uparrow}^{\dagger}c_{2\uparrow} + c_{2\uparrow}^{\dagger}c_{1\uparrow} + c_{1\downarrow}^{\dagger}c_{2\downarrow} + c_{2\downarrow}^{\dagger}c_{1\downarrow}) + U(n_{1\uparrow}n_{1\downarrow} + n_{2\uparrow}n_{2\downarrow})$$

**Setup the homogeneous fermionic Hubbard dimer**

$$H = -t(X \otimes \mathbf{1} + \mathbf{1} \otimes X) + \frac{U}{2}(\mathbf{1} + Z \otimes Z)$$

**Encode the fermionic Hamiltonian in qubits via a fermion to qubit mapping**

```
from qiskit import QuantumCircuit
import numpy as np

# instantiate qiskit quantum circuit object
circ = QuantumCircuit(2)

# initial ansatz for the VQE parameters
params = [1, 1, 1]

# Initial state prep
circ.ry(-np.pi/2, 0)
circ.ry(-np.pi/2, 1)

# Onsite gate
circ.cx(0, 1)
circ.rz(params[0], 1)
circ.cx(0, 1)

# chemical potential gate
circ.rz(params[1], 0)
circ.rz(params[1], 1)

# Hopping gates
circ.rx(params[2], 0)
circ.rx(params[2], 1)
\label{code:vqe_code}
```

**Create a quantum circuit**

**Obtain ground state energy at different electronic fillings {0, 1, 2, 3, 4} using a quantum computer**

$$\text{Quantum compute: } E(n_i, U) \forall n_i \in \{0, 1, 2, 3, 4\}$$

**Compute the local exchange correlation energy at different electronic fillings {0, 1, 2, 3, 4}**

$$E_{XC}^{QLDA}(n_i, U) = \frac{1}{L}E(n_i, U) - \frac{1}{L}\sum_{i}^{L}\frac{U}{4}\langle n_i^2 \rangle\frac{1}{L} - \sum_{ij\sigma}^{L}t_{ij\sigma}\langle c_{i\sigma}^{\dagger}c_{j\sigma} + c_{j\sigma}^{\dagger}c_{i\sigma}\rangle$$

**Construct the exchange correlation potential by numerical differentiation**

$$V_{XC}^{QLDA}(n_i, U) = \frac{\partial E_{XC}^{QLDA}(n, U)}{\partial n}\Big|_{n=n_i}$$

Figure 1A

Figure 1B

Figure 1B

Set external potential for the
inhomogenous Hubbard dimer
and fill with 2 electrons

$$H = -t(c_{1\uparrow}^{\dagger}c_{2\uparrow} + c_{2\uparrow}^{\dagger}c_{1\uparrow} + c_{1\downarrow}^{\dagger}c_{2\downarrow} + c_{2\downarrow}^{\dagger}c_{1\downarrow})$$
$$+ U(n_{1\uparrow}n_{1\downarrow} + n_{2\uparrow}n_{2\downarrow})$$
$$+ v_1(n_{1\uparrow} + n_{1\downarrow}) + v_2(n_{2\uparrow} + n_{2\downarrow})$$

Initial guess of the density

$$n_{\text{init}} \propto V_{\text{ext}} = (v_1, v_2)$$

Construct Kohn Sham potential

$$V_i^{\text{eff}}(n_i) = V_{\text{ext}}(i) + \frac{U}{2}n_i + V_{\text{XC}}(n_i)$$

Solve the Kohn Sham equations

$$H_{\text{KS}}|\phi_j\rangle = \epsilon_j|\phi_j\rangle$$

$$H_{\text{KS}} = -t(c_{1\uparrow}^{\dagger}c_{2\uparrow} + c_{2\uparrow}^{\dagger}c_{1\uparrow} + c_{1\downarrow}^{\dagger}c_{2\downarrow} + c_{2\downarrow}^{\dagger}c_{1\downarrow})$$
$$+ V_1^{\text{eff}}(n_1)(c_{1\uparrow}^{\dagger}c_{1\uparrow} + c_{1\downarrow}^{\dagger}c_{1\downarrow})$$
$$+ V_2^{\text{eff}}(n_2)(c_{2\uparrow}^{\dagger}c_{2\uparrow} + c_{2\downarrow}^{\dagger}c_{2\downarrow})$$

Construct the new density

$$n_1 = |\phi_1\rangle_1^2 + |\phi_2\rangle_1^2$$
$$n_2 = |\phi_1\rangle_2^2 + |\phi_2\rangle_2^2$$

No

Computation converged?

$$E_{GS}(\mathbf{n}^{GS}, U) = \epsilon_1^{KS} + \epsilon_2^{KS}$$
$$- V^{XC}(n_1, U)n_1 - V^{XC}(n_2, U)n_2$$
$$- \frac{1}{4}U(n_1^2 + n_2^2)$$
$$+ E_{XC}^{QLDA-TOT}[\mathbf{n}^{GS}, U]$$

Yes

Construct ground state energy

Figure 2A

Figure 2B

Figure 3

Figure 4

VXC for 1d chain of 1 sites

Figure 5

Figure 6

Figure 7

Coarse description of the quantum system at hand

ion

electron density

Computationally easier

Exact description of the quantum system at hand

ion

e⁻ e⁻ e⁻ e⁻ e⁻

Computationally hard

Figure 8

q_0: ─── Ry(-π/2) ──■── ─────────── ──■── Rz(1.0347) ── Rx(π/2) ───

q_1: ─── Ry(-π/2) ── X ── Rz(π) ── X ── Rz(1.0347) ── Rx(π/2) ───

Figure 9

Figure 10

EP 4 478 263 A1

Figure 11

Figure 12

Figure 13

Figure 14

Figure 15

Figure 16

Figure 17

Figure 18

Figure 19

EP 4 478 263 A1

Figure 20

Figure 21

Figure 22

**Physical systems**

**Quantum Simulation algorithms for covalent inhibitors**

**Representation**
- Efficient partitioning and reduction of quantum subspace via classical embedding
- Incorporation of symmetries into hardware connectivty within the fermionic represenation
- Variational quantum-hybrid algorithms

**Fermionic encoding**
- Implementation of optimal encodings for covalent inhibitors in myotonic dystrophy
- Exploitation of the locality of molecular hamiltonians

**Measurement**
- Efficient measurement strategies for molecular hamiltonians that can be used to parametrise approximations of the exchange correlation functional for Density Functional Theory

Figure 23

Figure 23 (continued)

Figure 24

Figure 25

**Classical region**

MM embedding

MM

Computation of forces within classical region using molecular mechanics

**Ground state properties of entire system**

$$E = E^{QM}(QM) + E^{MM}(QM + MM) - E^{MM}(MM)$$

The ground state energy of the combined system can be obtained from the above equation

**+**

**Quantum region**

QM embedding

QM

Separate the QM region from the MM region for which a DFT simulation using a XC potential computed on a quantum computer is used

**Prediction of ground state properties**

- Energy
- Forces
- Density

Using DFT the ground state properties of the QM region are computed

**Quantum computation**

Approximation of the exchange correlation energy and potential using a Quantum computer

$$V_{XC}^Q(\mathbf{r}) = \frac{\delta E_{XC}^Q[n(\mathbf{r})]}{\delta \mathbf{r}}$$

see Activity 1

**Classical simulation using DFT**

$$\left[\frac{\hbar^2}{2m}\nabla^2 + V_{\text{eff}}(\mathbf{r})\right]\phi_i(\mathbf{r}) = \epsilon_i\phi_i(\mathbf{r})$$

$$V_{\text{eff}}(\mathbf{r}) = V_{\text{ext}}(\mathbf{r}) + V_{\text{H}}(\mathbf{r}) + V_{\text{XC}}^Q(\mathbf{r})$$

Self consistent solutions of the Kohn Sham equations using quantum XC potential

Figure 25

(continued)

Figure 25

(continued)

EP 4 478 263 A1

EP 4 478 263 A1

Figure 26

Figure 27

Figure 28

Figure 29

Figure 30

EP 4 478 263 A1

Figure 31A

Figure 31B

## External potential = none

Figure 32A

Figure 32B

Figure 32C

Figure 33

Figure 34

Figure 35A

Figure 35B

EP 4 478 263 A1

Figure 36

Figure 37

Figure 38

Figure 39

Figure 40

Figure 41

EP 4 478 263 A1

Figure 42

Figure 43A

## External potential = quadratic

Figure 43B

## External potential = impurity

Figure 43C

Figure 44

Figure 45

Figure 46

Europäisches
Patentamt

European
Patent Office

Office européen
des brevets

# EUROPEAN SEARCH REPORT

**Application Number**

EP 24 15 5882

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRUNO SENJEAN ET AL: "A quantum advantage for Density Functional Theory ?", ARXIV.ORG, CORNELL UNIVERSITY LIBRARY, 201 OLIN LIBRARY CORNELL UNIVERSITY ITHACA, NY 14853, 4 April 2022 (2022-04-04), XP091211831, * page 1 - page 4 * | 1-15 | INV. G06N10/60 |
| A | NOUHAILA INNAN ET AL: "Electronic Structure Calculations using Quantum Computing", ARXIV.ORG, CORNELL UNIVERSITY LIBRARY, 201 OLIN LIBRARY CORNELL UNIVERSITY ITHACA, NY 14853, 13 May 2023 (2023-05-13), XP091507548, * abstract * * 1. Introduction * | 1-15 | |
| T | Sheridan Evan ET AL: "Enhancing density functional theory using the variational quantum eigensolver", arXiv.org, 28 February 2024 (2024-02-28), XP093180702, DOI: 10.48550/arxiv.2402.18534 Retrieved from the Internet: URL:https://arxiv.org/pdf/2402.18534 1. Introduction; * abstract * | | TECHNICAL FIELDS SEARCHED (IPC) G06N G16C |

The present search report has been drawn up for all claims

| Place of search | Date of completion of the search | Examiner |
|---|---|---|
| The Hague | 2 July 2024 | De Meyer, Arnaud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 23175487

**Non-patent literature cited in the description**

- **D. WECKER** ; **M. B. HASTINGS** ; **M. TROYER.** Progress towards practical quantum variational algorithms. *Phys. Rev. A*, 2015, vol. 92 (4), 042303
- **C. CADE et al.** Strategies for solving the Fermi-Hubbard model on near-term quantum computers. *Physical Review B*, 2020, vol. 102.23, 235122
- **Z. JIANG et al.** Quantum Algorithms to Simulate Many-Body Physics of Correlated Fermions. *Phys. Rev. Appl.*, 2018, vol. 9 (4), 044036
- **X.-Z. LUO et al.** Yao.jl: Extensible, Efficient Framework for Quantum Algorithm Design. *Quantum*, October 2020, vol. 4, 341
- **D. C. LIU** ; **J. NOCEDAL.** On the limited memory BFGS method for large scale optimization. *Mathematical Programming*, 1989, vol. 45, 503-528
- **S. G. JOHNSON**. *The NLopt nonlinear-optimization package*, 2007, https://github.com/stevengj/nlopt
- **M. J. P. HODGSON et al.** How Interatomic Steps in the Exact Kohn-Sham Potential Relate to Derivative Discontinuities of the Energy. *The Journal of Physical Chemistry Letters*, 2017, vol. 8.24, 5974-5980
- **M. IJÄS** ; **A. HARJU.** Lattice density-functional theory on graphene. *Phys. Rev. B*, 2010, vol. 82 (23), 235111
- **G. XIANLONG et al.** Bethe ansatz density-functional theory of ultracold repulsive fermions in one-dimensional optical lattices. *Physical Review B*, 2006, vol. 73.16, 165120
- **S. STANISIC et al.** Observing ground-state properties of the Fermi-Hubbard model using a scalable algorithm on a quantum computer. *Nature Communications*, 2022, vol. 13.1, 5743
- **A. V. IVANOV et al.** Quantum computation for periodic solids in second quantization. *Phys. Rev. Res.*, 2023, vol. 5 (1), 013200